# EUROPEAN PATENT SPECIFICATION

(11) **EP 1 497 265 B1**
(45) Date of publication and mention of the grant of the patent: **18.02.2009**
(21) Application number: 03718783.8
(22) Date of filing: 17.04.2003
(51) Int. Cl.: C07D 207/26, C07D 207/38, C07D 401/12, C07D 211/88, C07D 211/76, A61K 31/4015, A61K 31/4025, A61P 25/00, A61P 25/22, A61P 25/24

(54) **COMPOUNDS HAVING AFFINITY AT 5HT2C RECEPTOR AND USE THEREOF IN THERAPY**
VERBINDUNGEN MIT AFFINITÄT ZUM 5HT2C-REZEPTOR UND DEREN THERAPEUTISCHE VERWENDUNG
COMPOSES PRESENTANT UNE AFFINITE AVEC LE RECEPTEUR 5HT2C ET UTILISATION THERAPEUTIQUE DE CEUX-CI

(30) Priority: 19.04.2002 GB 0209029; 06.09.2002 GB 0220781
(43) Date of publication of application: 19.01.2005
(73) Proprietor: GLAXO GROUP LIMITED, Greenford, Middlesex UB6 ONN (GB)
(72) Inventor: DAMIANI, Federica GlaxoSmithKline S.p.A, I-37135 Verona (IT); HAMPRECHT, Dieter GlaxoSmithKline S.p.A, I-37135 Verona (IT); JAXA-CHAMIEC, Albert, A. GlaxoSmithKline S.p.A, I-37135 Verona (IT); MICHELI, Fabrizio GlaxoSmithKline S.p.A, I-37135 Verona (IT); PASQUARELLO, Alessandra GlaxoSmithKline S.p.A, I-37135 Verona (IT); TEDESCO, Giovanna GlaxoSmithKline S.p.A, I-37135 Verona (IT)
(74) Representative: Giddings, Peter John
(86) International application number: PCT/EP2003/004180
(87) International publication number: WO 2003/089409

(56) References cited:
- EP-A- 1 113 015
- WO-A-01/96308
- WO-A-97/48700
- WASER VON P G ET AL: "DIE ENTWICKLUNG NEUER ANTIEPILEPTIKA. I: ANTIKONVULSIVE WIRKUNG VONN-(P-SULFAMOYLPHENYL)-SUCCINIMID-DERIVA TEN" ARZNEIMITTEL FORSCHUNG. DRUG RESEARCH, EDITIO CANTOR. AULENDORF, DE, vol. 27, no. 2, 1977, pages 1942-1953, XP000615327 ISSN: 0004-4172

## Description

This invention relates to novel compounds having pharmacological activity, processes for their preparation, to compositions containing them and to their use in the treatment of CNS and other disorders.

WO 96/23783, WO 97/48699 and WO 97/48700 all disclose a series of indoline derivatives which are 5-HT_{2C} receptor antagonists and which are claimed to be useful in the treatment of various CNS disorders.

WO 01/96308 discloses pyridinone compounds which are useful for the treatment of neurodegenerative and CNS related diseases.

A novel class of compounds possessing 5-HT_{2C} receptor activity has been found. The present invention therefore provides, in a first aspect, a compound of formula (I) or a pharmaceutically acceptable salt thereof: 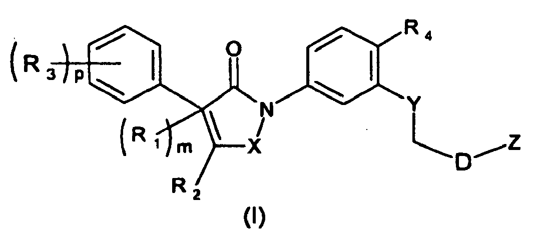 wherein:
R₁ is hydrogen, hydroxy, fluoro, chloro, C₁₋₆alkyl, C₃₋₇cyctoalkyl, C₃₋₇cycloalkyloxy, C₁₋₆alkoxy or haloC₁₋₆alkoxy;
m is 0 when 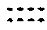 is a double bond and m is 1 when 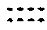 is a single bond;
R₂ is hydrogen, halogen, cyano, nitro, C₁₋₆alkyl, C₃₋₇cycloalkyl, C₃₋₇cycloalkyloxy, haloC₁₋₅alkyl, C₁₋₆alkoxy, haloC₁₋₆alkoxy, C₁₋₆alkylthio, amino, mono- or di-C₁₋₆alkylamino or an N-linked 4 to 7 membered heterocyclic group;
X is -CH₂-;
R₃ is halogen;
p is 1 or 2;
R₄ is hydrogen, halogen, hydroxy, cyano, nitro, C₁₋₆alkyl, C₁₋₆alkanoyl, C₃₋₇cycloalkyl, C₃₋₇cycloalkyloxy, haloC₁₋₆alkyl, C₁₋₆alkoxy, haloC₁₋₆alkoxy, C₁₋₆alkylthio; amino, mono- or di-C₁₋₆alkylamino or an N-linked 4 to 7 membered heterocyclic group;
Y is oxygen;
D is -CH₂-; and
Z is an optionally substituted N-linked 4 to 7 membered heterocyclic group.

The following terms, whether used alone or as part of another group are to be given the following meanings, unless otherwise stated.

The term "halogen" and its abbreviated form "halo" are used herein to describe fluorine, chlorine, bromine or iodine.

The term "alkyl" is used herein to describe a straight chain or branched fully saturated hydrocarbon group. "C₁₋₆alkyl" refers to alkyl groups having from one to six carbon atoms, including all isomeric forms, such as methyl, ethyl, propyl, isopropyl, butyl, isobutyl, sec-butyl, tert-butyl, pentyl, neopentyl, sec-pentyl, n-pentyl, isopentyl, tert-pentyl and hexyl.

The term "C₁₋₆alkanoyl" refers to an alkanoyl group having from 1 to 6 carbon atoms, such as methanoyl (or "formyl"), ethanoyl (or "acetyl"), propanoyl, isopropanoyl, butanoyl, isobutanoyl, sec-butanoyl, pentanoyl, neopentanoyl, sec-pentanoyl, isopentanoyl, tertpentanoyl and hexanoyl.

The term "C₁₋₆alkoxy" refers to a straight chain or branched chain alkoxy (or "alkyloxy") group having from one to six carbon atoms, including all isomeric forms, such as methoxy, ethoxy, propoxy, isopropoxy, butoxy, isobutoxy, sec-butoxy, tert-butoxy, pentoxy, neopentoxy, sec-pentoxy, n-pentoxy, isopentoxy, tert-pentoxy and hexoxy.

The term "C₃₋₇cycloalkyl" refers to a cycloalkyl group consisting of from 3 to 7 carbon atoms, such as cyclopropane, cyclobutane, cyclopentane, cyclohexane and cycloheptane. Optional substituents for C₃₋₇cycloalkyl includes one or more halogen, hydroxy, oxo, C₁₋₆alkyl, cyano, CF₃, OCF₃, C₁₋₆alkoxy and C₁₋₆alkanoyl.

The term "C₁₋₆alkylthio" refers to a straight chain or branched chain alkylthio group having from one to six carbon atoms, such as methylthio, ethylthio, propylthio, isopropylthio, butylthio, isobutylthio, sec-butylthio, tert-butylthio, pentylthio, neopentylthio, sec-pentylthio, n-pentylthio, isopentylthio, tert-pentylthio and hexylthio.

The term "mono- or di-C₁₋₆alkylamino" refers to an amino group which is substituted by one C₁₋₆alkyl group or an amino group which is substituted by two C₁₋₆alkyl groups, the two amino groups being the same or different. Examples of monoC1-6alkylamino include methylamine, ethylamine, propylamine, isopropylamine, butylamine, isobutylamine, sec-butylamine, tert-butylamine, pentylamine, neopentylamine, sec-pentylamine, n-pentylamine, isopentylamine, tert-pentylamine and hexylamine. Examples of di-C1-6alkylamino include dimethylamine, diethylamine, dipropylamine, diisopropylamine, dibutylamine, diisobutylamine, disec-butylamine, ditert-butylamine, dipentylamine, dineopentylamine, dihexylamine, butylmethylamino, isopropylmethylamino, ethylisopropylamino, ethylmethylamino, etc.

The terms "halo C₁₋₆alkoxy" or "haloC₁₋₆alkyl" are used to describe a C₁₋₆alkoxy or a C₁₋₆alkyl group, respectively, substituted with one or more halogens. Examples include - CHCl₂, -CF₃, -OCF₃, etc.

The term "heterocyclic group" is used herein to describe a stable aromatic or non-aromatic ring containing 1, 2 or 3 heteroatoms selected from nitrogen, sulphur and oxygen. Suitable examples of 4 to 7 membered heterocyclic groups include azetidinyl, pyrrolidinyl, imidazolidinyl, pyrazolidinyl, oxazolinyl, isothiazolidinyl, thiazolidinyl, pyrrolyl, pyrrolinyl, pyrazolinyl, imidazolyl, pyrazolyl, isothiazolyl, thiazolyl, piperidyl, piperazinyl, morpholinyl, pyridyl, pyridazinyl, pyrimidinyl, pyrazinyl, azepinyl, azepanyl, dioxolanyl, thienyl, tetrahydrothienyl, tetrahydrofuryl, dioxanyl and dithianyl.

The term "N-linked heterocyclic group" is used herein to describe a heterocyclic group which is linked to the remainder of the molecule via a nitrogen atom. Suitable examples of 4 to 7 membered N-linked heterocyclic groups include azetidinyl, pyrrolidinyl, imidazolidinyl, pyrazolidinyl, oxazolinyl, isothiazolidinyl, thiazolidinyl, pyrrolyl, pyrrolinyl, pyrazolinyl, imidazolyl, pyrazolyl, piperidyl, piperazinyl, morpholinyl and azepanyl.

More than one optional substituent may be present in the N-linked, which may be the same or different, and may be attached to any carbon atom of the heterocyclic group or an available nitrogen atom.

Suitable optional substituents for the N-linked include C₁₋₆alkyl, amino, mono- or di- C₁₋₆alkylamino, aryl, arylC₁₋₆alkyl, arylamino, hydroxy, C₁₋₆alkylamido, hydroxyC₁₋₆alkyl, C₁₋₆alkoxycarbonyl, halogen, haloC₁₋₆alkyl, a heteroaromatic group (such as indole or benzimidazole), an aromatic or non-aromatic N-linked or an aromatic or non-aromatic heterocycleC₁₋₆alkyl optionally substituted by C₁₋₆alkyl. Examples of aromatic or non-aromatic heterocycleC₁₋₆alkyl include heterocycle-methyl (such as pyridinyl-methyl and benzimidazolyl-methyl) and heterocycle-ethyl (such as morpholinyl-ethyl and indolyl-ethyl).

Substituents in the N-linked heterocyclic group may form a bridge structure, to form a group such as for example 2-oxa-5-azabicycto[2.2.1]heptyl. Such a bicyclic group may be further substituted by the substituents listed above. More than one substituent may be present on the same carbon atom to form spiro structures such as 1,4 and 1,5 dioxa spiro compounds.

When 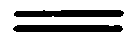 is a single bond, preferably R₁ is hydrogen, hydroxy or C₁₋₆alkoxy.

Preferably R₂ is hydrogen.

Preferably R₃ is chloro or fluoro, attached at the 3 or the 3,4-positions of the phenyl ring.

Preferably R₄ is C₁₋₆alkoxy (particularly methoxy), OCF₃, halogen or cyano.

Preferably Z is piperidyl. Preferred substituents include halogen (particularly fluoro) and C₁₋₆alkyl (particularly methyl).

Preferred compounds include:
- 3-(3,4-Dichloro-phenyl)-3-hydroxy-1-[4-methoxy-3-(2-piperidin-1-yl-ethoxy)-phenyl]-pyrrolidin-2-one
- 3-(3,4-Dichloro-phenyl)-3-hydroxy-1-[4-methoxy-3-(2-morpholin-4-yl-ethoxy)-phenyl]-pyrrolidin-2-one
- 3-(3,4-Dichloro-phenyl)-1-[4-methoxy-3-(2-piperidin-1-yl-ethoxy)-phenyl]-pyrrolidin-2-one
- 1-[4-Chloro-3-(2-piperidin-1-yl-ethoxy)-phenyl]-3-(3,4-dichloro-phenyl)-pyrrolidin-2-one
- 1-[4-Chloro-3-(2-piperidin-1-yl-ethoxy)-phenyl]-3-(3,4-dichloro-phenyl)-3-hydroxy-pyrrolidin-2-one
- 3-(3,4-Dichloro-phenyl)-1-(4-methoxy-3-[2-(4-methyl-piperidin-1-yl)-ethoxy]-phenyl)-pyrrolidin-2-one
- 3-(3,4-Dichloro-phenyl)-1-[4-methoxy-3-(2-piperidin-1-yl-ethoxy)-phenyl]-1,5-dihydro-pyrrol-2-one
- 3-(3,4-Dichloro-phenyl)-1-[4-methoxy-3-(2-morpholin-4-yl-ethoxy)-phenyl]-1,5-dihydropyrrol-2-one
- 1-[4-Chloro-3-(2-piperidin-1-yl-ethoxy)-phenyl]-3-(3,4-dichloro-phenyl)-1,5-dihydro-pyrrol-2-one
- 3-(3,4-Dichloro-phenyl)-1-[4-methoxy-3-(2-pyrrolidin-1-yl-ethoxy)-phenyl]-1,5-dihydro-pyrrol-2-one
- 3-(3-Fluoro-phenyl)-1-[4-methoxy-3-(2-piperidin-1-yl-ethoxy)-phenyl]-1,5-dihydro-pyrrol-2-one
- 3-(3,4-Dichloro-phenyl)-1-(-3-[2-(4,4-difluoro-piperidin-1-yl)-ethoxy]-4-methoxy-phenyl)-1,5-dihydro-pyrrol-2-one
- 3-(3-Fluoro-phenyl)-1-[4-methoxy-3-(2-piperidin-1-yl-ethoxy)-phenyl]-pyrrolidin-2-one
- 3-(3,4-Dichloro-phenyl)-1-[4-methoxy-3-(2-pyrrolidin-1-yl-ethoxy)-phenyl]-pyrrolidin-2-one
- 3-(3,4-Dichloro-phenyl)-1-[4-methoxy-3-(2-piperidin-1-yl-ethoxy)-phenyl]-3-methylpyrrolidin-2-one
- 3-(3-Chloro-phenyl)-1-{4-methoxy-3-[2-(4-methyl-piperidin-1-yl)-ethoxy]-phenyl}-1,5-dihydro-pyrrol-2-one
- 3-(3,4-Dichloro-phenyl)-1-(4-methoxy-3-[2-(4-methyl-piperidin-1-yl)-ethoxy]-phenyl)-3,4-dihydro-pyrrol-2-one
- 3-(3,4-Dichloro-phenyl)-1-(4-methoxy-3-[2-(4-methyl-piperidin-1-yl)-ethoxy]-phenyl)-4-methyl-1,5-dihydro-pyrrol-2-one
- 3-(4-Chloro-phenyl)-1-(4-methoxy-3-[2-(4-methyl-piperidin-1-yl)-ethoxy]-phenyl)-3,4-dihydro-pyrrol-2-one
- 3-(4-Chloro-phenyl)-1-(4-methoxy-3-[2-piperidin-1-yl)-ethoxy]-phenyl)-3,4-dihydro-pyrrol-2-one
- 3-(3,4-Dichloro-phenyl)-1-[4-methoxy-3-(piperidin-3-ylmethoxy)-phenyl]-pyrrolidin-2-one
- 3-(3,4-Dichloro-phenyl)-1-[4-methoxy-3-(1-methyl-piperidin-3-ylmethoxy)-phenyl]-pyrrolidin-2-one
- N-{4-[3-(3,4-dichlorophenyl)-2-oxo-2,5-dihydro-1H-pyrrol-1-yl]-2-[2-(1-piperidinyl)-ethoxy]phenyl}acetamide
- N{4-[3-(3,4-dichlorophenyl)-2-oxo-pyrrolidin-1-yl]-2-[2-(1-piperidinyl)ethoxy]phenyl}-acetamide
- 3-(3,4-Dichloro-phenyl)-1-(4-methoxy-3-[2-(4-methyl)-pipefidin-1-yl-ethoxy]-phenyl)-3-methyl-pyrrolidin-2-one
- 3-(3-Chloro-phenyl)-1-[4-methoxy-3-(2-pipefidin-1-yl-ethoxy)-phenyl]pyrrolidin-2-one
- 3-(3-Trifluoromethyl-phenyl)-1-[4-methoxy-3-(2-piperidin-1-yl-ethoxy)-phenyl]-pyrrolidin-2-one
- 3-(3-Trifluoromethyl-phenyl)-1-[4-methoxy-3-(2-pipefidin-1-yl-ethoxy)-phenyl]-1,5-dihydro-pyrrol-2-one
- 3-(3-Chloro-phenyl)-1-[4-methoxy-3-(2-piperidin-1-y1-ethoxy)-phenyl]-1,5-dihydropyrrol-2-one
- 3-(4-Fluoro-phenyl)-1-[4-methoxy-3-(2-piperidin-1-yi-ethoxy)-phenyl)-1,5-dihydropyrrol-2-one
- 3-(4-Fluoro-phenyl)-1-[4-methoxy-3-(2-piperidin-1-yl-ethoxy)-phenyl]-pyrrolidin-2-one
- 1-{4-Methoxy-3-[2-(4-methyl-piperidin-1-yl)-ethoxy]-phenyl)-3-(4-methyl-phenyl)-1,5-dihydro-pyrrol-2-one
- 1-{4-Methoxy-3-[2-(piperidin-1-yl)-ethoxy]-phenyl}-3-(4-methyl-phenyl)-1,5-dihydropyrrol-2-one
- 3-(4-Bromo-phenyl)-1-{4-methoxy-3-[2-(4-methyt-piperidin-1-yl)-ethoxy]-phenyl}-1,5-dihydro-pyrrol-2-one
- 1-{4-Methoxy-3-[2-(4-methyl-piperidin-1-yl)-ethoxy]-phenyl}-3-(4-trifluoromethylphenyl)-1,5-dihydro-pyrrol-2-one
- 3-(2-Chloro-phenyl)-1-{4-methoxy-3-[2-(4-methyl-piperidin-1-yl)-ethoxy]-phenyl}-1,5-dihydro-pyrrol-2-one
- 3-(3,4-Dichloro-phenyl)-3-hydroxy-1-{4-methoxy-3-[2-(4-methyl-piperidin-1-yl)-ethoxy)-phenyl}-pyrrolidin-2-one
- 3-(3,4-Dichloro-phenyl)-3-fluoro-1-{4-methoxy-3-[2-(4-methyl-piperidin-1-yl)-ethoxy]-phenyl}-pyrrolidin-2-one
- 3-(3,4-Dichloro-phenyl)-1-{4-methoxy-3-[(1-methyl-pyrrolidin-2-yl)-methoxy]-phenyl}-pyrrolidin-2-one
- 3-(3,4-Dichloro-phenyl)-1-{4-methoxy-3-[(1-methyl-pyrrolidin-2-yl)-methoxy]-phenyl}-3,4-dihydro-pyrrol-2-one
and pharmaceutically acceptable salts thereof.

The compounds of formula (I) can form acid addition salts. It will be appreciated that for use in medicine the salts of the compounds of formula (I) should be pharmaceutically acceptable. Suitable pharmaceutically acceptable salts will be apparent to those skilled in the art and include those described in J. Pharm. Sci., 1977, 66, 1-19, such as acid addition salts formed with inorganic acids e.g. hydrochloric, hydrobromic, sulfuric, nitric or phosphoric acid; and organic acids e.g. succinic, maleic, acetic, fumaric, citric, tartaric, benzoic, p-toluenesulfonic, methanesulfonic or naphthalenesulfonic acid.

The compounds of this invention may be in crystalline or non-crystalline form, and, if crystalline, may optionally be hydrated or solvated. This invention includes within its scope stoichiometric hydrates as well as compounds containing variable amounts of water.

Certain compounds of formula (I) are capable of existing in stereoisomeric forms (e.g. geometric or *("cis-trans")* isomers, diastereomers and enantiomers) and the invention extends to each of these stereoisomeric forms and to mixtures thereof including racemates. The different stereoisomeric forms may be separated one from the other by the usual methods, or any given isomer may be obtained by stereospecific or asymmetric synthesis. The invention also extends to any tautomeric forms and mixtures thereof.

The present invention also provides a process for the preparation of a compound of formula (I) or a pharmaceutically acceptable salt thereof, which process comprises:
(a) reacting a compound of formula (II): 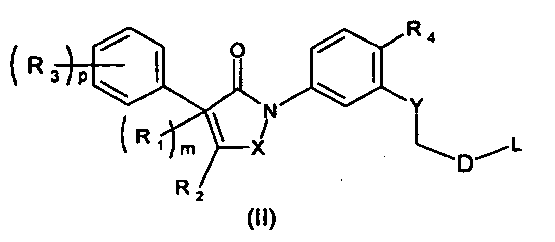 wherein R₁, R₂, R₃, R₄, m, p, X, 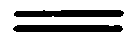 , Y and D are as defined for formula (I), and L is a leaving group, with a compound of formula (III):

   Z-H (III)

   wherein Z is as defined for formula (I); or
(b) cyclising a compound of formula (IV): 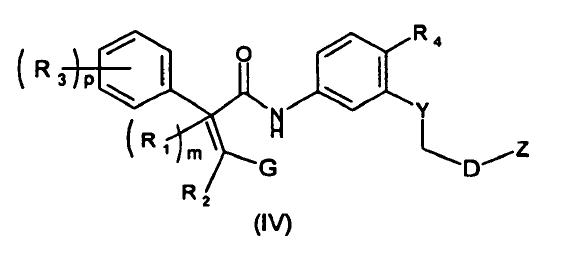
wherein R₁, R₂, m, R₃, p, R4, Y, D, Z and are as defined for formula (I) and G is a group -X=CH₂, wherein X is as defined for formula (I), dehydrogenated as required;
optionally followed by:
- removing any protecting groups; and/or
- converting a compound of formula (I) into another compound of formula (I); and/or
- forming a pharmaceutically acceptable salt.

For the reaction of process (a), suitably L is mesylate. The reaction may take place in a solvent such as DMF in the presence of sodium iodide and potassium carbonate.

The reaction of process (b) suitably takes place in a solvent such as THF in the presence of OsO₄ and NalO₄.

Compounds of formula (I) can be converted into further compounds of formula (I) using standard techniques. For example, and by way of illustration rather than limitation, a compound wherein X is -(HCOH)- may be converted to a compound wherein X is -(CH₂)-by using a suitable reducing agent such as triethylsilane-trifluoroacetic acid using dichloromethane as solvent, and a compound wherein R₁ is hydroxy may be converted to compound wherein m is 0 and 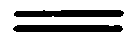 is a double bond by an elimination reaction in TFA.

Compounds of formulae (II), (III) and (IV) are commercially available or may be prepared according to methods described herein or may be prepared according to known methods or by analogous methods thereto.

Those skilled in the art will appreciate that it may be necessary to protect certain groups to carry out the above processes. Suitable protecting groups and methods for their attachment and removal are conventional in the art of organic chemistry, such as those described in Greene T.W. 'Protective groups in organic synthesis' New York, Wiley (1981).

Pharmaceutically acceptable salts may be prepared conventionally by reaction with the appropriate acid or acid derivative.

In another aspect, the present invention provides a pharmaceutical composition comprising a compound of formula (I) or a pharmaceutically acceptable salt thereof, and a pharmaceutically acceptable carrier or excipient.

In a further aspect, the present invention provides a process for preparing a pharmaceutical composition, the process comprising mixing a compound of formula (I) or a pharmaceutically acceptable salt thereof and a pharmaceutically acceptable carrier or excipient.

A pharmaceutical composition of the invention, which may be prepared by admixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders, injectable or infusible solutions or suspensions or suppositories. Orally administrable compositions are generally preferred.

Tablets and capsules for oral administration may be in unit dose form, and may contain conventional excipients, such as binding agents (e.g. pregelatinised maize starch, polyvinylpyrrolidone or hydroxypropyl methylcellulose);, fillers (e.g. lactose, microcrystalline cellulose or calcium hydrogen phosphate);, tabletting lubricants lubricants (e.g. magnesium stearate, talc or silica);, disintegrants (e.g. potato starch or sodium starch glycollate); and acceptable wetting agents (e.g. sodium lauryl sulphate). The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents (e.g. sorbitol syrup, cellulose derivatives or hydrogenated edible fats), emulsifying agents (e.g. lecithin or acacia), non-aqueous vehicles (which may include edible oils e.g. almond oil, oily esters, ethyl alcohol or fractionated vegetable oils), preservatives (e.g. methyl or propyl-p-hydroxybenzoates or sorbic acid), and, if desired, conventional flavourings or colorants, buffer salts and sweetening agents as appropriate. Preparations for oral administration may be suitable formulated to give controlled release of the active compound.

For parenteral administration, fluid unit dosage forms are prepared utilising a compound of the invention or pharmaceutically acceptable salt thereof and a sterile vehicle. Formulations for injection may be presented in unit dosage form e.g. in ampoules or in multi-dose, utilising a compound of the invention or pharmaceutically acceptable salt thereof and a sterile vehicle, optionally with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, e.g. sterile pyrogen-free water, before use. The compound, depending on the vehicle and concentration used, can be either suspended or dissolved in the vehicle. In preparing solutions, the compound can be dissolved for injection and filter sterilised before filling into a suitable vial or ampoule and sealing. Advantageously, adjuvants such as a local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilisation cannot be accomplished by filtration. The compound can be sterilised by exposure to ethylene oxide before suspension in a sterile vehicle. Advantageously, a surfactant or wetting agent is included in the composition to facilitate uniform distribution of the compound.

Lotions may be formulated with an aqueous or oily base and will in general also contain one or more emulsifying agents, stabilising agents, dispersing agents, suspending agents, thickening agents, or colouring agents. Drops may be formulated with an aqueous or non-aqueous base also comprising one or more dispersing agents, stabilising agents, solubilising agents or suspending agents. They may also contain a preservative.

The compounds of the invention may also be formulated in rectal compositions such as suppositories or retention enemas, e.g. containing conventional suppository bases such as cocoa butter or other glycerides.

The compounds of the invention may also be formulated as depot preparations. Such long acting formulations may be administered by implantation (for example subcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

For intranasal administration, the compounds of the invention may be formulated as solutions for administration via a suitable metered or unitary dose device or alternatively as a powder mix with a suitable carrier for administration using a suitable delivery device. Thus compounds of formula (I) may be formulated for oral, buccal, parenteral, topical (including ophthalmic and nasal), depot or rectal administration or in a form suitable for administration by inhalation or insufflation (either through the mouth or nose).

The compounds of the invention may be formulated for topical administration in the form of ointments, creams, gels, lotions, pessaries, aerosols or drops (e.g. eye, ear or nose drops). Ointments and creams may, for example, be formulated with an aqueous or oily base with the addition of suitable thickening and/or gelling agents. Ointments for administration to the eye may be manufactured in a sterile manner using sterilised components.

The compounds of the present invention have affinity for the 5-HT_{2C} receptor. The affinity can be determined by assessing their ability to displace [³H]-mesulergine from rat or human 5-HT_{2C} clones expressed in 293 cells *in vitro,* as described in WO 94/04533.

All the Example compounds were tested according to this assay and were found to have pKi values >5.8. Some compounds show a considerably higher affinity in the range of 7.0 to >9.0 in human cells.

The intrinsic activity of the compounds of this invention can be determined according to the [³⁵S]GTPyS functional assay which is described in WO 99/07700.

Compounds of formula (I) and their pharmaceutically acceptable salts are of use in the treatment of certain CNS disorders such as depression (which term is used herein to include bipolar depression, unipolar depression, single or recurrent major depressive episodes with or without psychotic features, catatonic features, melancholic features, atypical features or postpartum onset, seasonal affective disorder, dysthymic disorders with early or late onset and with or without atypical features, neurotic depression and social phobia, depression accompanying dementia for example of the Alzheimer's type vascular dementia with depressed mood, schizoaffective disorder or the depressed type, and depressive disorders resulting from general medical conditions including, but not limited to, myocardial infarction, diabetes, miscarriage or abortion, *etc*), anxiety including generalised anxiety and social anxiety disorder, schizophrenia, panic disorder, agoraphobia, social phobia, epilepsy, obsessive compulsive disorder and post-traumatic stress disorder, pain (particularly neuropathic pain), migraine, memory disorders, including dementia, amnesic disorders and age-associated memory impairment, disorders of eating behaviours including anorexia nervosa and bulimia nervosa, sexual dysfunction, sleep disorders (including disturbances of circadian rhythm, dyssomnia, insomnia, sleep apnea and narcolepsy), withdrawal from abuse of drugs such as of cocaine, ethanol, nicotine, benzodiazepines, alcohol, caffeine, phencyclidine (phencyclidine-like compounds), opiates (e.g. cannabis, heroin, morphine), sedative ipnotic, amphetamine or amphetamine-related drugs (e.g. dextroamphetamine, methylamphetamine) or a combination thereof, Alzheimer's disease, motor disorders such as Parkinson's disease, dementia in Parkinson's disease, neuroleptic-induced Parkinsonism and tardive dyskinesias, as well as other psychiatric disorders, disorders associated with spinal trauma and/or head injury such as hydrocephalus, gastrointestinal disorders such as IBS (Irritable Bowel Syndrome), Crohn's disease, ulcerative colitis, non-steroidal antiinflammatory drug induced damage) as well as microvascular diseases such as macular oedema and retinopathy.

It is to be understood that, as used herein, the term "treatment" refers to alleviation of established symptoms as well as prophylaxis.

Thus the present invention also provides a compound of formula (I) or a pharmaceutically acceptable salt thereof, for use as a therapeutic substance. In particular, the present invention provides a compound of formula (I) or a pharmaceutically acceptable salt thereof for use in the treatment of the above disorders. In particular the invention provides a compound of formula (I) or a pharmaceutically acceptable salt thereof for use as a therapeutic substance in the treatment of a CNS disorder. Preferably the CNS disorder is depression or anxiety.

Compounds of the invention may be administered in combination with other active substances such as 5HT3 antagonists, NK-1 antagonists, serotonin agonists, selective serotonin reuptake inhibitors (SSRI), noradrenaline re-uptake inhibitors (SNRI), tricyclic antidepressants and/or dopaminergic antidepressants.

Suitable 5HT3 antagonists which may be used in combination of the compounds of the inventions include for example ondansetron, granisetron, metoclopramide.

Suitable serotonin agonists which may be used in combination with the compounds of the invention include sumatriptan, rauwolscine, yohimbine, metoclopramide.

Suitable SSRIs which may be used in combination with the compounds of the invention include fluoxetine, citalopram, femoxetine, fluvoxamine, paroxetine, indalpine, sertraline, zimeldine.

Suitable SNRIs which may be used in combination with the compounds of the invention include venlafaxine and reboxetine.

Suitable tricyclic antidepressants which may be used in combination with a compound of the invention include imipramine, amitriptiline, chlomipramine and nortriptiline.

Suitable dopaminergic antidepressants which may be used in combination with a compound of the invention include bupropion and amineptine.

It will be appreciated that the compounds of the combination or composition may be administered simultaneously (either in the same or different pharmaceutical formulations), separately or sequentially.

The invention further provides a method of treatment of the above disorders in mammals including humans, which comprises administering to the sufferer a therapeutically safe and effective amount of a compound of formula (I) or a pharmaceutically acceptable salt thereof. In particular the invention provides a a method of treatment of a CNS disorder in mammals including humans, which comprises administering to the sufferer a therapeutically safe and effective amount of a compound of formula (I) or a pharmaceutically acceptable salt thereof. Preferably the disorder is depression or anxiety.

In another aspect, the invention provides for the use of a compound of formula (I) or a pharmaceutically acceptable salt thereof in the manufacture of a medicament for use in the treatment of the above disorders. In particular the present invention provides the use of a compound of formula (I) or a pharmaceutically acceptable salt thereof in the manufacture of a medicament for use in the treatment of a CNS disorder. Preferably the CNS disorder is depression or anxiety.

The composition of the present invention may contain from 0.1% to 99% by weight, preferably from 10 to 60% by weight, of the active material, depending on the method of administration. The dose of the compound used in the treatment of the aforementioned disorders will vary in the usual way with the seriousness of the disorders, the weight of the sufferer, and other similar factors. However, as a general guide suitable unit doses may be 0.05 to 1000 mg, more suitably 1.0 to 200 mg, and such unit doses may be administered more than once a day, for example two or three times a day. Such therapy may extend for a number of weeks or months. When administered in accordance with the invention, no unacceptable toxicological effects are expected with the compounds of the invention.

The following Descriptions and Examples illustrate the preparation of compounds of the present invention.

### Preparation 1:1-Benzyl-4,4-difluoro-piperidine

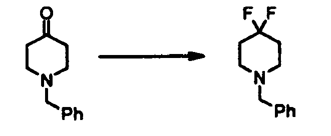

**Procedure:** Chem. Pharm. Bull. 1993, 41, 11, 1971.
**NMR (¹H, CDCl₃):** δ 7.4-7.25 (m, 5H), 3.55 (s, 2H), 2.54 (m, 4H), 1.99 (m, 4H). MS *(m*/*z):* 212 [MH]⁺

### Preparation 2 : 4,4-Difluoro-piperidine hydrochloride

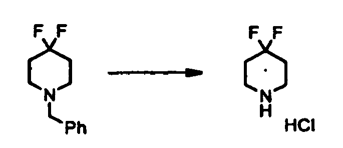

**Procedure:** Chem. Pharm. Bull. 1993, 41, 11, 1971.
**NMR** (¹H, **DMSO-d6):** δ 9.22 (bs, 2H), 3.20 (m, 4H), 2.25 (m, 4H). MS (*m*/*z*): 122 [MH-HCl]⁺

### Preparation 3: 2-Chloro-1-(4,4-difluoro-piperidin-1-yl)-ethanone

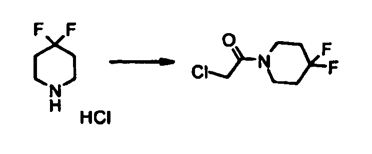

**Procedure:** To a solution of 4,4-difluoro-piperidine hydrochloride (1120 mg, 7.13 mmol) in anh. CH₂Cl₂ (10ml) was added, at 0°C and under N₂, TEA (2.5eq, 2.18 ml) and chloroacetyl chloride (1.1eq, 661µl). The reaction was stirred at room temperature for 1 hour. The solution was diluted with water (30 ml) and extracted with ethyl acetate (3x25 ml). The combined organic extracts were dried over anhydrous Na₂SO₄, filtered and concentrated to dryness *in vacuo* to give 1128 mg of the title product (yield: 80.3%).
**NMR (¹H, DMSO-d6):** δ 4.44 (s, 2H), 3.55 (m, 4H), 2.0 (m, 4H). **MS *(m*/*z):*** 198 [MH]⁺, 1Cl

### Preparation 4: 2-Chloro-5-nitro-phenol

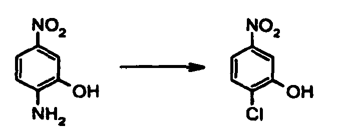

**Procedure:** J. Chem. Soc. 1896, 69, 1326.
**NMR (¹H, DMSO-d6):** δ 11.3 (s, 1 H), 7.75 (d, 1H), 7.66 (d, 1H), 7.64 (s, 1H).

### Preparation 5: Acetic acid 2-(2-methoxy-5-nitro-phenoxy)-ethyl ester

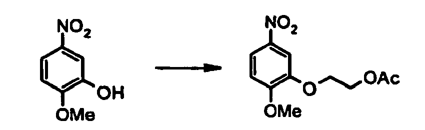

**Procedure:** To a solution of 2-methoxy-5-nitro-phenol (2202 mg, 13.02 mmol) in acetone (110 ml), under N₂, was added K₂CO₃ (4.45 eq., 8000 mg) and 2-bromoethyl acetate (2.1 eq, 3ml). The mixture was heated to reflux for 20 hours, filtered and then concentrated *in vacuo.* The crude product was triturated with AcOEt/cHex 1/1 (70ml) to give 2999 mg of the title product as a white solid (90.2%, mp: 120-121.3°C).
**NMR (¹H, CDCl₃):** δ 7.9 (dd, 1H), 7.75 (d, 1H), 6.9 (d, 1H), 4.45 (m, 2H), 4.3 (m, 2H), 3.95 (s, 3H), 2.05 (s, 3H).

### Preparation 6: 1-[2-(2-Methoxy-5-nitro-phonoxy)-ethyl]-piperidine

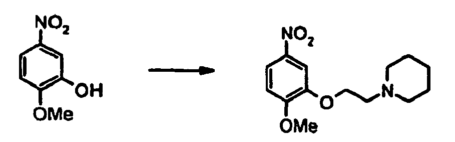

**Procedure:** To a solution of 2-methoxy-5-nitrophenol (1.5 g, 8.87 mmol) in DMF (18 ml) at room temperature were added K₂CO₃ (2.70 g, 19.52 mmol) and (1-(2-chloroethyl)piperidin hydrochloride) (1.79 g, 9.75 mmol). The suspension was stirred at room temperature for 24 hours. The suspension was diluted with water (20 ml) and extract with EtOAc (2x20 ml), the combined organic phases were dried over Na₂SO₄, filtered and concentrated to dryness *in vacuo,* to obtain the title product as a yellow oil.
**NMR (¹H, CDCl₃):** δ 7.85 (dd, 1H), 7.75 (d, 1H), 6.90 (d, 1H), 4.20 (t, 2H), 3.90 (s, 3H), 2.80 (t, 2H), 2.45-2.60 (m, 4H), 1.55-1.65 (m, 4H) (m, 4H), 1.35-1.50 (m, 2H).
**MS** (*m*/*z*): 281 [MH]⁺.

### Preparation 7: 1-(4,4-Difluoro-piperidine-1-yl)-2-(2-methoxy-4-nitro-phenoxy)-ethanone

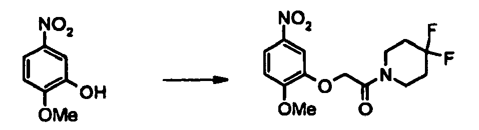

**Procedure:** 2-Methoxy-4-nitro-phenol (177 mg, 1.05 mmol), was dissolved in dry DMF ( 2 ml) under N₂. Potassium carbonate (158 mg, 1.1 eq) was added and the mixture was stirred for 15 min, then 2-chloro-1-(4,4-difluoro-piperidin-1-yl)-ethanone (228 mg, 1.1 eq) dissolved in dry DMF (3 ml) was added. After 20 hours a saturated solution of NH₄Cl was added and the mixture extracted with CH₂Cl₂ (2x25 ml). The organic phase was washed with NaOH 0.1 N (50 ml), water (50 ml), dried over Na₂SO₄ and concentrated in vacuo to give 405 mg of crude title prodcuct (mp 127-130 °C) that was utilized without further purification.
**NMR (¹H, CDCl₃):** δ 7.98 (dd, 1H), 7.77 (d, 1H), 6.96 (d, 1H), 4.84 (s, 2H), 3.98 (s, 3H), 3.8-3.65 (m, 4H), 2.15-1.95 (m, 4H). MS (*mlz*): 331 [MH]^{*}.

### Preparation 8: 1-[2-(2-Chloro-5-nitro-phenoxy)-ethyl]-piperidine

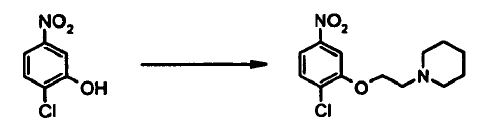

**NMR (¹H, CDCl₃):** δ 7.84 (d, 1H), 7.81 (dd, 1H), 7.52 (d, 1H), 4.30 (t, 2H), 2.92 (t, 2H), 2.6 (m, 4H), 1.5-1.7 (m, 6H). MS *(mlz):* 285 [MH]⁺, 1Cl.

### Preparation 9: Acetic acid 2-(5-amino-2-methoxy-phenoxy)-ethyl ester

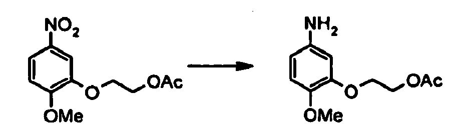

**Procedure:** To a suspension of acetic acid 2-(2-methoxy-5-nitro-phenoxy)-ethyl ester (2904 mg, 11.38 mmol) in methanol (85 ml) was added, under N₂, ammonium formate (5.4 eq, 3900 mg) and Pd/C 10% (cat, 1100 mg). The reaction was stirred at room temperature for 1.5 hours, then filtered on celite pad and concentrated to dryness *in vacuo.* The crude product was then dissolved in CH₂Cl₂ (50ml) and the organic phase was washed with brine (2x30ml) and water (1x30ml). The combined organic extracts were dried over anhydrous Na₂SO₄ filtered and concentrated to dryness *in vacuo.* Flash chromatography of the crude product (silica gel, cHex/AcOEt 4/6) gave 2299 mg of the title product as a white foam (yield: 89.7%).
**NMR (¹H, CDCl₃):** δ 6.7 (dd, 1H), 6.3 (d, 1H), 6.25 (dd, 1H), 4.4 (m, 2H), 4.15 (m, 2H), 3.75 (s, 3H), 2.05 (s, 3H). **MS (*m*/*z*):** 226.3 [MH]⁺.

### Preparation 10: 4-Methoxy-3-(2-piperidin-1-yl)ethoxy-phenylamine hydrochloride

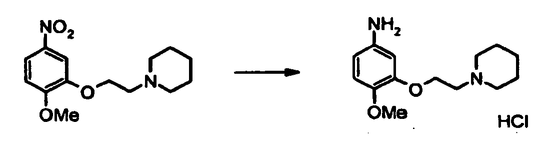

**Procedure:** To a solution of the compound of Preparation 6 (2.30 g, 8.21 mmol) in MeOH (43.18 ml) were added a solution of NH₄Cl (5 eq, 2.20 g) in H₂O (36.83 ml) and iron (3 eq, 1.38 g). After stirring at reflux for 3 hours, the solution was concentrated *in vacuo* and the crude was tritured with CH₂Cl₂ to obtain 1.82 g of th title compound as a brown gum (yield: 76%).
(¹H, DMSO-d6): δ 10.4-10.0 (broad, 1H), 6.72 (d, 1H), 6.34(d, 1H), 6.20 (dd, 1H), 5.4-5.0 (broad, 2H), 4.24 (t, 2H), 3.64 (s, 3H), 3.52 (bs, 2H), 3.42 (t, 2H), 3.00 (bs, 2H), 1.9-1.3 (bs, 6H). **MS *(m*/*z):*** 251 [MH]⁺.

### Preparation 11: 4-Chloro-3-(2-piperidin-1-yl-ethoxy)-phenylamine

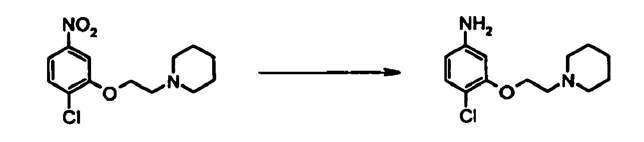

**Procedure:** To a solution of 1-[2-(2-chloro-5-nitro-phenoxy)-ethyl]-piperidine (782 mg, 2.75 mmol) in MeOH (8 ml) were added a solution of NH₄Cl (5 eq, 735 mg) in H₂O (8 ml) and iron (3 eq, 457 mg). After stirring at reflux for 12 hours, the solution was concentrated *in vacuo* and chromatographated over silica gel (CH₂Cl₂/MeOH 85/15) to give 757 mg of the title product as a green foam (yield: 87%).
**NMR (¹H, CD₃OD):** δ 7.0 (d, 1H), 6.4 (d, 1H), 6.2 (dd, 1H), 4.25 (t, 2H), 3.35 (t, 2H), 3.25 (s, 2H), 3.15 (m, 4H), 1.8-1.5 (m, 6H). **MS (*mlz*);** 255 [MH]⁺, 1Cl.

### Preparation 12: 2-(3,4-Dichloro-phenyl)-pent-4-enoic acid

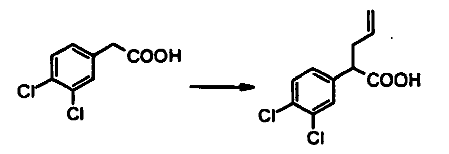

**Procedure:** A solution of 3,4-dichloro-phenylacetic acid (1.47 g, 7.17 mmol) in anh. THF (21 ml), under N₂, was treated with lithium bis(trimethylsilyl) amide (1M solution in THF, 2.2 eq., 16 ml) at -78°C for 30 minutes before allyliodide(1.65 eq., 1.1 ml) was added. The mixture was stirred for 3 hours at room temperature and quenched with water (20 ml). The reaction mixture was acidified to pH=4 with HCl 1N. The product was extracted with ethyl acetate (2x 15ml) and the organic phase was washed with brine (1x15ml), dried over Na₂SO₄ and concentrated *in vacuo.* The crude product was purified by flash chromatography (silica gel, AcOEt/cHex 2/8) to give 1137 mg of the title product as a pale yellow solid (81 %).
NMR (¹H, CDCl₃): δ 7.4 (m, 2H), 7.15 (dd, 1H), 5.7 (m, 1H), 5.05 (m, 2H), 3.6 (t, 1 H), 2.8 (m, 1H), 2.5 (m, 1H). **MS (m/z):** 244 [MH]⁺ +2Cl.

### Preparation 13: 2-(3-Fluoro-phenyl)-pent-4-enoic acid

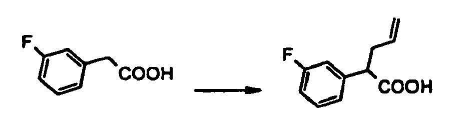

**Procedure:** To a solution of 3-fluorophenylacetic acid (1.0 g, 6.49 mmol) in THF (10 mL), at -78°C was added lithium bis(trimethylsilyl)amide (14.28 mmol). To solution was stirred at this temperature for 30 min and allyl bromide (0.84 mL. 9.74 mmol) was added. The solution was stirred at room temperature for 3 hours. Then the solution ws diluted with water, acidified with HCl 2 N and extracted with EtOAC (3x). The combined organic phases were dried over anhydrous Na₂SO₄ filtered and concentrated to dryness *in vacuo.* Flash chromatography of the crude product (silica gel, cHex/AcOEt 7/3) gave 1.120 g of the title product (yield = 89%, mp = 49°C)
**NMR (¹H, DMSO-d6):** δ 12.50 (bs, 1H), 7.35 (m, 1 H), 7.00-7.25 (m, 3H), 5,60-5.80 (m,1H), 4.90-5.10 (m, 1H), 3.65 (t, 1 H), 2.60-2.70 (m, 1 H), 2.35-2.50 (m, 1 H).

### Preparation 14: Acetic acid 2-(5-[2-(3,4-dichloro-phenyl)-pent-4-enoylamine]-2-methoxy-phenoxy)-ethyl ester

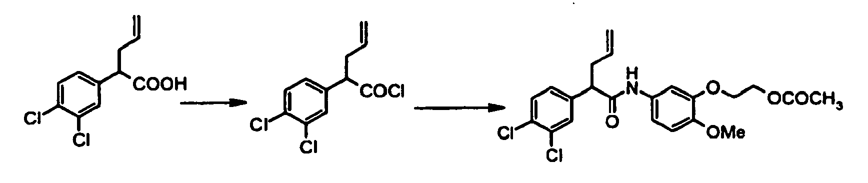

**Procedure:** To a solution of 2-(3,4-dichloro-phenyl)-pent-4-enoic acid (222.4 mg, 0.907 mmol) in anh. CH₂Cl₂ (4 ml), under N₂, was added, at 0°C, oxalyl chloride (1.9 eq., 0.15 ml) and DMF (cat). The reaction was stirred at 0°C for 15' and at room temperature for 18 hours. The reaction mixture was concentrated to dryness *in vacuo* and the crude intermediate was then dissolved, under N₂, in anh. CH₂Cl₂ (5 ml). Acetic acid 2-(5-amino-2-methoxy-phenoxy)-ethyl ester VVSI/6002/42/1) (1.22eq, 250 mg) and TEA (2 eq., 0.25 ml) were added to the reaction mixture at 0°C. The reaction was stirred at 0°C for 15' and at room temperature for 18 hours. The reaction mixture was concentrated to dryness *in vacuo* The crude intermediate was then dissolves in CH₂Cl₂ and the organic phase was washed with NH₄Cl sat (2x10m1) and brine (2x10ml). Flash chromatography of the crude product (silica gel, cHex/AcOEt 7/3) gave 259 mg of the title product as a white (yield: 63.1 %, mp: 112.7-114.2)
**NMR (¹H, DMSO-d6):** δ 10.02 (s, 1H), 7.60 (m, 2H), 7.36 (dd, 1H), 7.29 (d, 1H), 7.07 (dd, 1H), 6.88 (d, 1H), 5.70 (m, 1H), 5.07 (m, 1H), 4.98 (m, 1H), 4.30 (t, 2H), 4.09 (t, 2H), 3.74 (m, 1H), 3.70 (s, 3H), 2.75 (m, 1H), 2.45 (m, 1H), 2.02 (s, 3H) **MS *(m*/*z)***: 452 [MH]⁺+2Cl
mp: 112.7-114°C.

### Preparation 15: Acetic acid 2-(5-[2-(3-fluoro-phenyl)-pent-4-enoylamino]-2-methoxy-phenoxy)-ethyl ester

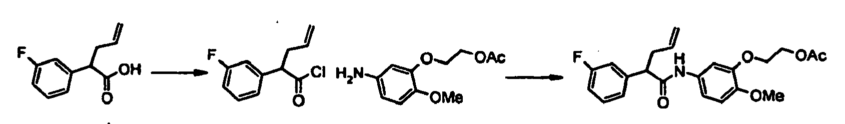

**NMR (¹H, CDCl₃):** δ 7.36 (d, 1H), 7.34 (m, 1H), 7.15 (d, 1H), 7.10 (dd, 1H), 7.04 (bs, 1H), 7.00 (m, 1H), 6.77-6.84 (m, 1H), 5.74 (m, 1H), 5.00-5.14 (m, 2H), 4.42 (t, 2H), 4.22 (t, 2H), 3.82 (s, 3H), 3.51 (t, 1H), 2.96 (m, 1H), 2.58 (m, 1H), 2.08 (s, 3H). **MS (*m*/*z*)**:402 [MH)⁺. mp: 108.7-109.3 °C

### Preparation 16: 2-(3,4-Dichloro-phenyl)-pent-4-enoic-acid [4-chloro-3-(2-piperidin-1-yl-ethoxy)-phenyl]-amide

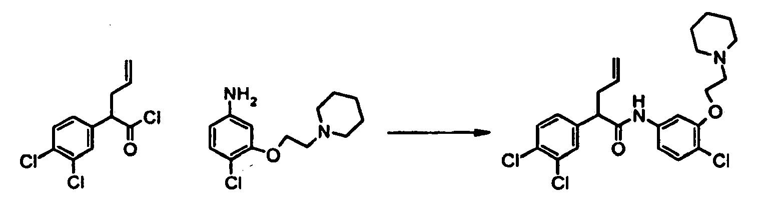

**NMR (¹H, CO₃OD):** δ 7.58 (d, 1H), 7.54 (d, 1H), 7.48 (d, 1H), 7.33 (dd, 1 H), 7.27 (d, 1H), 7.0 (dd, 1H), 5.77 (m, 1H), 5.15-5.0 (m, 2H), 4.23 (t, 2H), 3.70 (t, 1H), 3.02 (t, 2H), 2.9 (m, 1H), 2.82 (m, 4H), 2.5 (m, 1H), 1.69 (m, 4H), 1.54 (m, 2H). **MS (*mlz*)**: 481 [MH] +3Cl.

### Preparation 17: 2-(3,4-Dichloro-phenyl)-pent-4-enoic acid (3-[2-(4,4-difluoro-piperidin-1-yl)-ethoxy]-4-methoxy-phenyl)-amide

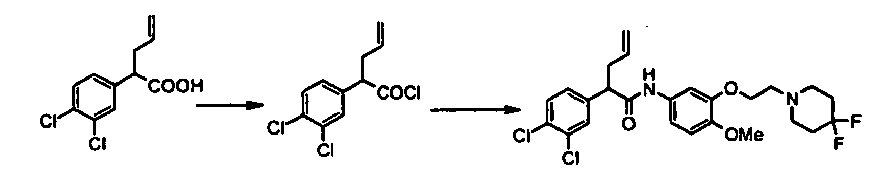

**NMR (¹H, DMSO-d6):** δ 9.96 (s, 1H), 7.57 (d, 1H), 7.55 (d, 1H), 7.32 (dd, 1H), 7.25 (d, 1H), 7.01 (dd, 1H), 6.82 (d, 1H), 5.61 (m, 1H), 5.06-4.93 (m, 2H), 3.96 (m, 2H), 3.72 (t, 1H), 3.65 (s, 3H), 2.72 (t, 2H), 2.56 (t, 4H), 2.45 (m, 2H), 1.9 (m, 4H) MS *(m*/*z):* 513[MH]⁺+2Cl.

### Preparation 18: Acetic acid 2-(5-[3-(3,4-dichloro-phenyl)-5-hydroxy-2-oxo-pyrrolidine-1-yl]-2-methoxy-phenoxy)-ethyl ester

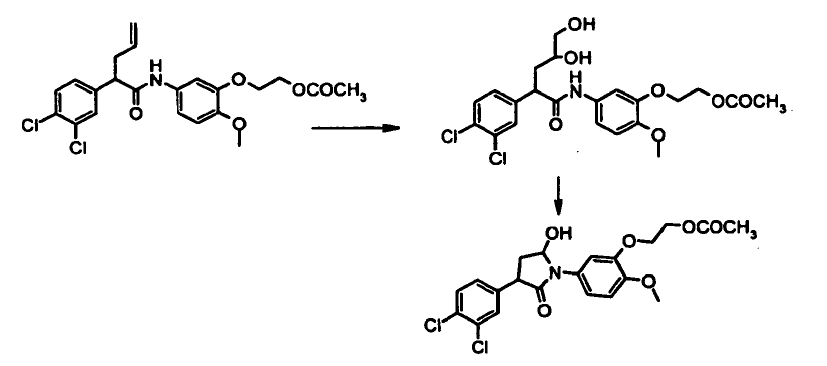

**Procedure:** To a solution acetic acid 2-(5-[2-(3,4-dichloro-phenyl)-pent-4-enoylamine]-2-methoxy-phenoxy)-ethyl ester (950 mg, 2.1 mmol) in acetone/H₂O 8/1 (37.5/4.6 ml) was added N-methyl-morpholine-N-oxide (2eq, 493 mg) and OsO₄ 4wt% sol. in water (cat, 1 ml). The reaction was stirred at room temperature for 18 hours and then quenched with 40ml of Na₂SO₃ sat. After 15 minutes stirring the diol was extracted with ethyl acetate (2x20ml), dried over Na₂SO₄, filtered and concentrated to dryness *in vacuo.* The crude product was then dissolved in THF/H₂O 1/1 (20/20ml) and potassium periodate (1.5eq, 673.7mg) was added. The reaction was stirred at room temperature for 2 hours. The solution was diluted with water (10ml) and extracted with ethyl acetate (3x10ml). The combined organic extracts were dried over anhydrous Na₂SO₄ filtered and concentrated to dryness *in vacuo.* Flash chromatography of the crude product (silica gel, cHex/AcOEt 5/15) gave 170mg of one diastereoisomer of the title product, 227 mg of the other diastereoisomer, and 236 mg of a mixture of both as a white foam (yield: 66% in 2 steps)
(51-1)
**NMR (¹H, DMSO-d6):** δ 7.61 (d, 1H), 7.60 (d, 1H), 7.32 (dd. 1H), 7.26 (d, 1H), 7.11 (dd, 1H), 6.98 (d, 1H), 6.44 (d, 1H), 5.60 (t, 1H), 4.31 (t, 2H), 4.18 (t, 2H), 4.13 (t, 1H), 3.75 (s, 3H), 2.5-2.3 (m, 2H), 2.03 (s, 3H).
**MS *(m*/*z):*** 454 [MH] +2Cl.
(51-3)
**NMR (¹H, DMSO-d6):** δ 7.68 (d, 1H), 7.63 (d, 1H), 7.41 (dd, 1H), 7.12 (d, 1H), 6.89 (m, 2H), 6.52 (d, 1H), 5.66 (m, 1H), 4.31 (m, 2H), 4.13 (m, 2H), 3.87 (dd, 1H), 3.75 (s, 3H), 2.9 (m, 2H), 2.02 (s, 3H), 1.9 (m, 1H).

### Preparation 19: Acetic acid 2-(5-[3-(3,4-dichloro-phenyl)-2-oxo-pyrrolidine-1-yl]-2-methoxy-phenoxy)-ethyl ester

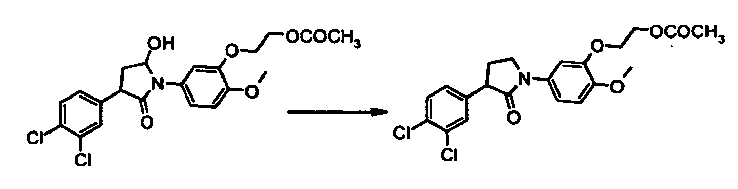

**Procedure:** To a solution of acetic acid 2-(5-[3-(3,4-dichloro-phenyl)-5-hydroxy-2-oxo-pyrrolidine-1-yl]-2-methoxy-phenoxy)-ethyl ester (46 mg, 0.101 mmol) and triethylsilane (1.5eq, 24µl) in CH₂Cl₂ (1ml) was added trifluoroacetic acid (10eq, 120 µl) dropwise. After stirring for 2 hours at room temperature , the solution was concentrated in vacuo, and then the solution was diluted with water (10ml) and extracted with ethylacetate (3x10ml). The combined organic exacts were dried over anhydrous Na₂SO₄, filtered and concentrated to dryness *in vacuo.* The crude product was purified by trituration in ether/petroleum ether 1/1 to give 30mg of the title product as a white solid (yield: 69%) (mp: 113°C)
**NMR (¹H, DMSO-d6):** δ 7.62 (d, 1H), 7.61 (d, 1H), 7.47 (d, 1H), 7.33 (dd, 1H), 7.09 (dd, 1H), 6.98 (d, 1H), 4.30 (t, 2H), 4.14 (t, 2H), 3.99 (t, 1 H), 3.86 (m, 2H), 3.74 (s, 3H), 2.55-2.2 (m+m, 1H+1H), 2.02 (s, 3H). **MS (*m*/*z*):** 375 [MH]⁺, 2Cl.

### Preparation 20: 3-(3,4-Dichloro-phenyl)-1-[3-(2-hydroxy-ethoxy)-4-methoxy-phenyl]-pyrrolidine-2-one

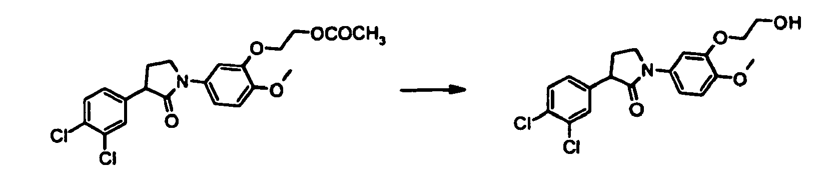

**Procedure:** To a solution of acetic acid 2-(5-[3-(3,4-dichloro-phenyl)-2-oxo-pyrrolidine-1-yl]-2-methoxy-phenoxy)-ethyl ester (524 mg, 1.19 mmol) in EtOH 95% (10ml), at r.t, was added LiOH.H₂O (2 eq., 100mg). After stirring for 2 hours at room temperature, the solution was concentrated *in vacuo,* and then diluted with water (10ml) and extracted with ethylacetate (3x10ml). The combined organic extracts were dried over anhydrous Na₂SO₄, filtered and concentrated to dryness *in vacuo.* to give 470mg of the title product (yield: 99%).
**NMR (¹H, OMSO-d6):** δ 7.62 (s, 1H), 7.61 (d, 1H), 7.47 (d, 1H), 7.33 (dd, 1H), 7.05 (dd, 1H), 6.96 (d, 1H), 4.82 (t, 1H), 3.99 (t, 1H), 3.92 (t, 2H), 3.86 (m, 2H), 3.70 (t, 2H), 3.74 (s, 3H), 2.55-2.22 (m, 2H). **MS *(m*/*z):*** 396 [MH]⁺+Cl

### Preparation 21: 2-(3-Fluoro-phenyl)-pent-4-enoic acid [3-(2-hydroxy-ethoxy)-4-methoxy-phenyl]-amide

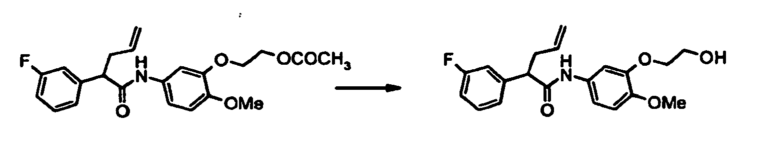

**Procedure:** To a solution of a compound of Preparation 15 (0.15 g, 0.37 mmol) in MeOH (1 mL) and THF (1 mL) at 45 °C was added an aqueous solution of KOH (5 M, 0.5 mL). After 15 min the mixture was cooled to 25 °C. Aqueous HCl (1 M, 3 mL) and EtOAc were added. The organic layer was collected, dried (MgSO₄), filtered and evaporated *in vacuo* to give the title compound (0.13 g, 0.36 mmol, 99%) as a colourless solid.
**NMR (¹H, CDCl₃, 300 MHz): δ** 7.27-7.40 (m, 2H), 6.93-7.15 (m, 4H), 6.71-6.85 (m, 2H), 5.62-5.80 (m, 1H), 4.98-5.11 (m, 2H), 4.05-4.11 (m, 2H), 3.86-3.90 (m, 2H), 3.80 (s, 3H), 3.50 (t, 1H), 2.90-3.02 (m, 1H), 2.46-2.60 (m, 1H), 1.91 (bs, 1H+H₂O). **MS (*m*/*z*)**: 360 [MH]⁺.

### Preparation 22: Methansulfonic acid 2-(5-[3-(3,4-dichloro-phenyl)-2-oxo-pyrrolidin-1-yl]-2-methoxy-phenoxy)-ethyl ether

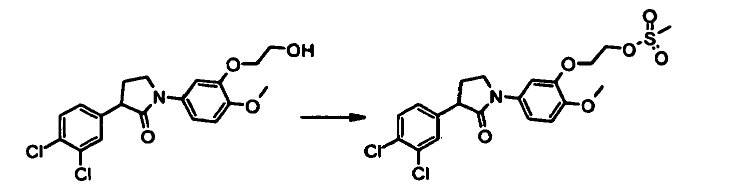

**Procedure**: To a solution of alcohol, 3-(3,4-Dichloro-phenyl)-1-[3-(2-hydroxy-ethoxy)-4-methoxy-phenyl]-pyrrolidine-2-one (470 mg, 1.18 mmol) in anh. CH₂Cl₂ (15.6 ml), at 0°C. under N₂, was added Et₃N (5 eq., 822 µl) and CH₃SO₂Cl (2eq., 180 µl). The reaction was stirred at room temperature for 3 hours. The reaction mixture was diluted with water (20 ml) and extracted with AcOEt (3x25 ml). The combined organic extracts were dried over Na₂SO₄, filtered and concentrated *in vacuo* concentrated to dryness *in vacuo.* to give 560 mg of the title compound as a light yellow foam (yield: 99%).
**NMR (¹H, DMSO-d6)**: δ 7.62 (m, 2H), 7.47 (d, 1H), 7.33 (dd, 1H), 7.13 (dd, 1H), 7.00 (d, 1H), 4.52 (m, 2H), 4.00 (m, 1H), 3.87 (m, 2H), 3.76 (s, 3H), 3.24 (s, 3H), 2.53 (m, 1H), 2.21 (m, 1H). **MS (*m*/*z*)*****:*** 474 [MH]⁺+Cl

### Preparation 23: Methanesulfonic acid 2-(5-[2-(3-fluoro-phenyl)-pent-4-enoylamino]-2-methoxy-phenoxy) ethyl ester

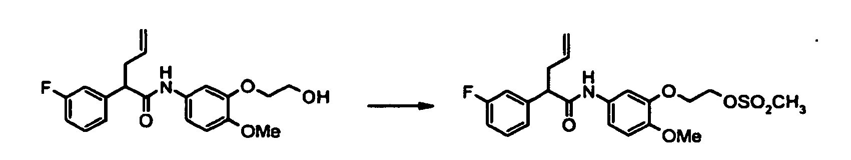

**Procedure:** To a partial solution of compound of Preparation 21 (0.13 g, 0.36 mmol) in DCM (2 mL) and THF (2 mL) was added N,N-diisopropylethylamine (0.13 mL) followed by methanesulfonyl chloride (0.037 mL) resulting in complete dissolution of the starting material. After 1.5 h water was added with stirring. After an additional 3 min the mixture was partitioned between aqueous HCl (1 M) and a 1:1 mixture of EtOAc and petroleum ether (40-60 °C). The organic layer was washed (water, brine) and the solvents removed *in vacuo* to give the title product as a slightly yellow film (0.18 g, quant.) containing residual ethyl acetate.
**NMR (¹H, CDCl₃, 300 MHz):** δ 7.26-7.37 (m, 2H), 7.04-7.16 (m, 3H), 6.94-7.02 (m, 1H), 6.73-6.84 (m, 2H), 5.65-5.80 (m, 1H), 4.97-5.11 (m, 2H), 4.53-4.58 (m, 2H), 4.21-4.26 (m, 2H), 3.77 (s, 3H), 3.50 (t, 1H), 3.13 (s, 3H), 2.87-3.00 (m, 1H), 2.48-2.57 (m, 1H). **MS (*mlz*)*****:*** 438 [MH]⁺.

### Preparation 24: 2-(3-fluoro-phenyl)-pent-4-enoic acid [4-methoxy-3-(2-piperidin-1-yl-ethoxy)-phenyl]-amide

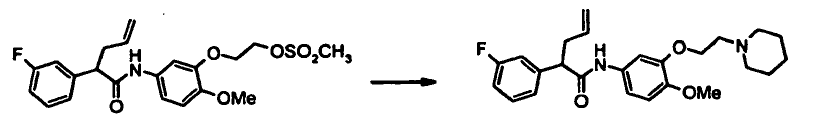

**NMR (¹H, CDCl₃):** δ 7.25-7.35 (m. 1H), ), 7.10-7.20 (m, 3H), 6.85-7.00 (m, 2H), 6.75 (d, 1H), 5.6-5.8 (m, 1H), 4.9-5.15 (m, 2H), 4.1-4.2 (m, 3H), 3.80 (s, 3H), 3.5 (t, 1H), 2.90-3.05 (m, 2H), ), 2.6-2.75 (m, 2H), 2.5-2.6 (m, 1H), 1.95-2.25 (m, 2H), 1.65-1.85 (m, 4H), 1.4-1.6 (m, 2H). **MS (*m*/*z*):** 427 [MH]⁺.

### Preparation 25: 4,4-Difluoro-1-[2-(2-methoxy-4-nitro-phenoxy)-ethyl]-piperidine

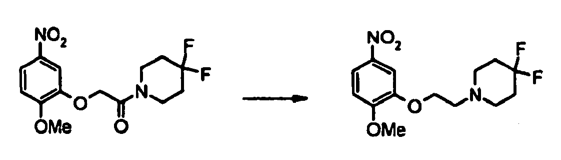

**Procedure**: 1-(4,4-Difluoro-piperidine-1-yl)-2-(2-methoxy-4-nitro-phenoxy)-ethanone (320 mg, 0.96 mmol) was dissolved in anh. THF (20 ml), 1M BH₃.THF (2.2eq, 2.4 ml) was added dropwise to the solution, and the mixture was heated at reflux for 5 hours. The solution was cooled to room temperature, CH₃OH (10 ml) was added, and the solvent was removed under reduced pressure. The residue was dissolved in CH₃OH (10 ml), 6N HCl (20ml) was added, and the solution was heated to reflux for 1 hour. The CH₃OH was removed under reduced pressure and the remaining aqueous solution was made basic (pH>10) with 2.5N NaOH. The basic solution was extracted with ethyl acetate (3x20ml). The combined organic extracts were dried over anhydrous Na₂SO₄ and the solvent was removed under reduced pressure to give 258 mg of the title product as a light yellow oil. (yield: 85%)
**NMR (¹H, CDCl₃)**: δ 7.91 (dd, 1H), 7.77 (d, 1H), 6.89 (d, 1H), 4.19 (t, 2H), 2.90 (t, 2H), 2.71 (m, 4H), 2.01 (m, 4H).

### Preparation 26: 4-[2-(4,4-Difluoro-piperidin-1-yl)-ethoxy]-3-methoxy-phenylamine

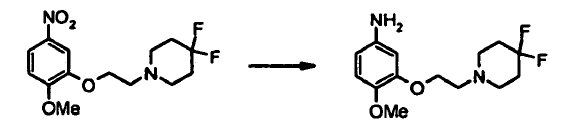

**NMR (¹H, CDCl₃):** δ 6.73 (d, 1H), 6.34 (d, 1H), 6.29 (dd, 1H), 4.18 (t, 2H), 3.78 (s, 3H), 3.02 (t, 2H), 2.89 (bs, 4H), 2.13 (m, 4H).

### Preparation 27: 4-Methoxy-3-(2-pyrrolidin-1-yl-ethoxy)-phenylamine

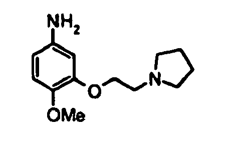

The title compound (brown solid) was prepared in an analogous manner to Preparations 9 and 11 using 2-methoxy-5-nitrophenol and 1-(2-chloroethyl)pyrrolidine hydrochloride.
**NMR (¹H, CDCl₃):** δ 6.70 (d, 1H), 6.30 (s, 1H), 6.20 (d, 1H), 4.08 (t, 2H), 3.75 (s, 3H), 3.40 (bs, 2H), 2.90 (t, 2H), 2.65-2.55 (m, 4H), 1.80-1.74 (m, 4H). MS (*m*/*z*): 237 [MH]⁺.

### Preparation 28: 2-(3,4-Dichloro-phenyl)-pent-4-enoic-acid [4-methoxy-3-(2-pyrrolidin-1-yl-ethoxy)-phenyl]-amide

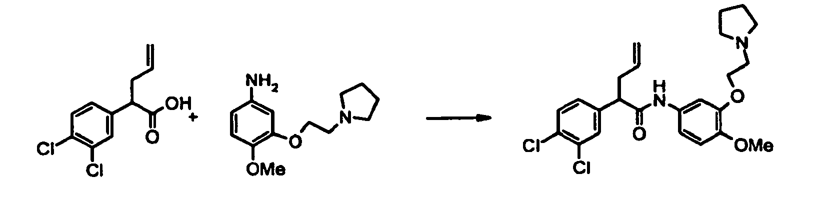

To a solution of 2-(3,4-dichloro-phenyl)-pent-4-enoic acid (0.19 g) in dry CH₂Cl₂ (3 ml), under N₂, was added, at 0°C, oxalyl chloride (0.10 ml) and DMF (cat). The reaction was allowed to warm to room temperature. After 1 h the reaction mixture was concentrated to dryness *in vacuo.* To this material was added a DMF (dry, 2 ml) solution of 4-methoxy-3-(2-pyrrolidin-1-yl-ethoxy)-phenylamine (0.22 g) which had been converted to the hydrochloride salt by treatment with HCl/Et₂O (1 eq.) followed by evaporation. The mixture was heated at 110 °C for 2 h, then partitioned between aqueous NaHCO₃ and EtOAc. The organic layer was washed (brine), concentrated and purified by column chromatography (silica gel, CH₂Cl₂ / MeOH / NH₃) to give the title compound (0.18 g) as an orange oil.
**NMR (¹H, CDCl₃):** δ 7.50-7.35 (m, 3H), 7.15 (s, 1H), 6.90 (d, 1H), 6.73 (d, 1H), 5.77-5.62 (m, 1H), 5.10-4.96 (m, 2H), 4.15-4.05 (m, 2H), 3.80 (s, 3H), 3.46 (t, 1H), 2.96-2.83 (m, 3H), 2.70-2.54 (m, 4H), 2.40 (bs, 2H), 1.85-1.73 (m, 4H). **MS (*m*/*z*):** 463 [MH]⁺, 2Cl.

### Preparation 29: 3-(3,4-Dichloro-phenyl)-5-hydroxy-1-[4-methoxy-3-(2-pyrrolidin-1-yl-ethoxy)-phenyl]-pyrrolidin-2-one

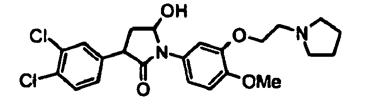

The title compound (colourless foam, ca. 1:1 mixture of diastereoisomers) was prepared from 2-(3,4-dichloro-phenyl)-pent-4-enoic-acid [4-methoxy-3-(2-pyrrolidin-1-yl-ethoxy)-phenyl]-amide in an analogous manner to the one described for Example 15.
**NMR (¹H, CDCl₃):** δ 7.44 (s, 0.5H), 7.28-7.12 (m, 2H), 7.06 (s, 0.5H), 6.95-6.80 (m, 2H), 6.68 (d, 1H), 5.54-5.44 (m, 1H), 3.96-3.85 (m, 3H), 3.67 (s, 3H), 3.60-3.53 (m, 0.5H), 3.78-3.70 (m, 2.5H), 2.47-2.18 (m, 5.5H), 2.00-1.91 (m, 0.5H), 1.67-1.55 (m, 4H). **MS (*m*/*z*):** 465 [MH]⁺, 2Cl.

### Preparation 30: [4-Methoxy-3-(2-piperidin-1-yl-ethoxy)-phenyl]-acetic acid 2-piperidin-1-yl-ethyl ester

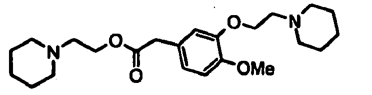

A mixture of (3-hydroxy-4-methoxy-phenyl)-acetic acid (1.85 g), dry DMF (25 ml), K₂CO₃ (5.9 g) and *N*-chloroethylpiperidine hydrochloride (3.74 g) was heated at 40 °C for 5 h. Volatiles were then removed *in vacuo* and the residue partitioned between water and EtOAc. The organic layer was washed (brine) and concentrated to give the title (3.76 g) compound as an orange oil.
**NMR (¹H, CDCl₃):** δ 6.93-6.80 (m, 3H), 4.22 (t, 2H), 4.14 (t, 2H), 3.82 (s, 3H), 3.55 (s, 2H), 2.82 (t, 2H), 2.66-2.40 (m, 10H), 1.66-1.40 (m, 12H).

### Preparation 31: [4-Methoxy-3-(2-piperidin-1-yl-ethoxy)-phenyl]-acetic acid methyl ester

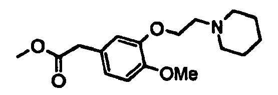

[4-Methoxy-3-(2-piperidin-1-yl-ethoxy)-phenyl]-acetic acid 2-piperidin-1-yl-ethyl ester (6.3 g) in MeOH (6 ml), THF (6 ml) and water (6 ml) containing KOH (1.7 g) was heated at 45 °C for 1 h and then allowed to cool to 25 °C over 90 min. With stirring in an ice bath conc. aqueous HCl (6 ml) was then added. The mixture was evaporated to dryness. The material was heated at reflux with HCl in MeOH (1 M) for 4 h, concentrated and extracted with DCM. The mixture was filtered and the solvent removed *in vacuo* to give the title compound (4.4 g) as an orange oil.
**NMR (¹H, CDCl₃):** δ 6.88-6.79 (m, 3H), 4.18 (t, 2H), 3.84 (s, 3H), 3.69 (s, 3H), 3.55 (s, 2H), 2.87 (t, 2H), 2.56 (bs, 4H), 1.66 (bs. 4H), 1.46 (bs, 2H).

### Preparation 32: 2-[4-Methoxy-3-(2-piperidin-1-yl-ethoxy)-phenyl]-pent-4-enoic acid methyl ester

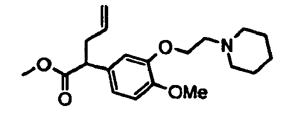

**Procedure:** To a solution of [4-methoxy-3-(2-piperidin-1-yl-ethoxy)-phenyl]-acetic acid methyl ester (2.1 g) in THF (dry, 15 ml) at -78 °C was slowly added lithium bis(trimethylsilyl)amide (1 M in THF, 8.2 ml). The solution was stirred at this temperature for 15 min before allyl bromide (0.59 ml) was added. After additional 30 min water and EtOAc were added with stirring. The mixture was allowed to warm to 25 °C, layers separated and the organic layer washed (brine), concentrated and submitted to column chromatography (silica gel, CH₂Cl₂ / MeOH / NH₃) to give the title compound (1.3 g) as a colourless oil.
**NMR (¹H, COCl₃):** δ 6.90-6.80 (m, 3H), 5.76-5.67 (m, 1H), 5.10-4.98 (m, 2H), 4.18 (bs, 2H), 3.83 (s, 3H), 3.65 (s, 3H), 3.56 (t, 1H), 2.86 (bs, 2H), 2.83-2.73 (m, 1H), 2.65-2.45 (m, 5H), 1.65 (bs, 4H), 1.47 (bs, 2H). **MS (*m*/*z*):** 348 [MH]⁺.

### Preparation 33: 2-[4-Methoxy-3-(2-piperidin-1-yl-ethoxy)-phenyl]-pent-4-enoic acid hydrochloride salt

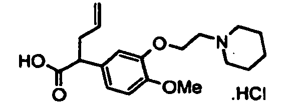

2-[4-Methoxy-3-(2-piperidin-1-yl-ethoxy)-phenyl]-pent-4-enoic acid methyl ester (1.3 g) in MeOH (2 ml), THF (2 ml) and water (2 ml) containing KOH (0.42 g) was heated at 45 °C for 1 h and then allowed to cool to 25 °C. The mixture was evaporated to dryness. THF (5 ml) and conc. aqueous HCl (0.62 ml) were added, the mixture concentrated, extracted with DCM, filtered and the solvent removed *in vacuo* to give the title compound (1.2 g) as an off-white foam.
**NMR (¹H, CD₃OD):** δ 7.05 (d, 1H), 6.96 (dd, 1H), 6.91 (d, 1H), 5.81-5.70 (m, 1H), 5.02 (dd, 1H), 4.92 (dd, 1H), 4.31 (dd, 2H), 3.83 (s, 3H), 3.48-3.42 (m, 3H), 3.37-3.28 (m, 4H), 2.78-2.69 (m, 1H), 2.44-2.35 (m, 1H), 1.91-1.84 (m, 4H), 1.72-1.63 (m, 2H). **MS *(mlz)*:** 334 [MH]⁺.

### Preparation 34: 2-[4-Methoxy-3-(2-piperidin-1-yl-ethoxy)-phenyl]-pent-4-enoic acid (3,4-dichlorophenyl)-amide

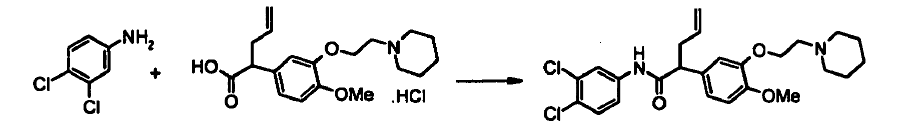

To a solution of 2-[4-methoxy-3-(2-piperidin-1-yl-ethoxy)-phenyl]-pent-4-enoic acid hydrochloride salt (0.46 g) in dry CH₂Cl₂ (4 ml), under N₂, was added, at 0°C, oxalyl chloride (0.11 ml) and DMF (cat). After 30 min the reaction mixture was concentrated to dryness *in vacuo.* To this material was added toluene (dry, 4 ml) and 3,4-dichloroaniline (0.20 g). The mixture was heated at 105 °C for 4 h, then partitioned between aqueous NaHCO₃ and EtOAc. The organic layer was washed (brine), concentrated and purified by column chromatography (silica gel, CH₂Cl₂ / MeOH / NH₃) to give the title compound (0.35 g) as a slightly brown oil.
**NMR (¹H, CDCl₃):** δ 7.74 (s, 1H), 7.55 (bs, 1H), 7.31 (bs, 2H), 6.96 (bs, 1H), 6.91-6.83 (m, 2H), 5.77-5.68 (m, 1H), 5.09 (d, 1H), 5.01 (d, 1H), 4.23-4.16 (m, 2H), 3.85 (s, 3H), 3.53 (t, 1H), 2.99-2.91 (m, 1H), 2.83 (t, 2H), 2.55 (bs, 4H), 1.68-1.62 (m, 4H), 1.47 (bs, 3H). **MS (*m*/*z*):** 477 [MH]⁺, 2Cl.

### Preparation 35: 2-[4-Methoxy-3-(2-piperidin-1-yl-ethoxy)-phenyl]-propanoic acid methyl ester

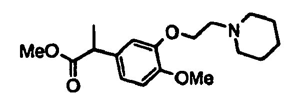

To a solution of [4-methoxy-3-(2-piperidin-1-yl-ethoxy)-phenyl]-acetic acid methyl ester (0.60 g) in THF (dry, 6 ml) at -78 °C was slowly added lithium bis(trimethylsilyl)amide (1 M in THF, 2.3 ml). The solution was stirred at this temperature for 15 min before iodomethane (0.12 ml) was added, then allowed to warm to 25 °C. After 16 h aqueous NaHCO₃ and EtOAc were added with stirring, layers separated and the organic layer washed (brine), concentrated and submitted to column chromatography (silica gel, CH₂Cl₂ / MeOH / NH₃) to give the title compound (0.27 g) as a slightly yellow oil.
**NMR (¹H, CD₃OD):** δ 6.88-6.75 (m, 3H), 4.12 (t, 2H), 3.80 (s, 3H), 3.67-3.34 (m, 4H), 2.78 (t, 2H), 2.50 (bs, 4H), 1.65-1.37 (m, 9H). **MS *(m*/*****z):*** 322 [MH]⁺.

### Preparation 36: 2-(3-Chloro-phenyl)-pent-4-enoic acid (3-[2-(4-methyl-piperidin-1-yl)-ethoxy]-4-methoxy-phenyl)-amide

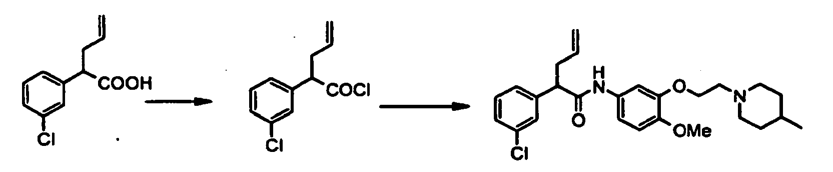

The title compound was prepared, in an analogous manner to the compound of Preparation 14, in 317 mg yield of brow oil (y= 73%) from 2-(3,4-dichloro-phenyl)-pent-4-enoic acid (200 mg).
**MS *(m*/*****z):*** 457 [MH]⁺, 1Cl.

### Preparation 37: 3-(3-Chloro-phenyl)-5-hydroxy-1-(4-methoxy-3-[2-(4-methyl-piperidin-1-yl)-ethoxy]-phenyl}-pyrrolidin-2-one

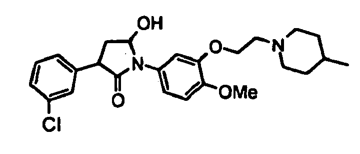

The title compound was prepared, in an analogous manner to the compound of Example 15, in 176 mg yield as brow oil (y= 55%) from 2-(3-chloro-phenyl)-pent-4-enoic acid {4-methoxy-3-[2-(4-methylpiperidin)-1-yl-ethoxy]-phenyl}-amide (317 mg).
**MS (*m*/*z*):** 459 [MH]⁺, 1 Cl.

### Preparations 38: 5-(tert-Butyl-dimethyl-silanyloxy)-2-(3,4-dichloro-phenyl)-pentanoic acid

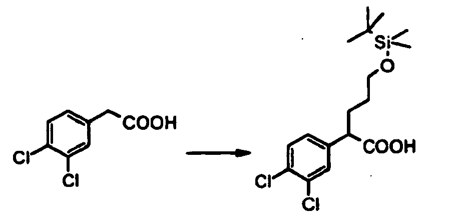

**Procedure**: A solution of (3,4-Dichloro-phenyl)-acetic acid (1.5 g, 7.3 mmol) in anh. THF (15 ml), under N₂, was treated with lithium bis(trimethylsilyl) amide (1M solution in THF, 2.2 eq., 16 ml) at -78°C for 30 minutes before (3-bromo-propoxy)-tert butyl-dimethyl-silane (1.7 eq., 2.9 ml) was added. The mixture was stirred for 2 hours at room temperature and quenched with saturated solution of Ammonium Chloride (20 ml). The product was extracted with ethyl acetate (2x 15 ml) and the organic phase was washed with brine (1x15 ml), dried over Na₂SO₄ and concentrated *in vacuo.* The crude product was purified by flash chromatography (silica gel, AcOEt/cHex 1/1 then AcOEt) to give 16 g of the title product as a pale yellow oil (56%).
**NMR (¹H, DMSO-d6)**: δ 12.6 (sb, 1H), 7.58 (d, 1H), 7.53 (d, 1H), 7.27 (dd, 1H), 3.59 (t, 1H), 3.54 (t, 2H), 2.00 (m, 1H), 1.7 (m, 1H), 1.4 (m, 1H), 1.3 (m, 1H), 0.82 (s, 9H), - 0.01 (s, 3H), - 0.02 (s, 3H).

### Preparation 39: 5-(tert-Butyl-dimethyl-silanyloxy)-2-(3,4-dichloro-phenyl)- pentanoic acid [4-methoxy-3-(2-piperidin-1-yl-ethoxy)-pheny]-amide

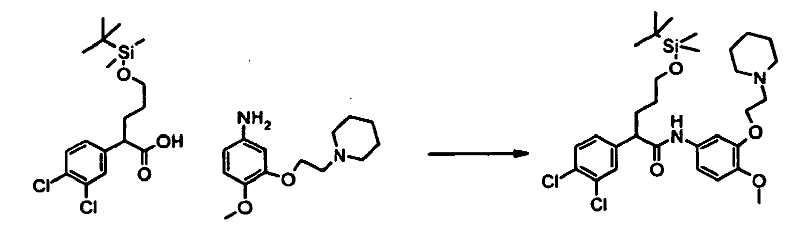

**Procedure:** To a solution of 5-(*tert*-butyl-dimethy-silanyloxy)-2-(3,4-dichloro-phenyl)-pentanoic acid (780 mg, 2.1 mmol) in anh. DMF (5 ml), under N₂, was added 4-methoxy-3-(2-piperidin-1-yl-ethoxy)-phenylamine (1 eq., 523 mg) followed by 1-hydroxybenzotriazole (1.2 eq., 360 mg) and 1-(dimethylaminopropyl)-3-ethylcarbodiimide hydrochloride (1.2 eq., 476 mg). The reaction was stirred at room temperature for 16 h. AcOEt was added (10 ml) and the organic phase was washed with H₂O (10 ml), NaHCO₃ sat. (10 ml) brine (10ml), dried over Na₂SO₄ and concentrated *in vacuo* to obtain the title product as a pale yellow foam 1.25 g (98%).
**NMR (¹H, CDCl₃):** δ 7.75 (s, 1H), 7.45 (s, 1H), 7.3 (d, 1 H), 7.2 (m, 1H), 7.00 (d, 1H), 6.7 (d, 1H), 4.1 (m, 2H), 3.75 (s, 2H), 3.5 (m, 2H), 2.6 (m, 4H), 1.6-1.3 (m, 8H), 0.9 (s, 9H), 0.5 (s, 6H)

### Preparation 40: 2-(3,4-dichloro-phenyl)-5-hydroxy pentanoic acid [4-methoxy-3-(2-piperidin-1-yl-ethoxy)-phenyl]-amide

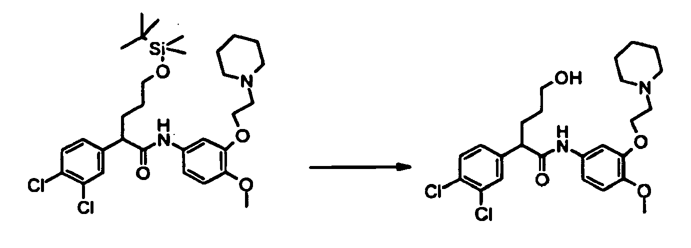

**Procedure:** To a solution of 5-(*tert-*butyl-dimethyl-silanyloxy)-2-(3,4-dichloro-phenyl)-pentanoic acid [4-methoxy-3-(2-piperidin-1-yl-ethoxy)-phenyl]-amide (780 mg, 2.1 mmol) in anh. DMF (2 ml), under N₂, triethylamine trihydrofluoride (0.5 eq., 0.17 ml) was added. The reaction was stirred at room temperature for 18 h. AcOEt was added (10 ml) and the organic phase was washed with NaHCO₃ sat. (10 ml), dried over Na₂SO₄ and concentrated *in vacuo* to obtain the title product as a white foam 1 g (100%).
**NMR (¹H, DMSO-d6)**: δ 9.98 (s, 1H), 7.6 (m, 2H), 7.34 (d, 1H), 7.29 (d, 1H), 7.07 (d, 1H), 7.2 (m, 1H), 6.85 (d, 1H), 4.42 (t, 1 H), 3.98 (t, 2H), 3.69 (s, 3H), 3.64 (t, 1H), 3.39 (q, 2H), 2.66 (bs, 2H), 2.46 (bm, 4H), 2.0 (m, 1H), 1.7 (m, 1H), 1.49 (bm, 4H), 1.37 (m, 2H), 1.33 (m, 2H). **MS (*m*/*z*):** 495 [MH]⁺,2Cl.

### Preparation 41: 2-Chloro-1-(4-metyl-piperidin-1-yl)-ethanone

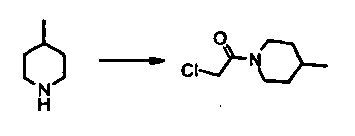

**Procedure:** To a solution of 4-metylpiperidine (10 g, 100 mmol) in anh. CH₂Cl₂ (100 ml) was added, at 0° C and under N₂. TEA (2.5 eq, 35 ml) and chloroacetyl chloride (1.2eq, 9.6 ml). The reaction was stirred at room temperature for 6 hours. The solution was diluted with water (60 ml) and extracted with ethyl acetate (3x100 ml). The combined organic extracts were dried over anhydrous Na₂SO₄, filtered and concentrated to dryness *in vacuo* to give 13.6 g of the title product (yield: 78%).
**NMR (¹H, CDCl₃):** δ 4.57 (d, 1 H), 4.11 (d, 2H), 3.82 (d, 1 H), 3.12 (t, 1H), 2.67 (t, 1 H), 1.67 (m, 4H), 1.22 (m, 1H), 1.02 (d, 3H). **MS (*m*/*z*):** 176 [MH]⁺, 1Cl

### Preparation 42: 2-(2-Methoxy-5-nitro-phenoxy)-1-(4-methyl-piperidin-1-yl)-ethanone

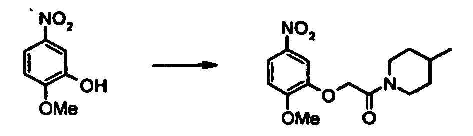

**Procedure:** To a solution of 2-methoxy-5-nitrophenol (350 mg, 2.06 mmol) in DMF (11 ml) at room temperature were added K₂CO₃ (312 mg, 2.2 mmol) and 2-chloro-1-(4-methyl-piperidin-1-yl)-ethanone (396 mg, 2.2 mmol). The suspension was stirred at room temperature for 24 hours. The suspension was diluted with water (20 ml) and extracted with EtOAc (2x20 ml), the combined organic phases were washed with NaOH 1N (3x20 ml), dried over Na₂SO₄, filtered and concentrated to dryness *in vacuo,* to obtain the title product as a red oil.(0.38 g, 58%)
**NMR (¹H, CDCl₃):** δ 8.00 (dd, 1H), 7.76 (d, 1H), 6.98 (d, 1H), 4.87 (s, 3H), 4.55 (d, 1H), 4.02 (s, 3H), 3.85 (d, 1H), 3.13 (t, 1H), 2.68 (t, 1H), 1.68 (m, 2H), 1.27 (m, 2H), 1.01 (d, 3H). **MS (*m*/*z*):** 309 [MH]⁺.

### Preparation 43: 1-[2-(2-Methoxy-5-nitro-phenoxy)-ethyl]-4-methyl-piperidine

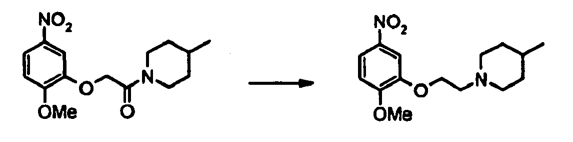

**Procedure:** 2-(2-Methoxy-5-nitro-phenoxy)-1-(4-methyl-piperidin-1-yl)-ethanone (11 g, 36 mmol) was dissolved in anh. THF (20 ml), 1M BH₃.THF (2.2eq, 78.5 ml) was added dropwise to the solution, and the mixture was heated at reflux for 4 hours. The solution was cooled to room temperature, CH₃OH (100 ml) was added, and the solvent was removed under reduced pressure. The residue was dissolved in CH₃OH (100 ml), 6N HCl (200 ml) was added, and the solution was heated to reflux for 0.5 h. The CH₃OH was removed under reduced pressure and the remaining aqueous solution was made basic (pH>10) with 2.5N NaOH. The basic solution was extracted with ethyl acetate (3x80 ml). The combined organic extracts were dried over anhydrous Na₂SO₄ and the solvent was removed under reduced pressure to give 9.4 g of the title product as a red oil. (yield: 89%) **NMR** (¹H, CDCl₃): δ 7.91 (dd, 1H), 7.80 (d, 1H), 6.90 (d, 1H), 4.21 (t, 2H), 3.96 (s, 3H), 2.97 (m, 2H), 2.85 (t, 2H), 2.12 (m, 2H), 1.64 (m, 2H), 1.4 (m, 1H), 1.29 (m, 2H), 0.92 (d, 3H). **MS (*m*/*z*):** 295 [MH]⁺.

### Preparation 44: 4-Methoxy-3-[2-(4-methyl-piperidin-1-yl)-ethoxy]-phenylamine

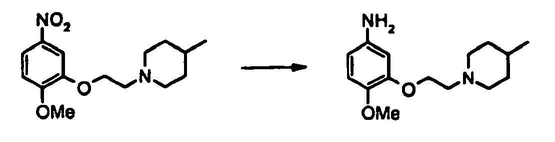

**Procedure:** To a solution of 1-[2-(2-methoxy-5-nitro-phenoxy)-ethyl]-4-methyl-piperidine (9.5 g, 32.3 mmol) in abs.EtOH (200 ml) were added Pd/C 10% (1 g) and the reaction mixture was hydrogenated at room temperature and at atmospheric pressure. After 5 hrs the reaction mixture was filtered through a celite pad and the solution was concentrated *in vacuo* to give 8.5 g of the title product as a brown solid (yield: 99%).
**NMR (¹H, DMSO-d₆):** δ 6.61 (d, 1H), 6.26 (d, 1H), 6.04 (dd, 1H), 4.60 (bs, 2H), 3.91 (t, 2H), 3.58 (s, 3H), 2.85 (m, 2H), 2.61 (t, 2H), 1.97 (m, 2H), 1.52 (m, 2H), 1.3 (m, 1H), 1.1 (m, 2H), 0.86 (d, 3H).

### Preparation 45: 2-(3,4-Dichloro-phenyl)-pent-4-enoic acid (4-methoxy-3-[2-(4-methyl-piperidin-1-yl)-ethoxy]-phenyl)-amide

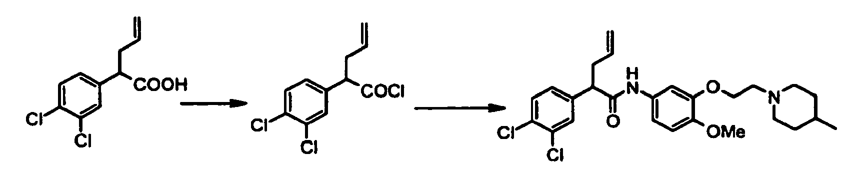

The title compound was prepared in an analogous manner to the compound of preparation 14
**NMR** (¹H, CDCl₃): δ 7.50 (bs, 1H), 7.43 (d, 1H), 7.25 (dd, 1H), 7.21 (bs, 1H), 6.92 (dd, 1H), 6.78 (d, 1 H), 5.74 (m, 1 H), 5.13 (d, 1 H), 5.03 (d, 1 H), 4.14 (t, 2H), 3.81 (s, 3H), 3.48 (t, 1 H), 3.03 (d, 2H), 2.95 (m, 1 H), 2.86 (t, 2H), 2.55 (m, 1 H), 2.18 (t, 2H), 1.65 (d, 2H), 1.4 (m, 1H), 1.32 (t, 1H), 0.94 (d, 3H). **MS (*m*/*z*):** 491[MH]⁺,2Cl.

### Preparation 46: 3-(3,4-Dichloro-phenyl)-5-hydroxy-1-(4-methoxy-3-[2-(4-methyl-piperidin-1-yl)-ethoxy]-phenyl)-pyrrolidin-2-one

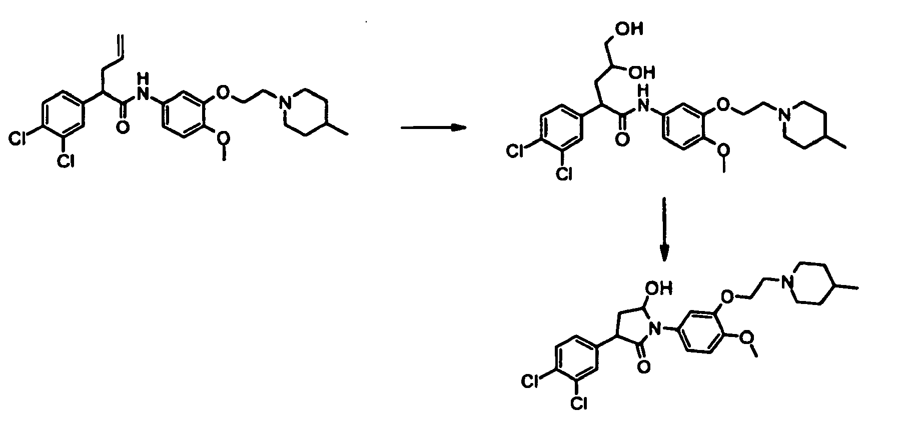

**Procedure** To a solution 2-(3,4-dichloro-phenyl)-pent-4-enoic acid (4-methoxy-3-[2-(4-methyl-piperidin-1-yl)-ethoxy]-phenyl)-amide (0.25 g, 0.51 mmol) in acetone/H₂O 8/1 (9.1/1.1 ml) was added *N*-methyl-morpholine-*N*-oxide (2 eq, 120 mg) and OsO₄ 4wt% sol. in water (cat., 100 µl). The reaction was stirred at room temperature for 18 hours and then quenched with 10 ml of Na₂SO₃ sat. After 15 minutes stirring the diol was extracted with ethyl acetate (2x20 ml), dried over Na₂SO₄, filtered and concentrated to dryness *in vacuo*. The crude product was then dissolved in THF/H₂O 1/1 (5/5 ml) and potassium periodate (1.5 eq, 0.16 g) was added. The reaction was stirred at room temperature for 2 hours. The solution was diluted with water (10 ml) and extracted with ethyl acetate (3x10 ml). The combined organic extracts were dried over anhydrous Na₂SO₄ ,filtered and concentrated to dryness *in vacuo.* Flash chromatography of the crude product (silica gel, CH₂Cl₂/CH₃OH 4/1) gave 0.14 g of the title product as a red oil. (yield: 57% in 2 steps)
**NMR (¹H, DMSO-d6):** δ 7.61 (m, 2H), 7.32 (dd, 1H), 7.25 (bs, 1H), 7.07 (dd, 1H), 6.95 (d, 1H), 6.45 (d, 1H), 5.61 (m, 1H), 4.20 (t, 1H), 4.01 (t, 2H), 3.74 (s, 3H), 2.67 (t, 2H), 2.90 (d, 2H), 2.45 (m, 1H), 2.35 (m, 1H), 2.20 (t, 2H), 1.56 (d, 2H), 1.23 (t, 2H), 1.3 (m, 1H), 0.87 (d, 3H).
**MS (*mlz*):** 493 [MH]⁺, 2Cl.

### Preparation 47: (3,4-Dichloro-phenyl)-acetic acid but-2-enyl ester

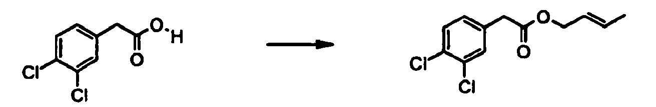

**Procedure:** To a solution of 3,4-dichloro-phenylacetic acid (2 g, 9.7 mmol) in anh. CH₂Cl₂ (40 ml), under N₂, was added, at 0°C, oxalyl chloride (1.9 eq., 1.6 ml) and DMF (cat). The reaction was stirred at room temperature for 3 hours. The reaction mixture was concentrated to dryness *in vacuo* and the crude intermediate was then dissolved, under N₂, in anh. CH₂Cl₂ (40 ml). Crotyl alcohol (2 eq, 1.7 ml) and pyridine (1.1 eq., 1.2 ml) were added to the reaction mixture at room temperature. The reaction was stirred at room temperature for 18 hours and then quenched with ammonium chloride (40 ml). The product was extracted with ethyl acetate (3x25 ml) dried over Na₂SO₄, filtered and concentrated to dryness *in vacuo.* The crude product was purified by flash chromatography (silica gel, AcOEt/cHex 19/1) to give 1.8 g of the title product as a pale yellow oil (72%).
**NMR (¹H, DMSO-d₆):** 7.57 (d, 1H), 7.56 (bs, 1H), 7.28 (dd, 1H), 5.74 (m, 1H), 5.57 (m, 1H), 4.49 (d, 2H), 3.73 (s, 2H), 1.67 (d, 3H).

### Preparation 48: 2-(3,4-Dichloro-phenyl)-3-methyl-pent-4-enoic acid

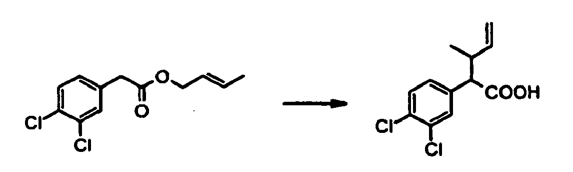

**Procedure:** To a stirred solution of lithium diisopropylamide (LDA) (1.8 ml, 3.5 mmol) in anh. THF (2 ml) was added *via* a syringe, a solution of the ester (3,4-dichloro-phenyl)-acetic acid but-2-enyl ester (0.38 g, 1.43 mmol) in THF (2 ml). After 30 min the solution was treated with trimethylsilyl chloride (0.45 mmol, 2.5 ml) and after a further 30 min at - 78°C, the mixture was warmed to ambient temperature during 30 min which was stirred to and heated at reflux for 2 hrs. It was then cooled to ambient temperature before the addition of water (5 ml) and acidification to pH 2 using HCl 1M. The product was extracted into ethyl acetate (3x10 ml). The combined organic extracts were dried over anhydrous Na₂SO₄ and the solvent was removed under reduced pressure. Flash chromatography of the crude product (cHex/EA 4/1 to 100% EA) gave 0.14 g of the title product as a brown oil. (yield: 38%) which is a mixture of diastereoisomers A/B 50/50.
**NMR (¹H, DMSO-d₆):** 12.62 (bs, 1H), 7.55 (m, 2H), 7.3 (dd, 1H), 5.81 (m, 1H_{A}), 5.45 (m, 1H_{B}), 5.01 (dd, 2H_{A}), 4.81 (dd, 2H_{B}), 3.43 (d, 1H), 2.78 (m, 1H), 1.06 (d, 3H_{A}), 0.74 (d, 3H_{B}).

### Preparation 49: 2-(3,4-Dichloro-phenyl)-3-methyl-pent-4-enoic acid (4-methoxy-3-[2-(4-piperidin-1-yl)-ethoxy]-phenyl)-amide

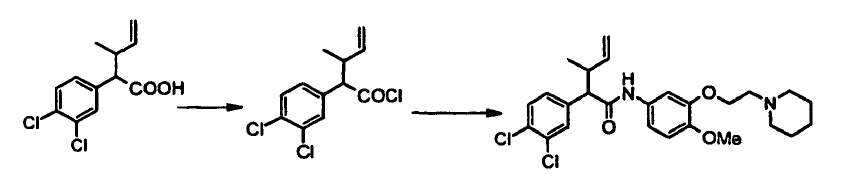

The title compound was prepared in an analogous manner to the compound of Preparation 14 mixture of diastereoisomers: A/B 60/40

**NMR (¹H, DMSO-d₆):** δ 10.05 (s, 1H_{B}), 9.94 (s, 1H_{A}), 7.65-7.50 (m, 2H), 7.38 (dd, 1H_{A}), 7.34 (dd, 1H_{B}), 7.27 (bs, 1H_{B}), 7.23 (bs, 1H_{A}), 7.06 (dd, 1H_{B}), 7.04 (dd, 1H_{A}), 6.85 (m, 1H), 5.84 (m, 1H_{A}), 5.50 (m, 1H_{B}), 5.10-5.00 (m, 2H_{A}), 4.88-4.82 (m, 2H_{B}), 3.97 (bs, 2H), 3.69 (s, 3H_{B}), 3.68 (s, 3H_{A}), 3.43 (m, 1H), 2.90 (m, 1H), 2.63 (bs, 2H), 2.41 (bs, 4H), 1.48 (bs, 4H), 2.41 (bs, 2H), 1.07 (3H_{B}), 0.76 (3H_{A}). **MS (*m*/*z*)**: 491 [MH]⁺,2Cl

### Preparation 50: 2-(4-Chloro-phenyl)-pent-4-enoic acid (4-methoxy-3-[2-(4-methyl-piperidin-1-yl)-ethoxy]-phenyl)-amide

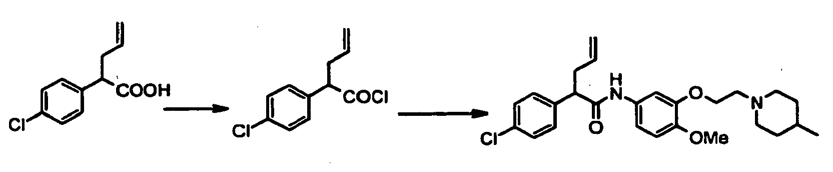

The title compound was prepared in an analogous manner to the compound of Preparation 14
**NMR (¹H, DMSO-d₆):** δ 9.94 (s, 1H), 7.38 (m, 4H), 7.26 (d, 1H), 7.05 (dd, 1H), 6.84 (d, 1H), 5.68 (m, 1H), 5.0 (dd, 2H), 3.95 (t, 2H), 3.70 (t, 1H), 3.68 (s, 3H), 2.63 (t, 2H), 2.55 (m, 2H), 2.85 (m, 2H), 1.97 (m, 2H), 1.53-1.08 (m, 4H), 1.3 (m, 1H), 0.86 (d, 3H). **MS (*m*/*z*)**: 457 [MH]⁺, 1Cl.

### Preparation 51: 2-(4-Chloro-phenyl)-pent-4-enoic acid (4-methoxy-3-[2-(4-piperidin-1-yl)-ethoxy]-phenyl)-amide

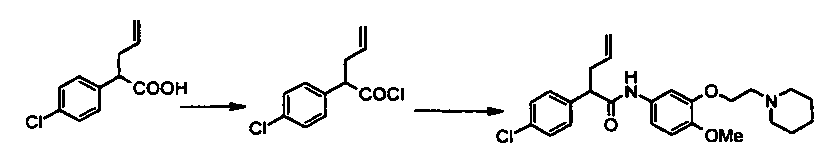

The title compound was prepared in an analogous manner to the compound of Preparation 14
**NMR (¹H, DMSO-d₆):** δ 9.95 (s, 1H), 7.38 (m, 4H), 7.26 (d, 1H), 7.05 (dd, 1H), 6.84 (d, 1H), 5.7 (m, 1H), 5.0 (dd, 2H), 3.95 (t, 2H), 3.70 (t, 1H), 3.68 (s, 3H), 2.61 (t, 2H), 2.5 (m, 2H), 2.40 (m, 4H), 1.53-1.3 (m, 6H). **MS (*mlz*):** 443 [MH]⁺, 1Cl.

### Preparation 52: 3-(4-Chloro-phenyl)-5-hydroxy-1-(4-methoxy-3-[2-(4-methyl-piperidin-1-yl)-ethoxy]-phenyl)-pyrrolidin-2-one

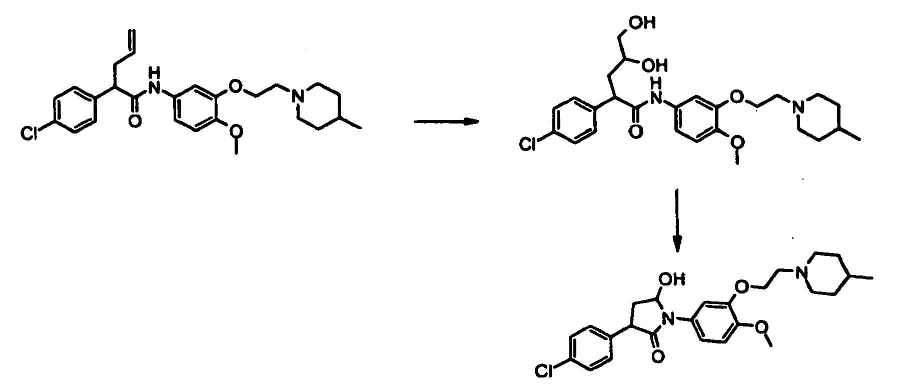

The title compound was prepared in an analogous manner to the compound of Preparation 18.
Two isomers A) and B) are present in 54/46 ratio.
**NMR (¹H, DMSO-d6):** δ 7.4-7.31 (d+d, 4H), 7.45-6.95 (m, 3H), 6.5-6.4 (d, 1H), 5.65 (m, 1H), 4.00 (t, 2H), 2.67 (t, 2H), 4.12-3.8 (m, 1H), 3.74 (s, 3H) 2.87-2.0 (m, 2H), 2.08-2.0 (m, 4H), 1.56-1.1 (m, 4H), 1.3 (m, 1H), 0.87 (d, 3H). **MS (*m*/*z*):** 459 [MH]⁺, 1Cl.

### Preparation 53: 3-(4-Chloro-phenyl)-5-hydroxy-1-(4-methoxy-3-[2-piperidin-1-yl)-ethoxy]-phenyl)-pyrrolidin-2-one

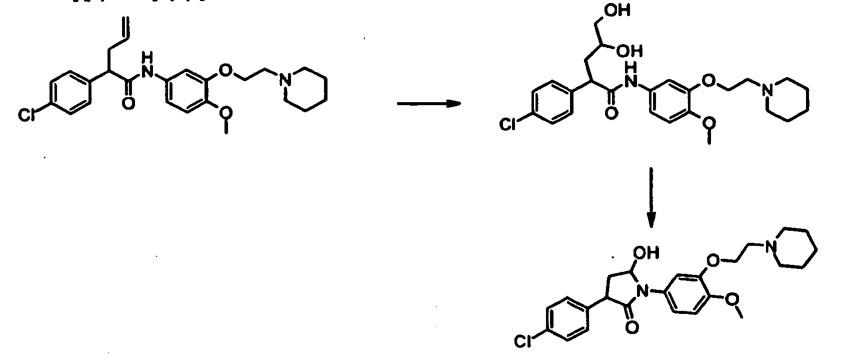

The title compound was prepared in an analogous manner to the compound of Preparation 18.
Two isomers A) and B) are present in 85/15 ratio.
**NMR (¹H, DMSO-d6):** δ 7.4-7.31 (d+d, 4H), 7.45-6.95 (m, 3H), 6.5-6.4 (d, 1H), 5.65 (m, 1H), 4.00 (t, 2H), 2.62 (t, 2H), 4.12-3.8 (m, 1H), 3.74 (s, 3H), 2.8-1.9 (m, 2H), 2.41 (m, 4H), 1.47 (m, 4H), 1.35 (m, 2H). **MS (*m*/*z*):** 445 [MH]⁺, 1Cl.

### Preparation 54: 3-(2-Methoxy-5-nitro-phenoxymethyl)-piperidine-1-carboxylic acid tert-butyl ester

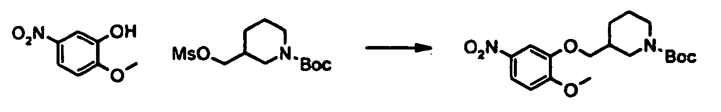

The product was prepared in an analogous manner to Preparation 6.
**NMR (¹H, DMSO-d6):** δ 7.91 (dd, 1H), 7.74 (d, 1H), 7.18 (d, 1 H), 3.97 (d, 2H), 3.94 (bs, 2H), 3.90 (s, 3H), 2.90 (bs, 2H), 1.90 (m, 1H), 1.80 (bs, 1H), 1.60 (bs, 1H), 1.35 (bs, 2H), 1.35 (s, 9H). **MS (*mlz*):** 267 [MH-Boc]⁺.

### Preparation 55: 3-(5-Amino-2-methoxy-phenoxymethyl)-piperidine-1-carboxylic acid tert-butyl ester

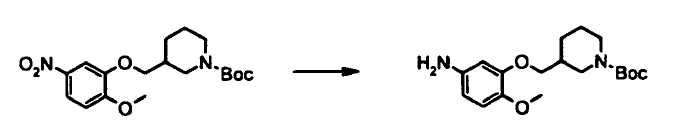

The product was prepared in an analogous manner to Preparation 44.
**NMR (¹H, DMSO-d6):** δ 6.64 (d, 1H), 6.25 (d, 1 H), 6.07 (dd, 1H), 4.69 (bs, 2H), 3.73 (m, 2H), 3.67 (t, 1H), 3.60 (s, 3H), 2.8 (t, 1H), 1.9 (m, 1H), 1.80 (m, 1H), 1.6 (m, 1H), 1.4 (m, 3H), 1.38 (s, 9H), 1.25 (m, 1H). **MS (*m*/*z*):** 237 [MH-Boc]⁺.

### Preparation 56: 3-{5-[2-(3,4-Dichloro-phenyl)-pent-4-enoylamino]-2-methoxy-phenoxymethyl}-piperidine-1-carboxylic acid tert-butyl ester

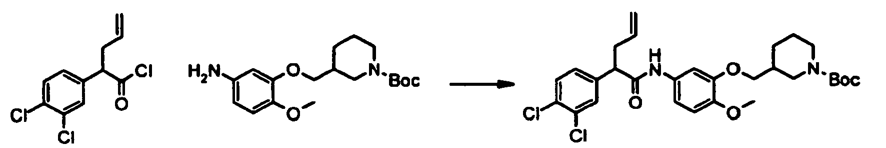

The product was prepared in an analogous manner to Preparation 14.
**NMR (¹H, DMSO-d6):** δ 9.96 (s, 1H), 7.55 (m, 2H), 7.32 (d, 1H), 7.22 (s, 1H), 7.01 (d, 1H), 6.82 (d, 1H), 5.66 (m, 1H), 5.03 (m, 1H), 4.94 (m, 1H), 3.9 (bs, 1H), 3.70 (m, 4H), 3.66 (s, 3H), 2.78 (m, 2H), 2.70 (m, 1H), 2.40 (m, 1H), 1.83 (m, 1H), 1.75 (m, 1H), 1.57 (m, 1H), 1.31 (s, 9H), 1.25 (m, 2H), **MS (*m*/*z*):** 463 [MH-Boc]⁺, 2Cl.

### Preparation 57: 3-5-[3-(3,4-Dichloro-phenyl)-5-hydroxy-2-oxo-pyrrolidin-1-yl]-2-methoxy-phenoxymethyl}-piperidine-1-carboxylic acid tert-butyl ester

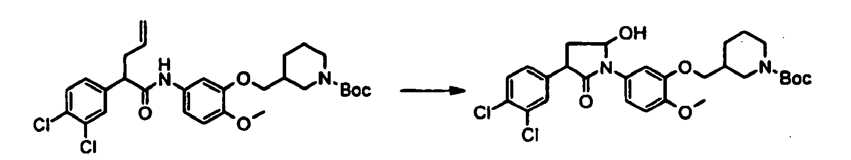

The product was prepared in an analogous manner to Example 15.
**MS (*m*/*z*):** 465 [M-Boc]⁺, 2Cl.

### Preparation 58: N-(2-Hydroxy-4-nitro-phenyl)-acetamide

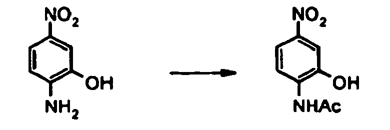

Procedure: J. Med. Chem. 1998, 41, 13, 2308.
**NMR (¹H, DMSO-d6):** δ 10.99 (bs, 1H), 9.51 (s, 1 H), 8.28 (d, 1H), 7.71 (dd, 1H), 7.66 (d, 1H), 2.2 (s, 3H).

### Preparation 59: N-[4-Nitro-2-(2-piperidin-1-yl-ethoxy)-phenyl)-acetamide

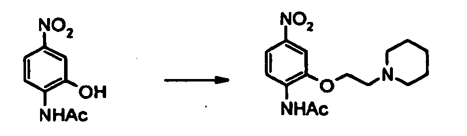

The product was prepared in an analogous manner to Preparation 6.
**NMR (¹H, CDCl₃):** δ 8.72 (bs, 1H), 8.55 (d, 1H), 7.91 (dd, 1H), 7.80 (d, 1H), 4.25 (t, 2H), 2.77 (t, 2H), 2.50 (m, 4H), 2.26 (s, 3H), 1.62 (m, 4H), 1.48 (m, 2H). **MS (*mlz*):** 308 [MH]⁺.

### Preparation 60: N-[4-Amino-2-(2-piperidin-1-yl-ethoxy)-phenyl)-acetamide

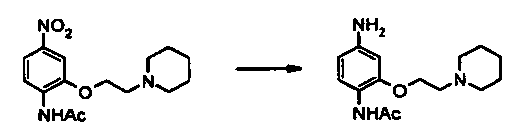

The product was prepared in an analogous manner to Preparation 10.
**NMR (¹H, DMSO-d6):** δ 8.63 (s, 1H), 7.23 (d, 1H), 6.25 (d, 1H), 6.07 (dd, 1H), 4.90 (s, 2H), 3.96 (t, 2H), 2.61 (t, 2H), 2.20 (s, 3H), 2.41 (m, 4H), 1.48 (m, 4H), 1.37 (m, 2H). **MS (*m*/*z*):** 278 [MH]⁺.

### Preparation 61: 2-(3,4-Dichloro-phenyl)-pent-4-enoic acid [4-acetylamino-3-(2-piperidin-1-yl-ethoxy)-phenyl]-amide

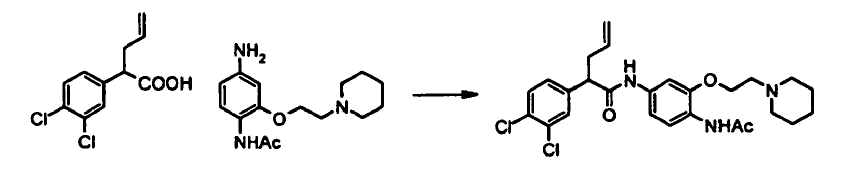

**Procedure:** To a solution of 2-(3,4-dichloro-phenyl)-pent-4-enoic acid (210 mg, 1.08 mmol) in dry DMF (5 ml) were added HOBt (292 mg, 2eq), EDC (310 mg, 1.5 eq) and TEA (0.376 ml, 2.5 eq). After 5 min. was added *N*-[4-Amino-2-(2-piperidin-1-yl-ethoxy)-phenyl)-acetamide (300 mg, 1 eq) dissolved in DMF (7 ml). After three days AcOEt was added, the organic phase was washed with 5% aq. NaHCO₃ and water, dried over Na₂SO₄ and concentrated *in vacuo* to give 398 mg of crude product, that was purified by flash chromatography (CH₂Cl₂/MeOH 9/1) to give 265 mg of the title compound (49 % yield).
**NMR (¹H, DMSO-d6):** δ 10.1 (s, 1H), 8.92 (s, 1H), 7.77 (d, 1H), 7.61 (m, 2H), 7.40 (d, 1H), 7.38 (dd, 1H), 7.05 (dd, 1H), 5.73 (m, 1H), 5.03 (dd, 2H), 4.06 (t, 2H), 3.8 (t, 1H), 2.8 (m, 1H), 2.67 (t, 2H), 2.44 (m, 4H), 2.4 (m, 1H), 2.05 (s, 3H), 1.50 (m, 4H), 1.40 (m, 2H). **MS (*mlz*)**: 504 [MH]⁺, 2Cl.

### Preparation 62: N-[4-[3-(3,4-Dichloro-phenyl)-5-hydroxy-2-oxo-pyrrolidin-1-yl]-2-(2-piperidin-1-yl-ethoxy)-phenyl]-acetamide

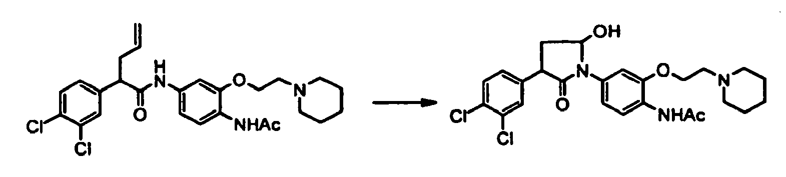

The product was prepared in an analogous manner to Example 15.
The product is a 60/40 mixture of a/b diastereoisomers.
**NMR (¹H, DMSO-d6):** δ 9.01 (s, 1H), 7.84 (d, 1H), 7.69 (d, 1Ha), 7.63 (m, 2H), 7.42 (dd, 1Hb), 7.40 (d, 1Ha), 7.32 (dd, 1Ha), 7.25 (d, 1Hb), 7.13 (dd, 1Ha), 7.02 (dd, 1Hb), 6.59 (d, 1Hb), 6.51 (d; 1Ha), 5.74, (m, 1Ha), 5.68 (m, 1Hb), 4.21 (m, 1Ha), 4.09 (t, 2H), 3.90 (m, 1Hb), 2.95 (m, 1Hb), 2.68 (m, 2H), 2.5 (m, 1Ha), 2.44 (m, 4H), 2.35 (m, 1Ha), 2.07 (s, 3H), 1.95 (m, 1Hb), 1.49 (m, 4H), 1.38 (m, 2H). **MS (*mlz*):** 506 [MH]⁺, 2Cl.

### Preparation 63: 2-(3,4-Dichloro-phenyl)-propionic acid methyl ester

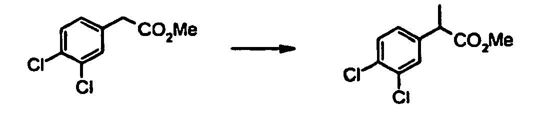

**Procedure:** A solution of methyl-3,4-dichloro-phenylacetate (2 g, 9.13 mmol) in anh. THF (30 ml), under N₂, was treated with lithium bis(trimethylsilyl) amide (1M solution in THF, 1.1 eq., 10 ml) at -78 °C for 30 minutes before methyl iodide (1.2 eq., 0.67 ml) was added. The mixture was stirred for 18 hours at room temperature, then quenched with water (20 ml) and extracted with ethyl acetate (2x 15 ml). The organic phase was washed with brine (1x15 ml), dried over Na₂SO₄ and concentrated *in vacuo* to give 1.68 g of the title product as a orange oil (79%), that was used as a crude.
**NMR (¹H, CDCl₃):** δ 7.20 (m, 2H), 7.0 (dd, 1H), 3.50 (s, 3H), 3.50 (m, 1H), 1.3 (d, 3H).

### Preparation 64: 2-(3,4-Dichloro-phenyl)-2-methyl-pent-4-enoic acid methyl ester

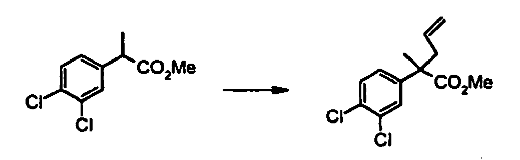

**Procedure:** A solution of 2-(3,4-dichloro-phenyl)-propionic acid methyl ester
(1.66 g, 7.12 mmol) in anh. THF (25 ml), under N₂, was treated with lithium bis(trimethylsilyl) amide (1M solution in THF, 1.1 eq., 7.8 ml) at -78 °C for 30 minutes before allyliodide (1.2 eq., 0.78 ml) was added. The mixture was stirred for 2 hours at room temperature, then quenched with water (20 ml) and extracted with ethyl acetate (2x 15 ml). The organic phase was washed with brine (1x15 ml), dried over Na₂SO₄ and concentrated *in vacuo.* The crude product was purified by flash chromatography (silica gel, cHex/AcOEt 9/1) to give 1.70 g of the title product as a pale yellow oil (87%).
**NMR (¹H, CDCl₃):** δ 7.35 (m, 2H), 7.0 (dd, 1H), 5.40 (m, 1H), 4.95 (m, 2H), 3.50 (s, 3H), 2.6 (m, 1 H), 2.4 (m, 1H), 1.2 (s, 3H).

### Preparation 65: 2-(3,4-Dichloro-phenyl)-2-methyl-pent-4-enoic acid

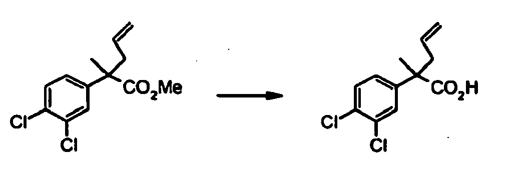

**Procedure:** To a solution of 2-(3,4-dichloro-phenyl)-2-methyl-pent-4-enoic acid methyl ester (0.908 g, 3.32 mmol) in MeOH (33 ml), was added NaOH 1 M (3 eq., 9.9 ml). The mixture was refluxed for 7 hours, then concentrated and washed with CH₂Cl₂. The aqueous phase was acidified to pH 3 with conc. HCl and extracted with CH₂Cl₂. The combined extracts were dried over Na₂SO₄ and concentrated *in vacuo* to give 806 mg of the title product as a colorless oil (94%), that was used as crude.
**NMR (¹H, CDCl₃):** δ 7.30 (m, 2H), 7.10 (dd, 1H), 5.40 (m, 1H), 4.95 (m, 2H), 2.60 (m, 1H), 2.45 (m, 1H), 1.50 (s, 3H).

### Preparation 66: 2-(3,4-Dichloro-phenyl)-2-methyl-pent-4-enoic acid {4-methoxy-3-[2-(4-methyl-piperidin-1-yl)-ethoxy]-phenyl}-amide

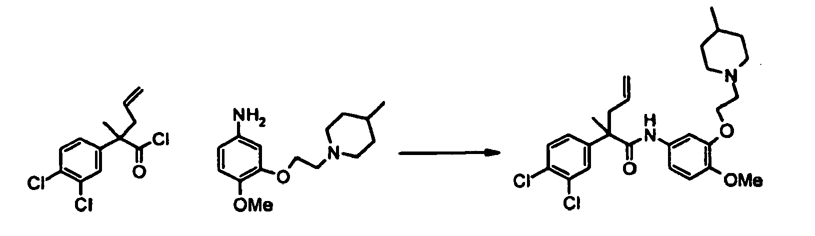

The product was prepared in an analogous manner to Preparation 14.
**NMR (¹H, CDCl₃):** δ 9.46 (bs, 1H), 7.30-6.80 (m, 5H), 6.59 (d, 1H), 5.35 (m, 1H), 4.94-4.80 (m, 2H), 4.04 (t, 2H), 3.60 (s, 3H), 3.34 (m, 2H), 3.10 (t, 2H), 2.70-2.28 (m, 4H), 1.64-1.29 (m, 1H), 1.43 (s, 3H), 1.25 (m, 4H), 0.72 (d, 3H). **MS (*m*/*z*):** 505 [MH]⁺, 2Cl.

### Preparation 67: 3-(3,4-Dichloro-phenyl)-5-hydroxy-1-{4-methoxy-3-[2-(4-methyl-piperidin-1-yl)-ethoxy]-phenyl}-3-methyl-pyrrolidin-2-one

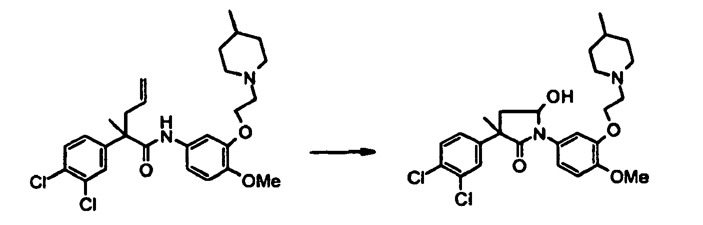

The product was prepared in an analogous manner to Example 15.
**MS (*m*/*z*)**: 507 [MH]⁺**,** 2Cl.

### Preparation 68: 2-(3-Chloro-phenyl)-pent-4-enoic acid

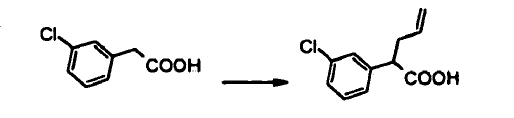

**Procedure:** The reaction was carried out as described for Preparation 12 on 1.00 g of 3-chlorophenylacetic acid. The title compound (1.23 g) was recovered as an orange oil.
**NMR (¹H, DMSO-d6):** δ 12.50 (bs, 1H), 7.15-7.40 (m, 4H), 5.60-5.80 (m,1H), 4.90-5.10 (m, 2H), 3.70 (t, 1H), 2.60-2.80 (m, 1H), 2.35-2.50 (m, 1H).

### Preparation 69: 2-(3-Chloro-phenyl)-pent-4-enoic acid [4-methoxy-3-(2-piperidin-1-yl-ethoxy)-phenyl]-amide

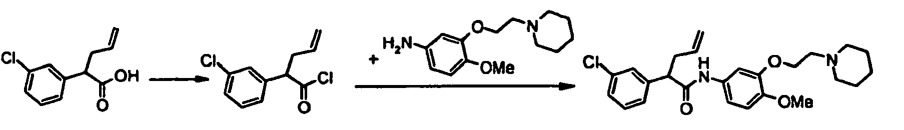

The reaction was carried out on 500 mg of 2-(3-chloro-phenyl)-pent-4-enoic acid as described in Preparation 14. The title compound (760 mg) was obtained as a brown oil.
**NMR** (¹H, CDCl₃): δ 7.35 (bs, 1H), 7.18-7.28 (m, 3H), 7.10 (bs, 1H), 6.83 (dd, 1H), 6.75 (d, 1H), 5.64-5.80 (m, 1H), 4.95-5.12 (m, 2H), 4.10 (t, 2H), 3.78 (s, 3H), 3.45 (t, 1H), 2.85-3.00 (m, 2H), 2.77 (t, 2H), 2.40-2.60 (m, 5H), 1.52-1.65 (m, 4H), 1.36-1.47 (m, 2H). **MS (*m*/*z*):** 443 [MH]⁺, 1Cl.

### Preparation 70: 3-(3-Chloro-phenyl)-5-hydroxy-1-[4-methoxy-3-(2-piperidin-1-yl-ethoxy)-phenyl]-pyrrolidin-2-one

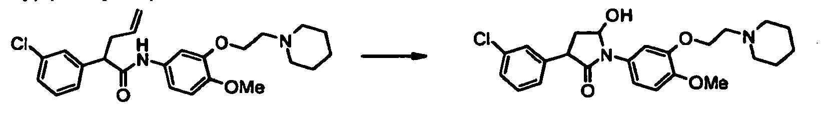

**Procedure:** The reaction was carried out on 2-(3-chloro-phenyl)-pent-4-enoic acid [4-methoxy-3-(2-piperidin-1-yl-ethoxy)-phenyl]-amide (700 mg) as described for Example 15 to give the title compound (430 mg, mixture of two diastereomers as a brown foam.
**MS (*m*/*z*):** 445[MH]⁺, 1Cl.

### Preparation 71: 2-(3-Trifluoromethyl-phenyl)-pent-4-enoic acid

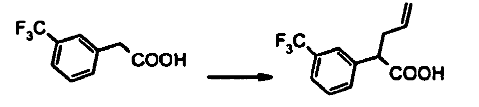

**Procedure:** The reaction was carried out as described for Preparation 12 on 1.00 g of 3-trifluoromethylphenylacetic acid. The title compound (1.18 g) was recovered as a yellow gum.
**NMR (¹H, DMSO-d6):** δ 12.45 (bs, 1H), 7.50-7.70 (m, 4H), 5.60-5.75 (m, 1H), 4.90-5.00 (m, 2H), 3.80 (t, 1H), 2.65-2.80 (m, 1H), 2.40-2.50 (m, 1H).

### Preparation 72: 2-(3-Trifluoromethyl-phenyl)-pent-4-enoic acid [4-methoxy-3-(2-piperidin-1-yl-ethoxy)-phenyl]-amide

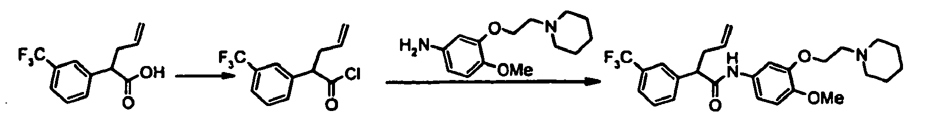

**Procedure:** The reaction was carried out on 300 mg of 2-(3-trifluoromethyl-phenyl)-pent-4-enoic acid as described in Preparation 14. The title compound (450 mg) was obtained as a white foam.
**NMR (¹H, DMSO-d6):** δ 10.03 (s, 1H), 7.71 (bs, 1H), 7.70 (d, 1H), 7.66 (bs, 1H), 7.61 (d, 1H), 7.59 (t, 1H), 7.06 (dd, 1H), 6.86 (d, 1H), 5.72 (m, 1H), 5.09 (d, 1H), 4.99 (d, 1H), 3.96 (bs, 2H), 3.80 (t, 1H), 3.67 (s, 3H), 3.63 (bs, 2H), 2.8 (m, 1H), 2.6 (m, 1H), 2.41 (bs, 4H), 1.47 (bm, 4H), 1.40 (bs, 2H). **MS (*m*/*z*):** 477 [MH]⁺.

### Preparation 73: 3-(3-Trifluoromethyl-phenyl)-5-hydroxy-1-[4-methoxy-3-(2-piperidin-1-yl-ethoxy)-phenyl]-pyrrolidin-2-one

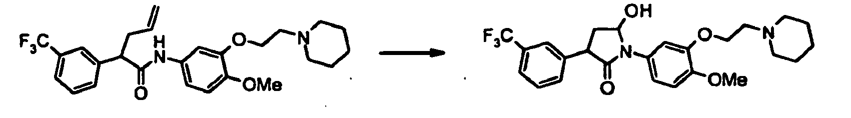

**Procedure:** The reaction was carried out on 2-(3-triftuoromethyl-phenyl)-pent-4-enoic acid [4-methoxy-3-(2-piperidin-1-yl-ethoxy)-phenyl]-amide (450 mg) as described for Example 13 to give the title compound (285 mg, mixture of two diastereomers in 2.5/4 ratio) as a colourless oil.
**NMR (¹H, CDCl₃)** of the major product: δ 7.57-7.79 (m, 4H), 7.13 (dd,1H), 7.01 (d, 1H), 6.99 (d, 1H), 6.46 (d, 1H), 5.65 (m, 1H), 4.30 (t, 2H), 3.98 (t, 1H), 3.76 (s, 3H), 2.91 (m, 2H), 2.62 (bs, 4H), 2.50 (m, 2H), 2.39 (m, 2H), 1.58 (bm, 4H), 1.41 (bs, 2H).
**MS (*m*/*z*):** 479 [MH]⁺.

### Preparation 74: 2-(4-Fluoro-phenyl)-pent-4-enoic acid

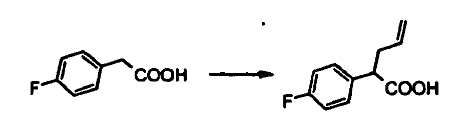

**Procedure:** The reaction was carried out as described for Preparation 12 on 1.00 g of 4-fluoromethylphenylacetic acid. The title compound (1.26 g) was recovered as a white foam.
**NMR (¹H, DMSO-d6):** δ 12.40 (bs, 1H), 7.25-7.40 (m, 2H), 7.05-7.15 (m, 2H), 5.60-5.75 (m, 1H), 4.90-5.05 (m, 2H), 3.60 (t, 1H), 2.60-2.75 (m, 1H), 2.30-2.45 (m, 1H).

### Preparation 75: 2-(4-Fluoro-phenyl)-pent-4-enoic acid [4-methoxy-3-(2-piperidin-1-yl-ethoxy)-phenyl]-amide

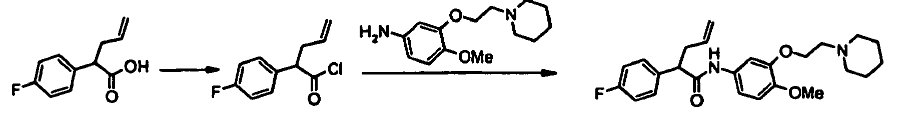

**Procedure:** The reaction was carried out on 600 mg of 2-(4-fluoro-phenyl)-pent-4-enoic acid as described in Preparation 14. The title compound (880 mg) was obtained as an orange gum.
**NMR (¹H, DMSO-d6):** δ 9.94 (s, 1H), 7.41 (t, 2H), 7.28 (d, 1H), 7.14 (t, 2H), 7.05 (dd, 1H), 6.85 (d,1H), 5.69 (m, 1H), 5.08 (d, 1H), 4.98 (d, 1H), 3.97 (t, 2H), 3.80 (t, 1H), 3.70 (t, 1H), 3.68 (s, 3H), 2.70 (m, 1H), 2.63 (t, 2H), 2.40 (bm, 4H), 1.48 (bm, 4H), 1.37 (bs, 2H). **MS (*m*/*z*):** 427 [MH]⁺.

### Preparation 76: 3-(4-Fluoro-phenyl)-5-hydroxy-1-[4-methoxy-3-(2-piperidin-1-yl-ethoxy)-phenyl]-pyrrolidin-2-one

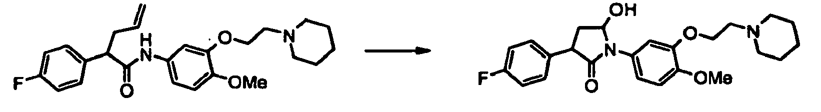

**Procedure:** The reaction was carried out on 2-(4-fluoromethyl-phenyl)-pent-4-enoic acid [4-methoxy-3-(2-piperidin-1-yl-ethoxy)-phenyl]-amide (750 mg) as described for Example 13 to give the title compound (570 mg, mixture of two diastereomers) as a brown foam.
**MS (*mlz*):** 429 [MH]⁺.

### Example 1: 3-(3,4-Dichloro-phenyl)-3-hydroxy-1-[4-methoxy-3-(2-piperidin-1-yl-ethoxy)-phenyl]-pyrrolidin-2-one

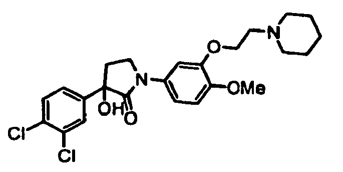

To a solution of methansulfonic acid 2-(5-[3-(3,4-dichtoro-phenyl)-2-oxo-pyrrolidin-1-yl]-2-methoxy-phenoxy)-ethyl ether (103 mg, 0.216 mmol) in DMF (5ml) was added piperidine (1eq, 22µl), Nal (cat.) and K₂CO₃ (1eq, 15.5µl). The reaction mixture was heated at 60°C for 5 hours under atm N₂ and then 18 hours at room temperature in the presence of air. The reaction mixture was diluted with water (20 ml) and extracted with AcOEt (3x25 ml). The combined organic extracts were dried over Na₂SO₄, filtered and concentrated *in vacuo* The crude product was purified by flash chromatography (silica gel, CH₂Cl₂/CH₃OH 18/2) to give 1.6 mg of title product as a yellow oil (3.8%).
**NMR (¹H, DMSO-d6):** δ 7.68 (d, 1H), 7.62 (d, 1 H), 7.48 (d, 1H), 7.40 (dd, 1 H), 7.12 (dd, 1H), 6.97 (d, 1H), 6.48 (s, 1H), 4.02 (t, 2H), 3.91-3.80 (m, 2H), 3.74 (s, 3H). 2.64 (m, 2H), 2.51-2.3 (m, 2H), 2.42 (m, 4H), 1.47-1.35 (m, 2H). **MS (*m*/*z*):** 479 [MH]⁺+ 2Cl

### Example 2: 3-(3,4-Dichloro-phenyl)-3-hydroxy-1-[4-methoxy-3-(2-morpholin-4-yl-ethoxy)-phenyl]-pyrrolidin-2-one

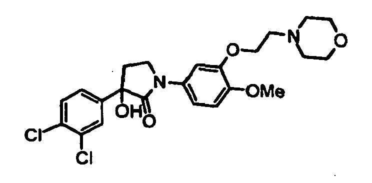

The product was prepared in an analogous manner as for Example 1.
**NMR (¹H, DMSO-d6):** δ 7.67 (d, 1H), 7.62 (d, 1H), 7.48 (d, 1H), 7.40 (dd, 1H), 7.12 (dd, 1H), 6.98 (d, 1H), 6.48 (s, 1H), 4.05 (t, 2H), 3.90-3.80 (m, 2H), 3.74 (s, 3H), 3.74 (s, 3H), 3.56 (m, 4H), 2.68 (m, 2H), 2.49-2.34 (m, 2H), 2.48 (m, 4H). **MS (*m*/*z*):** 481 [MH]⁺ 2Cl

### Example 3: 3-(3,4-Dichloro-phenyl)-1-[4-methoxy-3-(2-piperidin-1-yl-ethoxy)-phenyl]-pyrrolidin-2-one

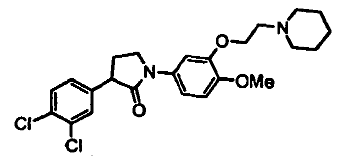

To a solution of crude methansulfonic acid 2-(5-[3-(3,4-dichloro-phenyl)-2-oxo-pyrrolidin-1-yl]-2-methoxy-phenoxy)-ethyl ether (0.09 mmol) in DMF (2ml) was added piperidine (1eq, 9µl), Nal (cat.) and DIPEA (1eq, 15.5µl). The reaction mixture was heated at 100°C for 3 hours. The reaction mixture was diluted with water (20 ml) and extracted with AcOEt (3x25 ml). The combined organic extracts were dried over Na₂SO₄, filtered and concentrated *in vacuo* The crude product was purified by flash chromatography (silica gel, CH₂Cl₂/CH3OH 18/2) to give 1.6 mg of the title product as a pale yellow solid (3.8%).

**NMR (¹H, CDCl₃):** δ .7.56 (d, 1H), 7.45-7.43 (m, 2H), 7.18 (dd, 1H), 6.96 (dd, 1H), 6.86 (d, 1H), 4.19 (t, 2H), 3.95-3.8 (m, 4H+2H), 2.65-2.57 (m, 1H+4H), 2.25 (m, 1H), 1.9-1.5 (m, 6H). **MS (*mlz*):** 463.4 [MH]⁺+ 2Cl

### Example 4: 1-[4-Chloro-3-(2-piperidin-1-yl-ethoxy)-phenyl]-3-(3,4-dichloro-phenyl)-pyrrolidin-2-one

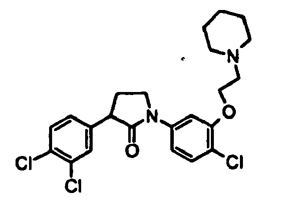

The title compound was prepared in an analogous manner to the compound of Example 3.
**NMR (¹H, CDCl₃):** δ 7.72 (d, 1H), 7.45 (d, 1H), 7.42 (d, 1H), 7.33 (d, 1H), 7,18 (dd, 1H), 6.90 (dd, 1H), 4.19 (t, 2H), 3.91 (dd, 2H), 3.84 (t, 1H), 2.86 (t, 2H), 2.65 (m, 1H), 2.57 (m, 4H), 2.3 (m, 1H) 1.6-1.4 (m, 6H). **MS (*m*/*z*):** 467 [MH]⁺, 3Cl.

### Example 5: 1-[4-Chloro-3-(2-piperidin-1-yl-ethoxy)-phenyl]-3-(3,4-dichloro-phenyl)-3-hydroxy-pyrrolidin-2-one

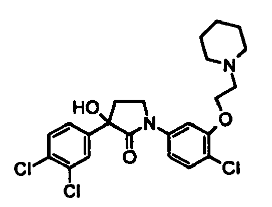

1-[4-Chloro-3-(2-piperidin-1-yl-ethoxy)-phenyl]-3-(3,4-dichloro-phenyl)-pyrrolidin-2-one (26 mg, 0.055 mmol) was dissolved in DMF (1 ml), tBuOK (9 mg, 1.3 eq) was added and the mixture was stirred at room temperature in the presence of air for 30 min. A saturated solution of NH₄Cl was added and extracted with CH₂Cl₂. The extracts were dried over Na₂SO₄ and concentrated in vacuo. The residue was purified by flash chromathography (silica gel, CH₂Cl₂/MeOH 9/1), to give 13 mg of the title product (49 % yield).
**NMR (¹H, CDCl₃):** δ 7.70 (d, 1H), 7.63 (m, 2H), 7.43 (d, 1H), 7.42 (dd, 1H), 7.28 (dd, 1 H), 6.57 (s, 1H), 4.14 (t, 2H), 3.98 (m, 1H), 3.85 (m, 1H), 2.69 (m, 2H), 2.53 (m, 1H), 2.45 (m, 4H), 2.35 (m, 1H), 1.47-1.35 (m, 6H). **MS (*m*/*z*):** 483 [MH]⁺, 3CI.

### Example 6: 3-(3,4-Dichloro-phenyl)-1-(4-methoxy-3-[2-(4-methyl-piperidin-1-yl)-ethoxy]-phenyl)-pyrrolidin-2-one

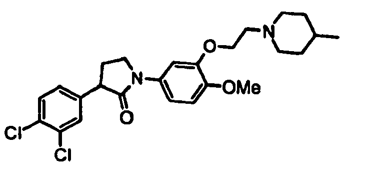

The title compound was prepared in an analogous manner to the compound of Example 3.
**NMR (¹H, CDCl₃):** δ .7.57 (d, 1H), 7.44 (d+d, 1H+1H), 7.19 (dd, 1H), 6.96 (dd, 1H), 6.86 (d, 1H), 4.17 (bt, 2H), 3.90 (m, 2H), 3.83 (t, 1H), 3.86 (s, 3H), 3.00 (m, 2H). 2.84 (bt, 2H), 2.66 (m, 1H), 2.28 (m, 1H), 2.10 (m, 2H), 1.6-1.2 (m, 5H), 0.93 (d, 3H). **MS (*mlz*):** 477 [MH]⁺ 2Cl

### Example 7: 3-(3,4-Dichloro-phenyl)-1-[4-methoxy-3-(2-piperidin-1-yl-ethoxy)-phenyl]-1,5-dihydro-pyrrol-2-one

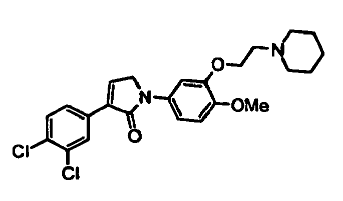

3-(3,4-Dichloro-phenyl)-3-hydroxy-1-[4-methoxy-3-(2-piperidin-1-yl-ethoxy)-phenyl]-pyrrolidin-2-one (17.2 mg, 0.035 mmol) was dissolved in concentrated hydrochloric acid (1.7 ml). The reaction was heated at 40°C for 3 hours, and then concentrated to dryness *in vacuo*. A solution of saturated NaHCO₃ was added until pH>7 and the product was extracted with ethylacetate (2x10ml). The combined extracts were dried over anhydrous Na₂SO₄, filtered and concentrated to dryness *in vacuo.* The crude product was purified by flash chromatography (silica gel, AcOEt/CH₃OH 4/6) to give 11.8 mg of title product as a brown oil (55.2%).
**NMR (¹H, CDCl₃):** 8.05 (d, 1H), 7.78 (d, 1H), 7.63 (d, 1H), 7.48 (d, 1H), 7.31 (t, 1H), 7.04 (dd, 1H), 6.88 (d, 1H), 4.45 (d, 2H), 4.20 (t, 2H), 3.87 (s, 3H). 2.85 (t, 2H), 2.54 (m, 4H), 1.62 (m, 4H), 1.45 (m, 2H). **MS (*m*/*z*):** 461.1 [MH]⁺+2Cl

### Example 8: 3-(3,4-Dichloro-phenyl)-1-[4-methoxy-3-(2-morpholin-4-yl-ethoxy)-phenyl]-1,5-dihydro-pyrrol-2-One

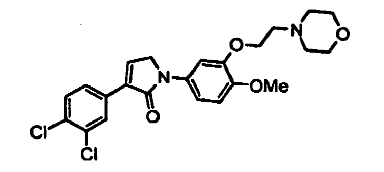

The product was prepared in an analogous manner to Example 7.
**NMR (¹H, CDCl₃):** δ. 8.04 (d, 1H), 7.77 (dd, 1H), 7.72 (d, 1H), 7.48 (d, 1H), 7.31 (t, 1H), 6.96 (dd, 1H), 6.88 (d, 1H), 4.45 (d, 2H), 4.21 (t, 2H), 3.86 (s, 3H), 3.74 (m, 4H), 2.87 (t, 2H), 2.60 (m, 4H). **MS (*m*/*z*):** 463 [MH]⁺+ 2Cl

### Example 9: 1-[4-Chloro-3-(2-piperidin-1-yl-ethoxy)-phenyl]-3-(3,4-dichloro-phenyl)-1,5-dihydro-pyrrol-2-one

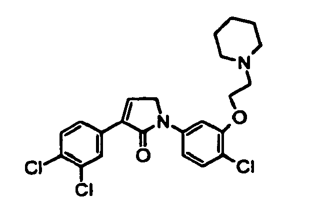

1-[4-Chloro-3-(2-piperidin-1-yl-ethoxy)-phenyl]-3-(3,4-dichloro-phenyl)-5-hydroxy-pyrrolidin-2-one (51 mg, 0.1 mmol) was dissolved in TFA (1 ml). The reaction mixture was stirred at room temperature for 6 hours, then concentrated in vacuo. A saturated solution of NaHCO₃ was added and the mixture was extracted with ethyl acetate, dried over Na₂SO₄ and concentrated in vacuo. The residue was purified by flash chromathography over silica gel (CH₂Cl₂/MeOH 95/5) to give 29 mg of the title product as a colorless oil (yield: 60 %). **NMR (¹H, CDCl₃):** δ 8.0 (d, 1H), 7.83 (d, 1H), 7.73 (dd, 1H), 7.46 (d, 1H), 7,32 (d, 1H), 7.30 (m, 1H), 6.96 (dd, 1H), 4.43 (d, 2H), 4.21 (t, 2H), 2.85 (t, 2H), 2.56 (m, 4H), 1.5-1.4 (m, 6H). **MS (*mlz*):** 465 [MH]⁺, 3Cl.

### Example 10: 3-(3,4-Dichloro-phenyl)-1-[4-methoxy-3-(2-pyrrolidin-1-yl-ethoxy)-phenyl]-1,5-dihydro-pyrrol-2-one

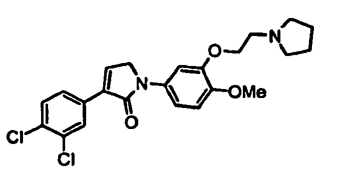

The title product was prepared in an analogous manner to Example 7.
**NMR (¹H, CDCl₃):** δ .8.06 (d, 1H), 7.78 (dd, 1H), 7.64 (d, 1H), 7.49 (d, 1H), 7.32 (t, 1H), 7.05 (dd, 1 H), 6.89 (d, 1H), 4.45 (d, 2H), 4.22 (t, 2H), 3.87 (s, 3H), 2.99 (t, 2H), 2.68 (m, 4H), 1.83-1.77 (m, 4H). **MS (*m*/*z*):** 447 [MH]⁺+ 2Cl

### Example 11: 3-(3-Fluoro-phenyl)-1-[4-methoxy-3-(2-piperidin-1-yl-ethoxy)-phenyl]-1,5-dihydro-pyrrol-2-one

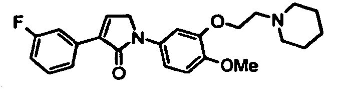

The title compound was prepared in an analogous manner to the compound of Example 3.

**NMR (¹H, CDCl₃):** δ 7.55-7.70 (m, 3H), 7.25-7.40 (m, 2H), 6.95-7.10 (m, 2H), 6.85 (d, 1H), 4.40 (d, 2H), 4.10-4.20 (m, 2H), 3.85 (s, 3H), 2.85 (t, 2H), 2.45-2.60 (m, 4H), 1.55-1.70 (m, 4H), 1.35-1.50 (m, 2H). **MS (*m*/*z*):** 411[MH]⁺.

### Example 12: 3-(3,4-Dichloro-phenyl)-1-(-3-[2-(4,4-difluoro-piperidin-1-yl)-ethoxy]-4-methoxy-phenyl)-1,5-dihydro-pyrrol-2-one

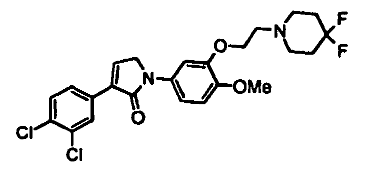

The title compound was prepared in an analogous manner to the compound of Example 3.

**NMR (¹H, CDCl₃):** δ 8.06 (d, 1H), 7.78 (dd, 1H), 7.76 (d, 1H), 7.50 (d, 1H), 7.33 (t, 1H), 6.99 (bd, 1H), 6.90 (d, 1H), 4.44 (d, 2H), 4.22 (m, 2H), 3.87 (s, 3H), 2.95 (m, 2H), 2.75 (m, 4H), 2.05 (m, 4H). **MS (*m*/*z*):** 497.3 [MH]⁺+2Cl

### Reference Example 13: 3-(3-Fluoro-phenyl)-5-hydroxy-1-[4-methoxy-3-(2-piperidin-1-yl-ethoxy)-phenyl]-pyrrolidin-2-one

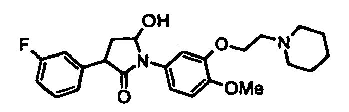

To a solution of 2-(3-fluoro-phenyl)-pent-4-enoic acid [4-methoxy-3-(2-piperidin-1-yl-ethoxy)-phenyl]-amide(85 mg, 0.2 mmol) in THF/H₂O (5/1, 4 mL) was added OsO₄ (4% wt in water, 0.02 mmol) and NalO₄ (86 mg, 0.4 mmol). After 18 hours the suspension was diluted with water and the aqueous phase was extracted with EtOAc (2x). The combined organic phases were dried over Na₂SO₄. filtered and concentrated to dryness *in vacuo.* The crude was purified by flash chromatography (CH₂Cl₂/MeOH/ NH₄OH 95/5/0.5) to give 65 mg of title product as a brown oil. (y = 75%) **MS (*mlz*):** 29[MH]⁺.

### Example 14: 3-(3-Fluoro-phenyl)-1-[4-methoxy-3-(2-piperidin-1-yl-ethoxy)-phenyl]-pyrrolidin-2-one

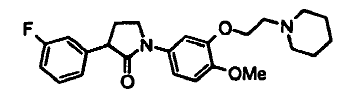

The title compound was prepared in an analogous manner to the compound of Example 3.
**NMR (¹H, CDCl₃):** δ 7.56 (d, 1H), 7.30 (m, 1H), 7.08 (d, 1H), 7.02 (m, 1 H), 6.95 (m, 1H), 6.93 (dd, 1H), 6.83 (d, 1H), 4.16 (t, 2H), 3.9-3.8 (m, 3H), 3.82 (s, 3H), 2.84 (t, 2H), 2.64 (bs, 4H), 2.62 (m, 1H), 2.24 (m, 1H), 1.60 (m, 4H), 1.43 (m, 2H). **MS (*m*/*z*):** 413[MH]⁺.

### Example 15: 3-(3,4-Dichloro-phenyl)-1-[4-methoxy-3-(2-pyrrolidin-1-yl-ethoxy)-phenyl]-pyrrolidin-2-one hydrochloride salt

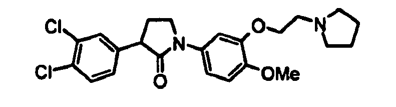

To 3-(3,4-dichloro-phenyl)-5-hydroxy-1-[4-methoxy-3-(2-pyrrolidin-1-yl-ethoxy)-phenyl]-pyrrolidin-2-one (17 mg) in DCM (dry, 0.3 ml) and Et₃SiH (0.03 ml) at 0 °C was added dropwise a solution of trifluoroacetic acid (0.028 ml) in DCM (dry, 0.3 ml). The mixture was allowed to warm to 25 °C. After 8 h volatiles were removed *in vacuo* and the residue submitted to column chromatography (silica gel, CH₂Cl₂ / MeOH / NH₃) to give, after conversion to the hydrochloride salt by treatment with excess HCl (1 M in Et₂O) followed by evaporation *in vacuo* and trituration of the residue with Et₂O, the title compound (8 mg) as a slightly yellow film.
**NMR (¹H, CD₃OD):** δ 7.60 (d, 1H), 7.54-7.5 (m, 2H), 7.30 (dd, 1H), 7.12 (dd, 1H), 7.08 (d, 1H), 4.35 (t, 2H), 4.02-3.92 (m, 3H), 3.89 (s, 3H), 3.82-3.76 (m, m, 2H), 3.65 (t, 2H), 3.28-3.20 (m, 2H), 2.71-2.63 (m, 1H), 2.32-2.16 (m, 3H), 2.11-2.01 (m, 2H). **MS (*m*/*z*):** 449 [MH]⁺. 2Cl.

### Example 16: 3-(3,4-Dichloro-phenyl)-1-[4-methoxy-3-(2-piperidin-1-yl-ethoxy)-phenyl]-3-methyl-pyrrolidin-2-one hydrochloride salt

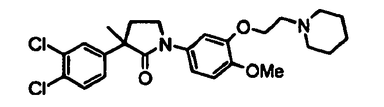

The title compound (racemic, colourless foam. **MS (*m*/*z*):** 477 [MH]⁺, 2Cl.) was prepared from 2-(3,4-dichloro-phenyl)-2-methyl-pent-4-enoic acid and 4-methoxy-3-(2-piperidin-1-yl-ethoxy)-phenylamine in an analogous manner to the one described for Preparation 28 and Reference Example 13.
Separation of the enantiomers was achieved by preparative HPLC using a chiral stationary phase followed by salt formation as described for Example 15 to give Enantiomer 1 (slightly yellow solid) and Enantiomer 2 (slightly yellow solid) of the title compound.
**Enantiomer 1: NMR (¹H, CD₃OD):** δ 7.60 (d, 1H), 7.56 (d, 1H), 7.48 (d, 1H), 7.36 (dd, 1H), 7.10-7.02 (m, 2H), 4.34 (t, 2H), 3.92-3.82 (m, 4H), 3.80-3.74 (m, 1H), 3.68 (bd, 2H), 3.53 (t, 2H), 3.06 (bt, 2H), 2.57-2.50 (m, 1H), 2.36-2.28 (m, 1H), 2.00-1.93 (m, 2H), 1.88-1.73 (m, 3H), 1.60-1.45 (m, 4H).
**Enantiomer 2: NMR (¹H, CD₃OD):** Essentially identical to the data for Enantiomer 1.

### Example 17: 3-(3-Chloro-phenyl)-1-{4-methoxy-3-[2-(4-methyl-piperidin-1-yl)-ethoxy]-phenyl}-1,5-dihydro-pyrrol-2-one hydrochloride salt

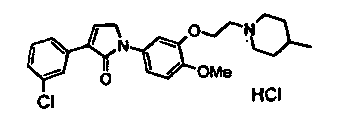

The title compound was prepared, in an analogous manner to the compound of Example 15 (HCl salt formation), in 44 mg yield as yellow solid (y= 62%) from 2-(3chloro-phenyl)-5-hydroxy-1-{4-methoxy-3-[2-(4-methylpiperidin)-1-yl-ethoxy]-phenyl}-pyrrolidin-2-one (90 mg)
**NMR (¹H, DMSO):** δ 9.85 (bs,1 H), 8.11 (s, 1H), 7.94 (d, 1H), 7.90 (t, 1H), 7.68 (d, 1H), 7.45 (m, 2H), 7.28 (dd, 1H), 7.06 (d, 1H), 4.62 (d, 2H), 4.37 (t, 2H), 3.78 (s, 3H), 3.60 (m, 2H), 3.40 (m, 2H), 3.00 (m, 2H), 1.82 (m, 2H), 1.60 (m, 1H), 1.45 (m, 2H), 0.90 (d, 3H).
**MS (*mlz*):** 441 [MH]⁺, 1Cl. Mp=99-101°C.

### Reference Example 18: 3-(3,4-Dichloro-phenyl)-1-[4-methoxy-3-(2-piperidin-1-yl-ethoxy)-phenyl]-piperidin-2-one hydrochloride salt

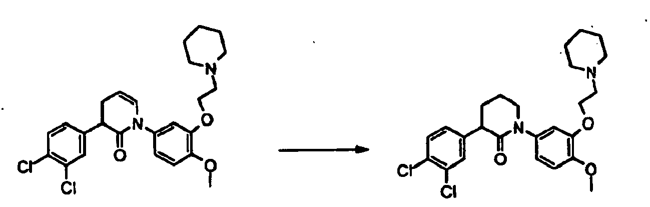

**Procedure:** To a solution of 3-(3,4-dichloro-phenyl)-1-[4-methoxy-3-(2-piperidin-1-yl-ethoxy)-phenyl]-3,4-dihydro-1H-pyridin-2-one (50 mg, 0.105 mmol) in anh. CH₂Cl₂ (1 ml), under N₂, was added triethyl silane (3 eq., 0.05 ml). The reaction was stirred at room temperature for 1 h, then trifluoroacetic acid (6 eq., 0.05 ml) was added. The reaction was stirred at room temperature for 16 h. The reaction was concentrated *in vacuo*. The crude product was purified by flash chromatography (silica gel, CH₂Cl₂/MeOH from 24/1 to 4/1) to obtain 10 mg of desired compound as the free base.
**NMR (¹H, CDCl₃):** δ 7.36 (d, 1H), 7.34 (d, 1H), 7.10 (dd, 1H), 6.90 (bs, 3H), 4.37 (s, 2H), 3.80 (s, 3H), 3.8-3.6 (m, 5H), 3.42 (s, 2H), 2.83 (t, 2H), 2.30 (m, 1H), 2.1-1.8 (m, 7H), 1.35 (m, 2H). **MS (m/z):** 477 [MH]⁺, 2Cl.

From this material the hydrochloride salt was obtained as described for Example 17.
**MS (m/z):** 477 [MH]⁺, 2Cl.

### Example 19: 3-(3,4-Dichloro-phenyl)-1-(4-methoxy-3-[2-(4-methyl-piperidin-1-yl)-ethoxy]-phenyl)-3,4-dihydro-pyrrol-2-one hydrochloride salt

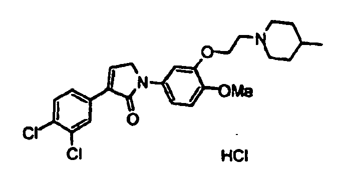

The title compound was prepared in an analogous manner to the one described for the compound of Example 9 and 15 (HCl salt formation).
**NMR (¹H, DMSO-d₆):** δ .9.8+9.5 (2bs, 1H), 8.30 (d, 1H), 7.97 (dd, 1H), 7.94 (t, 1H), 7.68 (d, 1H), 7.65 (d, 1H), 7.23 (dd, 1H), 7.03 (d, 1H), 4.60 (d, 2H), 4.32 (t, 2H), 3.75 (s, 3H), 3.56 (d, 2H), 3.45 (m, 2H), 3.01 (q, 2H), 1.78 (d, 2H), 1.57 (bm, 1H), 1.38 (q, 2H), 0.89 (d, 3H). **MS (*m*/*z*):** 477 [MH]⁺, 2Cl

### Example 20: 3-(3,4-Dichloro-phenyl)-1-(4-methoxy-3-[2-(4-methyl-piperidin-1-yl)-ethoxy]-phenyl)-4-methyl-1,5-dihydro-pyrrol-2-one hydrochloride salt

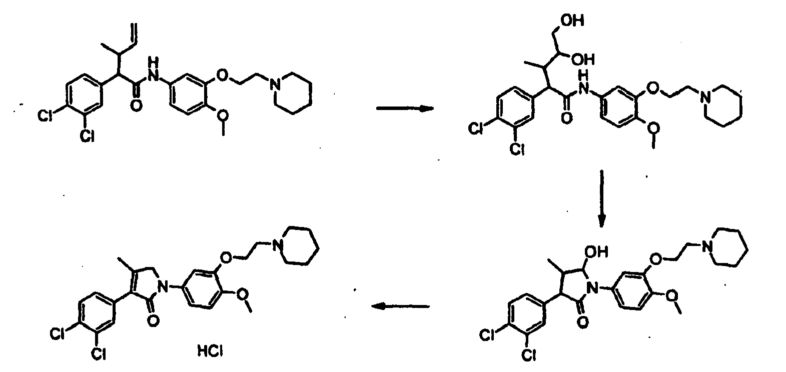

The title compound was prepared in an analogous manner to the one described for the compound of Reference Example 13 followed by Example 9 and 15 (HCl salt formation).
**NMR (¹H, CD₃OD):** δ 7.74 (d, 1H), 7.63 (d, 1H), 7.71 (d, 1H), 7.44 (dd, 1H), 7.18 (dd, 1H), 7.09 (d, 1H), 4.53 (s, 2H), 4.34 (t, 2H), 3.56 (t, 2H), 3.5-3.1 (bs, 4H), 3.9 (s, 3H), 2.26 (s, 3H), 1.91 (bs, 4H), 1.7 (bs, 2H). **MS (*m*/*z*):** 475 [MH]⁺, 2Cl.

### Example 21: 3-(4-Chloro-phenyl)-1-(4-methoxy-3-[2-(4-methyl-piperidin-1-yl)-ethoxy]-phenyl)-3,4-dihydro-pyrrol-2-one hydrochloride salt

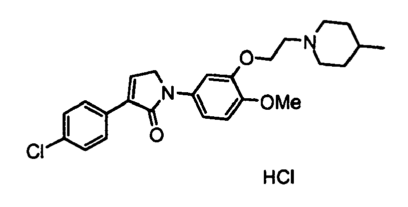

The title compound was prepared in an analogous manner to the one described for the compound of Example 9 and 15 (HCl salt formation).
**NMR (¹H, DMSO-d₆):** δ .9.9+9.76 (2bs, 1H), 8.02 (d, 2H), 7.82 (t, 1H), 7.67 (d, 1H), 7.51 (d, 2H), 7.27 (dd, 1 H), 7.06 (d, 1H), 4.61 (d, 2H), 4.36 (t, 2H), 3.78 (s, 3H), 3.59 (d, 2H), 3.48 (m, 2H), 3.04 (q, 2H), 1.81 (m, 1H), 1.60 (bm, 1H), 1.44 (m, 2H), 1.01-0.92 (d-d, 3H). **MS (*m*/*z*):** 441 [MH]⁺, 1Cl.

### Example 22: 3-(4-Chloro-phenyl)-1-(4-methoxy-3-[2-piperidin-1-yl)-ethoxy]-phenyl)-3,4-dihydro-pyrrol-2-one hydrochloride salt

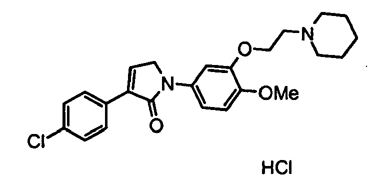

The title compound was prepared in an analogous manner to the one described for the compound of Example 9 and 15 (HCl salt formation).
**NMR (¹H, DMSO-d₆):** δ 9.8 (bs, 1 H), 8.00 (d, 2H), 7.8 (t, 1H), 7.67 (d, 1H), 7.50 (d, 2H), 7.27 (dd, 1 H), 7.05 (d, 1H), 4.60 (d, 2H), 4.35 (t, 2H), 3.78 (s, 3H), 3.6/4.4 (m, 4H), 3.00 (m, 2H), 1.85-1.65 (m, 4H), 1.4 (m, 2H). **MS (*m*/*z*):** 427 [MH⁺], 1Cl

### Example 23: 3-(3,4-Dichloro-phenyl)-1-[4-methoxy-3-(piperidin-3-ylmethoxy)-phenyl]-pyrrolidin-2-one

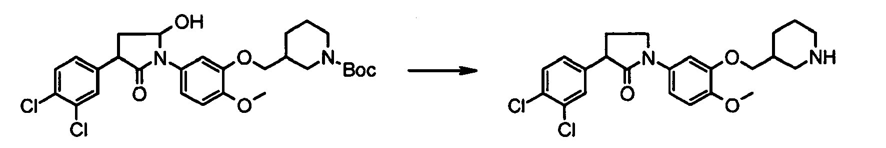

The product was prepared in an analogous manner to Preparation 17.
**NMR (¹H, DMSO-d6):** δ 7.62-7.60 (m, 2H), 7.47 (t, 1H), 7.33 (dd, 1 H), 7.20 (dd, 1 H), 6.94 (d, 1 H), 3.98 (t, 1H), 3.85 (m, 2H), 3.75 (m, 2H), 3.74 (s, 3H), 3.04 (m, 1 H), 2.3 (m, 1H), 2.86 (m, 1 H), 2.45 (m, 1H), 2.55 (m, 1H), 2.2 (m, 1 H), 1.87 (m, 1 H), 1.79 (m, 1 H), 1.58 (m, 1 H), 1.37 (m, 1H), 1.16 (m, 2H). **MS (*m*/*z*):** 449 [MH]⁺, 2Cl.

### Example 24: 3-(3,4-Dichloro-phenyl)-1-[4-methoxy-3-(1-methyl-piperidin-3-ylmethoxy)-phenyl]-pyrrolidin-2-one

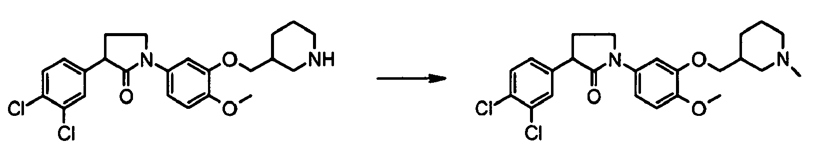

**Procedure:** To a solution of 3-(3,4-dichloro-phenyl)-1-[4-methoxy-3-(piperidin-3-ylmethoxy)-phenyl]-pyrrolidin-one (0.064 g, 0.14 mmol) in acetonitrile/dichloromethane 2/1 (3 ml), was added formaldehyde 37% in water (2.5 eq., 0.03 ml) and sodium triacetoxy borohydride (1.5 eq., 45 mg). After 3h the mixture was concentrated, diluited with CH₂Cl₂ and washed with NaHCO₃ 5%. The organic phase was dried over Na₂SO₄ and concentrated *in vacuo* to give a colorless oil, that was purified by flash chromatography (CH₂Cl₂/MeOH 9/1) to give 52 mg of the title compound (80 % yield).
**NMR (¹H, DMSO-d6):** δ 7.62 (d, 1H), 7.61 (d, 1H), 7.47 (t, 1H), 7.33 (dd, 1 H), 7.03 (dd, 1H), 6.96 (d, 1H), 3.99 (t, 1H), 3.86 (dd, 2H), 3.77 (m, 2H), 3.74 (s, 3H), 2.85-2.75 (bs, 1H), 2.7-2.6 (bs, 1 H), 2.55 (m, 1 H), 2.21 (m, 1 H), 2.16 (bs, 3H). 2.0 (bs, 1H), 2.0-1.8 (bs, 2H), 1.69 (m, 1H), 1.62 (m, 1H), 1.6 (m, 1 H), 1.48 (m, 1H). **MS (*m*/*z*):** 463 [MH]⁺, 2Cl.

### Example 25: N-{4-[3-(3,4-dichlorophenyl)-2-oxo-2,5-dihydro-1H-pyrrol-1-yl]-2-[2-(1-piperidinyl)ethoxy]phenyl}acetamide hydrochloride

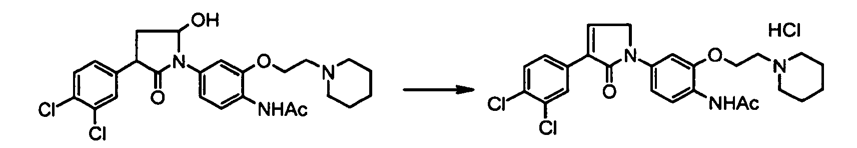

The product was prepared in an analogous manner to Example 9.
**NMR (¹H, DMSO-d6):** δ 10.2 (bs, 1 H), 9.53 (s, 1 H), 8.34 (d, 1 H), 8.0 (dd, 1 H), 8.0 (t, 1H), 7.87 (d, 1 H) 7.79 (d, 1H), 7.72 (d, 1H), 7.24 (dd, 1H), 4.66 (d, 2H), 4.39 (t, 2H), 3.52 (t, 2H), 3.52 (m, 2H), 2.99 (m, 2H), 2.15 (s, 3H), 1.81 (m, 4H), 1.72 (m, 1 H), 1.42 (m, 1 H).
**MS (*mlz*):** 488 [MH]', 2Cl.

### Example 26: N-{4-[3-(3,4-dichlorophenyl)-2-oxo-pyrrolidin-1-yl]-2-[2-(1-piperidinyl)ethoxy]phenyl}acetamide hydrochloride

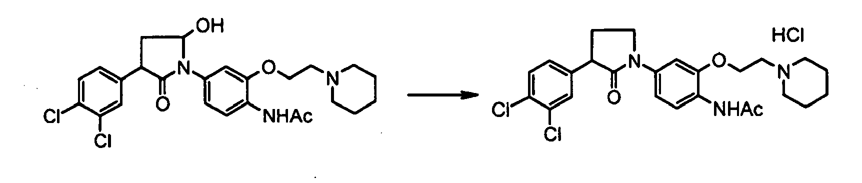

The product was prepared in an analogous manner to Preparation 17.
**NMR (¹H, DMSO-d₆):** δ 10.28 (bs, 1H), 9.55 (s, 1H), 7.89 (d, 1H), 7.63 (d, 1H), 7.63 (m, 2H), 7.34 (dd, 1H), 7.11 (dd, 1H), 4.34 (t, 2H), 4.03 (t, 1H), 3.90 (t, 2H), 3.49 (t, 2H), 3.48 (m, 2H), 2.97 (m, 2H), 2.57 (d, 1H), 2.20 (d, 1H), 2.15 (s, 3H), 1.87 (m, 4H), 1.72 (m, 1H), 1.42 (m, 1H). **MS (*m*/*z*):** 490 [MH]⁺, 2Cl.

### Example 27: 3-(3,4-Dichloro-phenyl)-1-{4-methoxy-3-[2-(4-methyl)-piperidin-1-yl-ethoxy]-phenyl}-3-methyl-pyrrolidin-2-one hydrochloride

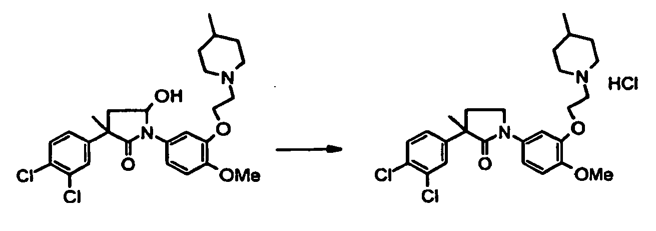

The product was prepared in an analogous manner to Preparation 17
**NMR (¹H, DMSO-d6):** δ 9.75 (bs, 1H), 7.69 (d, 1H), 7.62 (d, 1H), 7.57 (d, 1H), 7.44 (dd, 1H), 7.15 (dd, 1H), 7.04 (d, 1H), 4.34 (t, 2H), 3.95 (m, 1H), 3.69 (m, 1H), 3.72 (s, 3H), 3.58 (m, 2H), 3.40 (m, 2H), 3.05 (m, 2H), 2.62 (m, 1H), 2.27 (m, 1H), 1.49 (s, 3H), 1.81 (m, 2H), 1.40 (m, 2H), 1.51 (m, 1H), 0.92 (d, 3H). **MS (*mlz*):** 491 [MH]⁺, 2Cl.

### Example 28: 3-(3-Chloro-phenyl)-1-[4-methoxy-3-(2-piperidin-1-yl-ethoxy)-phenyl]-pyrrolidin-2-ons hydrochloride salt

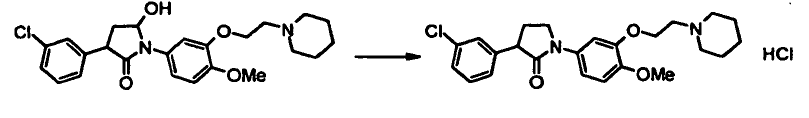

**Procedure:** The title compound (52 mg) was obtained as a brown solid in an analogous manner to the one described for Reference Example 18 followed by HCl salt formation as for Example 15.
**NMR (¹H, DMSO):** δ 9.80 (bs,1 H), 7.57 (d, 1H), 7.42-7.32, 7.28 (m, 4H), 7.16 (dd, 1H), 7.04 (d, 1H), 4.32 (t, 2H), 3.95 (t, 1H), 3.89 (m, 2H), 3.79 (s, 3H), 3.55 (d, 2H), 3.46 (m, 2H), 3.02 (q, 2H), 2.55 (m, 1H), 2.20 (m, 1H), 1.82 (d, 2H), 1.70 (m, 2H), 1.40 (bm, 2H). **MS (*mlz,* free base):** 429 [MH]⁺, 1Cl**.**

### Example 29: 3-(3-Trifluoromethyl-phenyl)-1-[4-methoxy-3-(2-piperidin-1-yl-ethoxy)-phenyl]-pyrrolidin-2-one

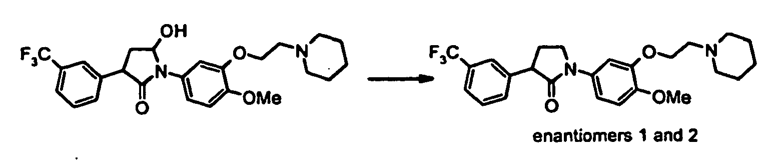

**Procedure:** The racemic title compound was obtained in an analogous manner to the one described for Reference Example 18. Separation of the enantiomers was achieved by preparative HPLC using a chiral stationary phase to give Enantiomer 1 (yellow oil, 3 mg) and Enantiomer 2 (colourless gum, 6 mg) of the title compound.
**Enantiomer 1: NMR (¹H, CDCl₃):** δ 7.56-7.44 (m, 4H), 7.55 (d, 1H), 6.94 (dd, 1H), 6.84 (d, 1H), 4.17 (t, 2H), 3.88 (m, 3H), 3.83 (s, 3H), 2.84 (m, 2H), 2.66 (m, 1H), 2.54 (bs, 4H), 2.30 (m, 1 H), 1.61 (m, 4H), 1.43 (m, 2H).
**MS (*m*/*z*):** 463 [MH]⁺.
**Enantiomer 2: NMR (¹H, CDCl₃); MS:** Essentially identical to the data for Enantiomer 1.

### Example 30: 3-(3-Trifluoromethyl-phenyl)-1-[4-methoxy-3-(2-piperidin-1-yl-ethoxy)-phenyl]-1,5-dihydro-pyrrol-2-one

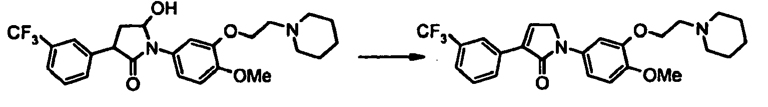

**Procedure:** The title compound (20 mg) was obtained as a yellow oil in an analogous manner to the one described for Example 11.
**NMR (¹H, DMSO):** δ 8.15 (bs, 1H), 8.13 (d, 1H), 7.66 (d, 1H), 7.63 (d, 1H), 7.56 (t, 1H), 7.38 (t, 1H), 7.08 (dd, 1H), 6.90 (d, 1H), 4.49 (d, 2H), 4.23 (t, 2H), 3.88 (s, 3H), 2.87 (t, 2H), 2.55 (bs, 4H), 1.65 (bm, 4H), 1.46 (bs, 2H). **MS (*m*/*z*):** 461 [MH]⁺.

### Example 31: 3-(3-Chloro-phenyl)-1-[4-methoxy-3-(2-piperidin-1-yl-ethoxy)-phenyl]-1,5-dihydro-pyrrol-2-one hydrochloride

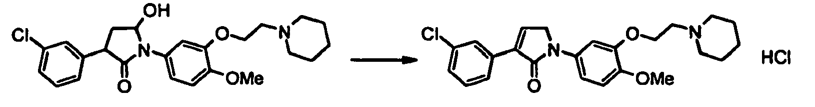

**Procedure:** The title compound (52 mg) was obtained as a brown gum in an analogous manner to the one described for Example 9 followed by HCl salt formation as for Example 15.
**NMR (¹H, DMSO):** δ 9.80 (bs, 1H), 8.11 (t, 1H), 7.94 (dt, 2H), 7.70 (t, 1H), 7.40-7.50 (m, 2H), 7.28 (dd, 1H), 7.07 (d, 1H), 4.63 (d, 2H), 4.35 (t, 2H), 3.79 (s, 3H), 3.57 (d, 2H), 3.50 (m, 2H), 3.03 (q, 2H), 1.85 (d, 2H), 1.71 (m, 2H), 1.20-1.40 (m, 1H).
**MS (*mlz;* free base):** 427 [MH]⁺, 1Cl.

### Example 32: 3-(4-Fluoro-phenyl)-1-[4-methoxy-3-(2-piperidin-1-yl-ethoxy)-phenyl]-1,5-dihydro-pyrrol-2-one hydrochloride

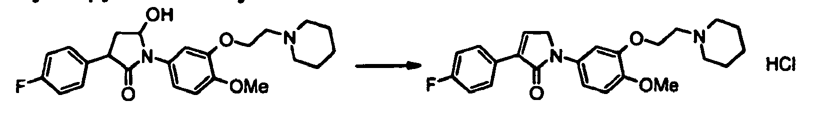

**Procedure:** The title compound (28 mg) was obtained as a yellow solid in an analogous manner to the one described for Example 9 followed by HCl salt formation as for Example 15.
**NMR (¹H, DMSO):** δ 7.96 (m, 2H), 7.55 (t, 1H), 7.23 (d, 1H), 7.21 (dd, 1H), 7.16 (m, 2H), 7.10 (d, 1H), 4.57 (d, 2H), 4.41 (t, 2H), 3.91 (s, 3H), 3.58 (t, 2H), 3.74/3.11 (d/t, 2/2H), 1.99/1.85 (d/t, 2/2H), 1.85/1.60 (m/m, 1/1H). **MS (m/z;free base):** 411 [MH]⁺.
Mp=207-209 °C.

### Example 33: 3-(4-Fluoro-phenyl)-1-[4-methoxy-3-(2-piperidin-1-yl-ethoxy)-phenyl]-pyrrolidin-2-one hydrochloride salt

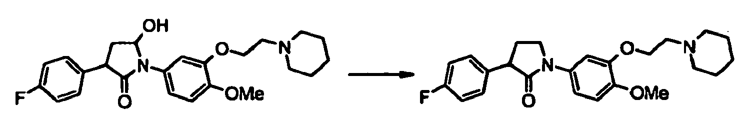

**Procedure:** The title compound (52 mg) was obtained as a yellow gum in an analogous manner to the one described for Example 21 followed by HCl salt formation as for Example 15.
**NMR (¹H, DMSO-d6):** δ 9.8 (s, 1H), 7.59 (d, 1H), 7.34 (m, 2H), 7.17 (m, 2H), 7.16 (dd, 1H), 7.04 (d, 1H), 4.32 (t, 2H), 3.93 (t, 1H), 3.80 (s, 3H), 3.77 (m, 2H), 3.56 (m, 2H), 3.47 (q, 2H), 3.03 (m, 2H), 2.55 (m, 1H), 2.15 (m, 1H), 1.70-1.80 (m, 4H), 1.45 (m, 2H). **MS (*m*/*z*; free base):** 413 [MH]⁺.

### Example 34: 1-{4-Methoxy-3-[2-(4-methyl-piperidin-1-yl)-ethoxy]-phenyl}-3-(4-methyl-phenyl)-1,5-dihydro-pyrrol-2-one hydrochloride salt

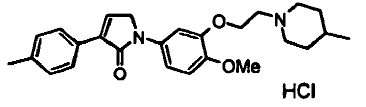

5-Hydroxy-1-{4-methoxy-3-[2-(4-methyl-piperidin-1-yl)-ethoxy]-phenyl}-3-(4-methylphenyl)-pyrrolidin-2-one (80 mg, prepared in analogy to Preparations 13, 14 and 46) was dissolved in TFA (2 ml). The reaction mixture was stirred at room temperature overnight, then concentrated *in vacuo.* A saturated solution of NaHCO₃ was added and the mixture was extracted with ethyl acetate, dried over Na₂SO₄ and concentrated *in vacuo.* The residue was purified by flash chromathography over silica gel (CH₂Cl₂/MeOH from 1/0 to 98/2) to give 35 mg of the free base of the title compound. The product dissolved in CH₂Cl₂ (1.5ml), at 0°C, was treated with HCl (1 M in Et₂O, 0.17ml) followed by evaporation *in vacuo* and trituration of the residue with Et₂O to give the title compound (30 mg) as a white solid (40%).
**NMR (¹H, CDCl₃):** δ 12.4 (bs, 1H), 7.79 (d, 2H), 7.63 (d, 1H), 7.24 (dd, 1H), 7.23 (d, 2H), 7,27 (s, 1H), 6.91 (d, 1H), 4.60 (t, 2H), 4.45 (d, 2H), 3.85 (s, 3H), 3.70 (m, 2H), 3.42 (t, 2H), 2.90 (m, 2H), 2.38 (s, 3H), 2.10/1.80 (m, 4H), 1.6 (m, 1), 1.05 (d, 3H); **MS (*m*/*z*):** 421 [M+H]⁺.

Examples 35-38 were prepared in analogy to Example 38:

### Example 35: 1-{4-Methoxy-3-[2-(piperidin-1-yl)-ethoxy]-phenyl}-3-(4-methyl-phenyl)-1,5-dihydro-pyrrol-2-one hydrochloride salt

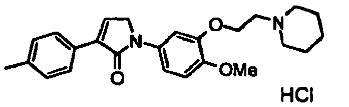

NMR (¹H, DMSO-d₆): δ 10.00 (bs, 1H), 7.87 (d, 2H), 7.68 (s, 1H), 7.67 (s, 1H), 7.28 (dd, 1H), 7,22 (d, 2H), 7.06 (d, 1H), 4.57 (s, 2H), 4.38 (t, 2H), 3.78 (s, 3H), 3.57/3.04 (d/t, 2H/2H), 3.48 (q, 2H), 2.32 (s, 3H), 1.80/1.70 (m, 2H/2H), 1.70/1.40 (m, 1H/1H); **MS (*m*/*z*):** 407 [M+H]⁺.

### Example 36: 3-(4-Bromo-phenyl)-1-{4-methoxy-3-[2-(4-methyl-piperidin-1-yl)-ethoxy]-phenyl}-1,5-dihydro-pyrrol-2-one hydrochloride salt

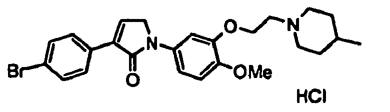

NMR (¹H, DMSO-d₆): δ 10.06 (bs, 1H), 7.95 (d, 2H), 7.83 (t, 1H), 7.67 (d, 1H), 7.63 (d, 2H), 7,28 (dd, 1H), 7.06 (d, 1H), 4.60 (d, 2H), 4.38 (t, 2H), 3.78 (s, 3H), 3.58 (m, 2H), 3.46 (m, 2H), 3.03 (m, 2H), 1.80 (m, 2H), 1.60 (m, 1H), 1.46 (m, 2H), 0.92 (d, 3H); **MS (*mlz*):** 485 [M+H]⁺, 1Br.

### Example 37: 1-{4-Methoxy-3-[2-(4-methyl-piperidin-1-yl)-ethoxy]-phenyl}-3-(-4-trifluoromethyl-phenyl)-1,5-dihydro-pyrrol-2-one hydrochloride salt

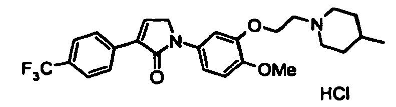

**NMR (¹H, DMSO-d₆):** δ 10.00/9.83 (bs, 1H), 8.20 (d, 2H), 7.96 (t, 1H), 7.81 (d, 2H), 7.68 (d, 1H), 7,29 (dd, 1H), 7.07 (d, 1H), 4.65 (d, 2H), 4.37 (t, 2H), 3.78 (s, 3H), 3.59 (m, 2H), 3.49 (m, 2H), 3.04 (m, 2H), 1.80/1.40 (m, 4H), 1.60 (m, 1H), 0.91 (d, 3H); **MS (*m*/*z*):** 475 [M+H]⁺.

### Example 38: 3-(2-Chloro-phenyl)-1-{4-methoxy-3-[2-(4-methyl-piperidin-1-yl)-ethoxy]-phenyl}-1,5-dihydro-pyrrol-2-one hydrochloride salt

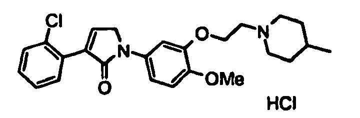

**NMR (¹H, DMSO-d₆):** δ 9.90 (bs, 1H), 7.61 (d, 1H), 7.56 (t, 1H), 7.50/7.36 (m/m 2H/2H). 7.26 (dd, 1H), 7.04 (d, 1H), 4.64 (d, 2H), 3.75 (s, 3H), 3.34 (t, 2H), 3.56/3.01 (m/m, 2H/2H), 3.44 (m, 2H), 1.78/1.42 (m/m, 2H/2H), 1.55 (bm, 1H), 0.88 (d, 3H); **MS (*m*/*z*):** 441 [M+H]⁺, 1Cl.

### Example 39: 3-(3,4-Dichloro-phenyl)-3-hydroxy-1-{4-methoxy-3-[2-(4-methyl-piperidin-1-yl)-ethoxy]-phenyl}-pyrrolidin-2-one

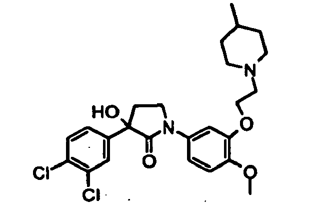

3-(3,4-Dichloro-phenyl)-1-{4-methoxy-3-[2-(4-methyl-piperidin-1-yl)-ethoxy]-phenyl}-pyrrolidin-2-one (730 mg, 1.53 mmol) was dissolved in DMF (10 ml), tBuOK (340 mg, 2 eq) was added and the mixture was stirred at room temperature in the presence of air for 15 min. A saturated solution of NaHCO₃ was added and the mixture extracted with CH₂Cl₂. The extracts were dried over Na₂SO₄ and concentrated *in vacuo.* The residue was purified by column chromathography (silica gel, CH₂Cl₂/MeOH 9/1), to give 260 mg of the title product (34%).
**NMR (¹H, CDCl₃):** δ 7.55 (m, 2H), 7.40 (d, 1H), 7.25 (d, 1H), 7.00 (d, 1H), 6:85 (d, 1H), 4.15 (m, 2H), 3.80 (s, 3H), 3.70 (m, 1H), 3.00 (m, 2H), 3.85 (m, 2H), 2.50 (m, 1H), 2.40 (m, 1H), 2.10 (m, 1H), 1.90-1.50 (m, 6H), 1.25 (m, 2H), 0.85 (d, 3H). **MS (*mlz*):** 493
[MH]⁺ (2Cl).

### Example 40: 3-(3,4-Dichloro-phenyl)-3-fluoro-1-{4-methoxy-3-[2-(4-methyl-piperidin-1-yl)-ethoxy]-phenyl}-pyrrolidin-2-one hydrochloride salt

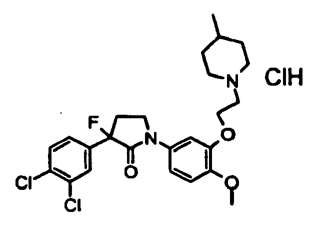

3-(3,4-Dichloro-phenyl)- 3-hydroxy-1-{4-methoxy-3-[2-(4-methyl-piperidin-1-yl)-ethoxy]-phenyl}-pyrrolidin-2-one (210 mg, 0.43 mmol) was dissolved in CH₂Cl₂ (3 ml), (diethylamino)sulfur trifluoride (DAST, 56 µl, 1 eq) was added at 0 °C and the mixture was stirred for 1 h. A saturated solution of NaHCO₃ was added and the mixture extracted with CH₂Cl₂. The extracts were dried over Na₂SO₄ and concentrated in vacuo. The residue was purified by preparative HPLC to give, after conversion to the hydrochloride salt by treatment with excess HCl (1 M in Et₂O) followed by evaporation *in vacuo* and trituration of the residue with Et₂O, 72 mg of the title product (34%).
**NMR (¹H, Acetone-d₆):** δ 13.2 (bs, 1H), 7.81 (d, 1H), 7.69 (m, 2H), 7.61 (dd, 1H), 7.37 (bdd, 1H), 7.09 (d, 1H), 4.66 (bm, 2H), 4.15 (m, 2H), 3.89 (s, 3H), 3.64 (bm, 2H), 3.45 (bm, 2H), 3.09 (bm, 2H), 3.05-2.09 (m, 2H), 2:00-1.6 (bm, 5H), 1:02 (d, 3H). **MS (*m*/*z*):** 495 [MH]⁺ (2Cl).

### Example 41: 3-(3,4-Dichloro-phenyl)-1-{4-methoxy-3-[(1-methyl-pyrrolidin-2-yl)-methoxy]-phenyl}-pyrrolidin-2-one

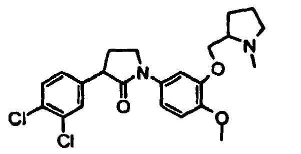

The product was prepared in an analogous manner to Example 24.
**NMR (¹H, CD₃OD):** δ 7.57 (d, 1H), 7.54 (d, 1H), 7.53 (d, 1 H), 7.29 (dd, 1H), 7.12/7.09 (m, 2H), 4.45/4.2 (m, 2H), 3.97 (m, 3H), 3.90 (m, 1H), 3.87 (s, 3H), 3.70/3.25 (m, 2H), 3.14 (s, 3H), 2.65/2.30 (m, 2H), 2.4/2.1 (m,2H), 2.25/2.1 (m, 2H). **MS (*m*/*z*):** 449 [MH]⁺ (2Cl).

### Example 42: 3-(3,4-Dichloro-phenyl)-1-{4-methoxy-3-[(1-methyl-pyrrolidin-2-yl)-mothoxy]-phenyl}-3,4-dihydro-pyrrol-2-one hydrochloride salt

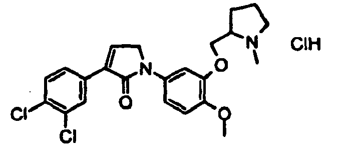

3-(3,4-Dichloro-phenyl)-1-{4-methoxy-3-[(1-methyl-pyrrolidin-2-yl)-methoxy]-phenyl}-pyrrolidin-2-one (134 mg, 0.276 mmol) was dissolved in DMF (1 ml), tBuOK (124 mg, 4 eq) was added and the mixture was stirred at room temperature in the presence of air for 10 min. A saturated solution of NaHCO₃ was added and the mixture extracted with CH₂Cl₂. The extracts were dried over Na₂SO₄ and concentrated *in vacuo.* The residue was suspended in HCl 37% and heated in a microwave oven to 130°C for 10 minutes. The solvent was removed and the residue was purified by column chromathography (silica gel, CH₂Cl₂/MeOH 9/1), to give, after conversion to the hydrochloride salt by treatment with excess HCl (1 M in Et₂O) followed by evaporation *in vacuo* and trituration of the residue with Et₂O, 10 mg of the title product (8%).
**NMR (¹H, CDCl₃):** δ 12.65 (bs, 1H), 8.1 (s, 1H), 7.75 (d, 1H), 7.70 (s, 1H), 7.50 (d, 1H), 7.4 (d, 1H), 7.35 (s, 1H), 6.90 (d, 1H), 4.85 (t, 1H), 4.55 (d, 1H), 4.35 (d, 1H), 3.95 (s, 1H), 3.85 (s, 3H), 3.65 (bs, 1H), 3.10 (s, 3H), 2.90 (s, 2H), 2.30 (bm, 2H), 2.10 (bm, 2H). **MS (*m*/*z*):** 447 [MH]⁺ (2Cl).

## Claims

1. A compound of formula (I) or a pharmaceutically acceptable salt thereof wherein: 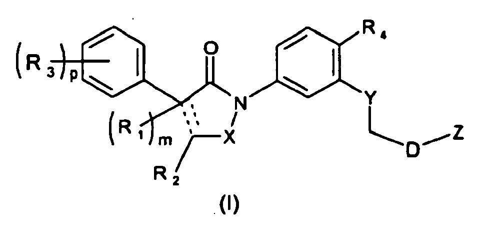
R₁ is hydrogen, hydroxy, fluoro, chloro, C₁₋₆alkyl, C₃₋₇cycloalkyl, C₃₋₇cycycloalkyloxy, C₁₋₆alkoxy or haloC₁₋₆alkoxy;
m is 0 when 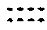 is a double bond and m is 1 when 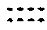 is a single bond;
R₂ is hydrogen, halogen, cyano, nitro, C₁₋₆alkyl, C₃₋₇cycloalkyl, C₃₋₇cycloalkyloxy, haloC₁₋₆alkyl, C₁₋₆alkoxy, haloC₁₋₆alkoxy, C₁₋₆alkylthio, amino, mono- or di-C₁₋₆alkylamino or an N-linked 4 to 7 membered heterocyclic group;
X is -CH₂-;
R₃ is halogen;
p is 1 or 2;
R₄ is hydrogen, halogen, hydroxy, cyano, nitro, C₁₋₆alkyl, C₁₋₆alkanoyl, C₃₋₇cycloalkyl, C₃₋₇cycloalkyloxy, haloC₁₋₆alkyl, C₁₋₆alkoxy, haloC₁₋₆alkoxy, C₁₋₆alkylthio, amino, mono- or di-C₁₋₆alkylamino or an N-linked 4 to 7 membered heterocyclic group;
Y is oxygen;
D is -CH₂-; and
Z is an optionally substituted N-linked 4 to 7 membered heterocyclic group.

2. A compound as claimed in claim 1 wherein when 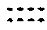 is a single bond, R₁ is hydrogen, hydroxy or C₁₋₆alkoxy.

3. A compound as claimed in claim 1 having the following formula (1a): 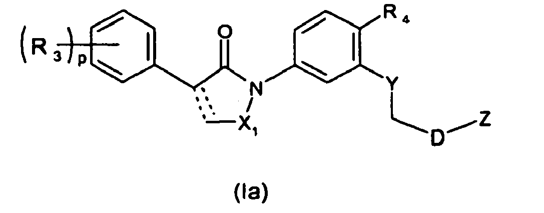 wherein R₃, p, R₄, Y, D, Z, 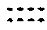 are as defined in claim 1 and X₁ is -CH₂-.

4. A compound as claimed in any of claims 1-3, wherein p is 1 or 2 and R₃ is/are halogen, attached at the 3 or the 3,4-positions of the phenyl ring.

5. A compound as claimed in any of claims 1-4, wherein R₄ is C₁₋₆alkoxy, OCF₃, halogen or cyano.

6. A compound as claimed in any of claims 1-5, wherein R₃ is chloro or fluoro.

7. A compound as claimed in any of claims 1-6, wherein R₄ is methoxy.

8. A compound as claimed in any of claims 1-7, wherein Z is piperidyl.

9. A compound as claimed in claim 1 which is:
3-(3,4-Dichloro-phenyl)-3-hydroxy-1-[4-methoxy-3-(2-piperidin-1-yl-ethoxy)-phenyl]-pyrrolidin-2-one
3-(3,4-Dichloro-phenyl)-3-hydroxy-1-[4-methoxy-3-(2-morpholin-4-yl-ethoxy)-phenyl]-pyrrolidin-2-one
3-(3,4-Dichloro-phenyl)-1-[4-methoxy-3-(2-piperidin-1-yl-ethoxy)-phenyl]-pyrrolidin-2-one
1-[4-Chloro-3-(2-piperidin-1-yl-ethoxy)-phenyl]-3-(3,4-dichloro-phenyl)-pyrrolidin-2-one
1-[4-Chloro-3-(2-piperidin-1-yl-ethoxy)-phenyl]-3-(3,4-dichloro-phenyl)-3-hydroxy-pyrrolidin-2-one
3-(3,4-Dichloro-phenyl)-1-(4-methoxy-3-[2-(4-methyl-pipendin-1-yl)-ethoxy]-phenyl)-pyrrolidin-2-one
3-(3,4-Dichloro-phenyl)-1-[4-methoxy-3-(2-piperidin-1-yl-ethoxy)-phenyl]-1,5-dihydro-pyrrol-2-one
3-(3,4-Dichloro-phenyl)-1-[4-methoxy-3-(2-morpholin-4-yl-ethoxy)-phenyl]-1,5-dihydro-pyrrol-2-one
1-[4-Chloro-3-(2-piperidin-1-yl-ethoxy)-phenyl]-3-(3,4-dichloro-phenyl)-1,5-dihydro-pyrrol-2-one
3-(3,4-Dichloro-phenyl)-1-[4-methoxy-3-(2-pyrrolidin-1-yl-ethoxy)-phenyl]-1,5-dihydro-pyrrol-2-one
3-(3-Fluoro-phenyl)-1-[4-methoxy-3-(2-piperidin-1-yl-ethoxy)-phenyl]-1,5-dihydro-pyrrol-2-one
3-(3,4-Dichloro-phenyl)-1-(-3-[2-(4,4-difluoro-piperidin-1-yl)-ethoxy]-4-methoxy-phenyl)-1,5-dihydro-pyrrol-2-one
3-(3-Fluoro-phenyl)-1-[4-methoxy-3-(2-piperidin-1-yl-ethoxy)-phenyl]-pyrrolidin-2-one
3-(3,4-Dichloro-phenyl)-1-[4-methoxy-3-(2-pyrrolidin-1-yl-ethoxy)-phenyl]-pyrrolidin-2-one
3-(3,4-Dichloro-phenyl)-1-[4-methoxy-3-(2-piperidin-1-yl-ethoxy)-phenyl]-3-methyl-pyrrolidin-2-one
3-(3-Chloro-phenyl)-1-{4-methoxy-3-[2-(4-methyl-piperidin-1-yl)-ethoxy]-phenyl}-1,5-dihydro-pyrrol-2-one
3-(3,4-Dichloro-phenyl)-1-(4-methoxy-3-[2-(4-methyl-piperidin-1-yl)-ethoxy]-phenyl)-3,4-dihydro-pyrrol-2-one
3-(3,4-Dichloro-phenyl)-1-(4-methoxy-3-[2-(4-methyl-piperidin-1-yl)-ethoxy]-phenyl)-4-methyl-1,5-dihydro-pyrrol-2-one
3-(4-Chloro-phenyl)-1-(4-methoxy-3-[2-(4-methyl-piperidin-1-yl)-ethoxy]-phenyl)-3,4-dihydro-pyrrol-2-one
3-(4-Chloro-phenyl)-1-(4-methoxy-3-[2-piperidin-1-yl)-ethoxy]-phenyl)-3,4-dihydro-pyrrol-2-one
3-(3,4-Dichloro-phenyl)-1-[4-methoxy-3-(piperidin-3-ylmethoxy)-phenyl]-pyrrolidin-2-one
3-(3,4-Dichloro-phenyl)-1-[4-methoxy-3-(1-methyl-piperidin-3-ylmethoxy)-phenyl]-pyrrolidin-2-one
*N*-{4-[3-(3,4-dichlorophenyl)-2-oxo-2,5-dihydro-1*H*-pyrrol-1-yl]-2-[2-(1-piperidinyl)ethoxy]phenyl}acetamide
*N*-{4-[3-(3,4-dichlorophenyl)-2-oxo-pyrrolidin-1-yl]-2-[2-(1-piperidinyl)ethoxy]phenyl}acetamide
3-(3,4-Dichloro-phenyl)-1-{4-methoxy-3-[2-(4-methyl)-piperidin-1-yl-ethoxy]-phenyl}-3-methyl-pyrrolidin-2-one
3-(3-Chloro-phenyl)-1-[4-methoxy-3-(2-piperidin-1-yl-ethoxy)-phenyl]-pyrrolidin-2-one
3-(3-Trifluoromethyl-phenyl)-1-[4-methoxy-3-(2-piperidin-1-yl-ethoxy)-phenyl]-pyrrolidin-2-one
3-(3-Trifluoromethyl-phenyl)-1-[4-methoxy-3-(2-piperidin-1-yl-ethoxy)-phenyl]-1,5-dihydro-pyrrol-2-one
3-(3-Chloro-phenyl)-1-[4-methoxy-3-(2-piperidin-1-yl-ethoxy)-phenyl]-1,5-dihydro-pyrrol-2-one
3-(4-Fluoro-phenyl)-1-[4-methoxy-3-(2-piperidin-1-yl-ethoxy)-phenyl]-1,5-dihydro-pyrrol-2-one
3-(4-Fluoro-phenyl)-1-[4-methoxy-3-(2-piperidin-1-yl-ethoxy)-phenyl]-pyrrolidin-2-one
1-{4-Methoxy-3-[2-(4-methyl-piperidin-1-yl)-ethoxy]-phenyl}-3-(4-methyl-phenyl)-1,5-dihydro-pyrrol-2-one
1-{4-Methoxy-3-[2-(piperidin-1-yl)-ethoxy]-phenyl}-3-(4-methyl-phenyl)-1,5-dihydro-pyrrol-2-one
3-(4-Bromo-phenyl)-1-{4-methoxy-3-[2-(4-methyl-piperidin-1-yl)-ethoxy]-phenyl}-1,5-dihydro-pyrrol-2-one
1-{4-Methoxy-3-[2-(4-methyl-piperidin-1-yl)-ethoxy]-phenyl}-3-(4-trifluoromethyl-phenyl)-1,5-dihydro-pyrrol-2-one
3-(2-Chloro-phenyl)-1-{4-methoxy-3-[2-(4-methyl-piperidin-1-yl)-ethoxy]-phenyl}-1,5-dihydro-pyrrol-2-one
3-(3,4-Dichloro-phenyl)-3-hydroxy-1-{4-methoxy-3-[2-(4-methyl-piperidin-1-yl)-ethoxy]-phenyl} -pyrrolidin-2-one
3-(3,4-Dichloro-phenyl)-3-fluoro-1-{4-methoxy-3-[2-(4-methyl-piperidin-1-yl)-ethoxy]-phenyl}-pyrrolidin-2-one
3-(3,4-Dichloro-phenyl)-1-{4-methoxy-3-[(1-methyl-pyrrolidin-2-yl)-methoxy]-phenyl}-pyrrolidin-2-one
3-(3,4-Dichloro-phenyl)-1-{4-methoxy-3-[(1-methyl-pyrrolidin-2-yl)-methoxy]-phenyl}-3,4-dihydro-pyrrol-2-one
or a pharmaceutically acceptable salt thereof.

10. A process for the preparation of a compound as defined in any of claims 1-9, which process comprises:
(a) reacting a compound of formula (II): 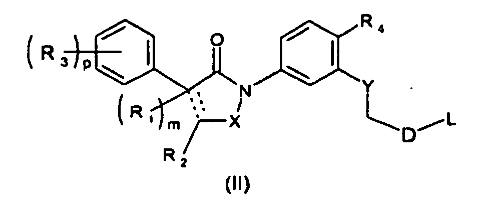 wherein R₁, R₂, R₃, R₄, m, p, X, 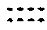 , Y and D are as defined for formula (I), and L is a leaving group, with a compound of formula (III):
Z-H (III)
wherein Z is as defined for formula (I); or
(b) cyclising a compound of formula (IV): 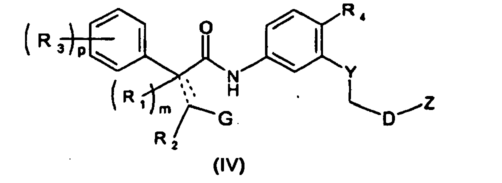
wherein R₁, R₂, m, R₃, p, R₄, Y, D, Z and 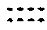 are as defined for formula (I) and G is a group -X=CH₂, wherein X is as defined for formula (I), dehydrogenated as required;
optionally followed by:
removing any protecting groups; and/or
converting a compound of formula (I) into another compound of formula (I); and/or
forming a pharmaceutically acceptable salt.

11. A pharmaceutical composition comprising a compound as defined in any of claims 1-9 and a pharmaceutically acceptable carrier or excipient.

12. A process for preparing a pharmaceutical composition as defined in claim 11, the process comprising mixing a compound a compound as defined in any of claims 1-9 and a pharmaceutically acceptable carrier or excipient.

13. A compound as defined in any of claims 1-9 for use as a therapeutic substance.

14. A compound as defined in any of claims 1-9 for use in the treatment of a CNS disorder.

15. A compound as defined in any of claims 1-9 for use in the treatment of depression or anxiety.

16. Use of a compound as defined in any of claims 1-9 in the manufacture of a medicament for use in the treatment of a CNS disorder.

17. The use of a compound as defined in any of claims 1-9 in the manufacture of a medicament for use in the treatment of depression or anxiety.

## Patentansprüche

1. Verbindung der Formel (I) oder ein pharmazeutisch verträgliches Salz davon, wobei: 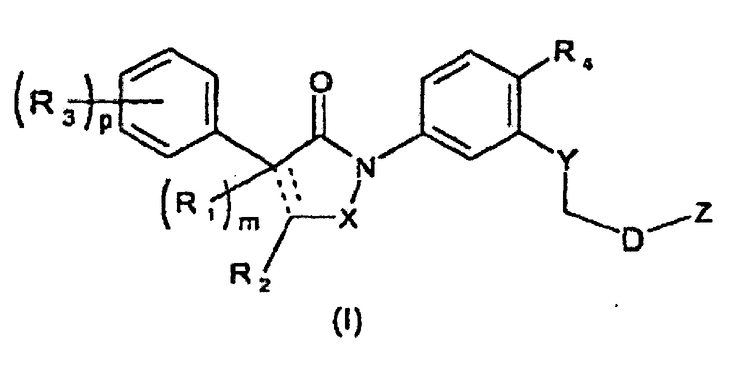
R₁ ein Wasserstoffatom, eine Hydroxygruppe, ein Fluor- oder Chloratom, ein C₁₋₆-Alkylrest, ein C₃₋₇-Cycloalkylrest, ein C₃₋₇-Cycloalkyloxyrest, ein C₁₋₆-Alkoxyrest oder ein Halogen-C₁₋₆-Alkoxyrest ist;
m die Bedeutung 0 hat, wenn 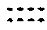 eine Doppelbindung ist, und m die Bedeutung 1 hat, wenn 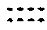 eine Einfachbindung ist;
R₂ ein Wasserstoff- oder Halogenatom, eine Cyanogruppe, eine Nitrogruppe, ein C₁₋₆-Alkylrest, ein C₃₋₇-Cycloalkylrest, ein C₃₋₇-Cycloalkyloxyrest, ein Halogen-C₁₋₆-Alkylrest, ein C₁₋₆-Alkoxyrest, ein Halogen-C₁₋₆-Alkoxyrest, ein C₁₋₆-Alkylthiorest, eine Aminogruppe, ein Mono- oder Di-C₁₋₆-Alkylaminorest oder ein N-verknüpfter 4- bis 7-gliedriger heterocyclischer Rest ist;
X -CH₂- ist;
R₃ ein Halogenatom ist;
p 1 oder 2 ist;
R₄ ein Wasserstoff- oder Halogenatom, eine Hydroxygruppe, eine Cyanogruppe, eine Nitrogruppe, ein C₁₋₆-Alkylrest, ein C₁₋₆-Alkanoylrest, ein C₃₋₇-Cycloalkylrest, ein C₃₋₇-Cycloalkyloxyrest, ein Halogen-C₁₋₆-Alkylrest, ein C₁₋₆-Alkoxyrest, ein Halogen-C₁₋₆-Alkoxyrest, ein C₁₋₆-Alkylthiorest, eine Aminogruppe, ein Mono- oder Di-C₁₋₆-Alkylaminorest oder ein N-verknüpfter 4- bis 7-gliedriger heterocyclischer Rest ist;
Y ein Sauerstoffatom ist;
D -CH₂- ist; und
Z ein gegebenenfalls substituierter N-verknüpfter 4- bis 7-gliedriger heterocyclischer Rest ist.

2. Verbindung wie in Anspruch 1 beansprucht, wobei, wenn 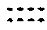 eine Einfachbindung ist, R₁ ein Wasserstoffatom, eine Hydroxygruppe oder ein C₁₋₆-Alkoxyrest ist.

3. Verbindung wie in Anspruch 1 beansprucht mit der folgenden Formel (Ia): 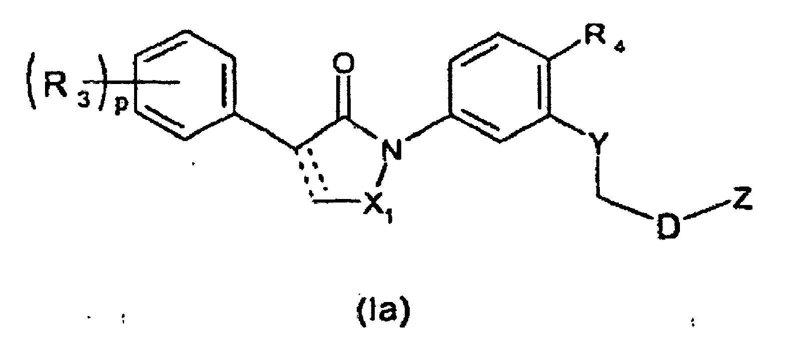 wobei R₃, p, R₄, Y, D, Z, 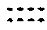 wie in Anspruch 1 beansprucht sind und X₁ -CH₂- ist.

4. Verbindung wie in einem der Ansprüche 1 bis 3 beansprucht, wobei p 1 oder 2 ist und R₃ jeweils ein Halogenatom ist, welches an Position 3 oder welche an Positionen 3 und 4 des Phenylrings gebunden ist/sind.

5. Verbindung wie in einem der Ansprüche 1 bis 4 beansprucht, wobei R₄ ein C₁₋₆-Alkoxyrest, eine Gruppe OCF₃, ein Halogenatom oder eine Cyanogruppe ist.

6. Verbindung wie in einem der Ansprüche 1 bis 5 beansprucht, wobei R₃ ein Chlor- oder Fluoratom ist.

7. Verbindung wie in einem der Ansprüche 1 bis 6 beansprucht, wobei R₄ eine Methoxygruppe ist.

8. Verbindung wie in einem der Ansprüche 1 bis 7 beansprucht, wobei Z ein Piperidylrest ist.

9. Verbindung wie in Anspruch 1 beansprucht, nämlich
3-(3,4-Dichlor-phenyl)-3-hydroxy-1-[4-methoxy-3-(2-piperidin-1-yl-ethoxy)-phenyl]-pyrrolidin-2-on
3-(3,4-Dichlor-phenyl)-3-hydroxy-1-[4-methoxy-3-(2-morpholin-4-yl-ethoxy)-phenyl]-pyrrolidin-2-on
3-(3,4-Dichlor-phenyl)-1-[4-methoxy-3-(2-piperidin-1-yl-ethoxy)-phenyl]-pyrrolidin-2-on
1-[4-Chlor-3-(2-piperidin-1-yl-ethoxy)-phenyl]-3-(3,4-dichlor-phenyl)-pyrrolidin-2-on
1-[4-Chlor-3-(2-piperidin-1-yl-ethoxy)-phenyl]-3-(3,4-dichlor-phenyl)-3-hydroxy-pyrrolidin-2-on
3-(3,4-Dichlor-phenyl)-1-(4-methoxy-3-[2-(4-methyl-piperidin-1-yl)-ethoxy]-phenyl)-pyrrolidin-2-on
3-(3,4-Dichlor-phenyl)-1-[4-methoxy-3-(2-piperidin-1-yl-ethoxy)-phenyl]-1,5-dihydro-pyrrol-2-on
3-(3,4-Dichlor-phenyl)-1-[4-methoxy-3-(2-morpholin-4-yl-ethoxy)-phenyl]-1,5-dihydro-pyrrol-2-on
1-[4-Chlor-3-(2-piperidin-1-yl-ethoxy)-phenyl]-3-(3,4-dichlor-phenyl)-1,5-dihydro-pyrrol-2-on
3-(3,4-Dichlor-phenyl)-1-[4-methoxy-3-(2-pyrrolidin-1-yl-ethoxy)-phenyl]-1,5-dihydro-pyrrol-2-on
3-(3-Fluor-phenyl)-1-[4-methoxy-3-(2-piperidin-1-yl-ethoxy)-phenyl]-1,5-dihydro-pyrrol-2-on
3-(3,4-Dichlor-phenyl)-1-(3-[2-(4,4-difluor-piperidin-1-yl)-ethoxy]-4-methoxy-phenyl)-1,5-dihydro-pyrrol-2-on
3-(3-Fluor-phenyl)-1-[4-methoxy-3-(2-piperidin-1-yl-ethoxy)-phenyl]-pyrrolidin-2-on
3-(3,4-Dichlor-phenyl)-1-[4-methoxy-3-(2-pyrrolidin-1-yl-ethoxy)-phenyl]-pyrrolidin-2-on
3-(3,4-Dichlor-phenyl)-1-[4-methoxy-3-(2-piperidin-1-yl-ethoxy)-phenyl]-3-methylpyrrolidin-2-on
3-(3-Chlor-phenyl)-1-{4-methoxy-3-[2-(4-methyl-piperidin-1-yl)-ethoxy]-phenyl}-1,5-dihydro-pyrrol-2-on
3-(3,4-Dichlor-phenyl)-1-(4-methoxy-3-[2-(4-methyl-piperidin-1-yl)-ethoxy]-phenyl)-3,4-dihydro-pyrrol-2-on
3-(3,4-Dichlor-phenyl)-1-(4-methoxy-3-[2-(4-methyl-piperidin-1-yl)-ethoxy]-phenyl)-4-methyl-1,5-dihydro-pyrrol-2-on
3-(4-Chlor-phenyl)-1-(4-methoxy-3-[2-(4-methyl-piperidin-1-yl)-ethoxy]-phenyl)-3,4-dihydro-pyrrol-2-on
3-(4-Chlor-phenyl)-1-(4-methoxy-3-[2-(piperidin-1-yl)-ethoxy]-phenyl)-3,4-dihydro-pyrrol-2-on
3-(3,4-Dichlor-phenyl)-1-[4-methoxy-3-(piperidin-3-ylmethoxy)-phenyl]-pyrrolidin-2-on
3-(3,4-Dichlor-phenyl)-1-[4-methoxy-3-(1-methyl-piperidin-3-ylmethoxy)-phenyl]-pyrrolidin-2-on
*N-*{4-[3-(3,4-Dichlor-phenyl)-2-oxo-2,5-dihydro-1*H*-pyrrol-1-yl]-2-[2-(1-piperidinyl)-ethoxy]-phenyl}-acetamid
*N-*{4-[3-(3,4-Dichlor-phenyl)-2-oxo-pyrrolidin-1-yl]-2-[2-(1-piperidinyl)-ethoxy]-phenyl}-acetamid
3-(3,4-Dichlor-phenyl)-1-{4-methoxy-3-[2-(4-methyl)-piperidin-1-yl-ethoxy]-phenyl}-3-methyl-pyrrolidin-2-on
3-(3-Chlor-phenyl)-1-[4-methoxy-3-(2-piperidin-1-yl-ethoxy)-phenyl]-pyrrolidin-2-on
3-(3-Trifluormethyl-phenyl)-1-[4-methoxy-3-(2-piperidin-1-yl-ethoxy)-phenyl]-pyrrolidin-2-on
3-(3-Trifluormethyl-phenyl)-1-[4-methoxy-3-(2-piperidin-1-yl-ethoxy)-phenyl]-1,5-dihydro-pyrrol-2-on
3-(3-Chlor-phenyl)-1-[4-methoxy-3-(2-piperidin-1-yl-ethoxy)-phenyl]-1,5-dihydro-pyrrol-2-on
3-(4-Fluor-phenyl)-1-[4-methoxy-3-(2-piperidin-1-yl-ethoxy)-phenyl]-1,5-dihydro-pyrrol-2-on
3-(4-Fluor-phenyl)-1-[4-methoxy-3-(2-piperidin-1-yl-ethoxy)-phenyl]-pyrrolidin-2-on
1-{4-Methoxy-3-[2-(4-methyl-piperidin-1-yl)-ethoxy]-phenyl}-3-(4-methyl-phenyl)-1,5-dihydro-pyrrol-2-on
1- {4-Methoxy-3-[2-(piperidin-1-yl)-ethoxy]-phenyl} -3-(4-methyl-phenyl)-1,5-dihydro-pyrrol-2-on
3-(4-Brom-phenyl)-1-{4-methoxy-3-[2-(4-methyl-piperidin-1-yl)-ethoxy]-phenyl}-1,5-dihydro-pyrrol-2-on
1-{4-Methoxy-3-[2-(4-methyl-piperidin-1-yl)-ethoxy]-phenyl} -3-(4-trifluormethylphenyl)-1,5-dihydro-pyrrol-2-on
3-(2-Chlor-phenyl)-1- {4-methoxy-3-[2-(4-methyl-piperidin-1-yl)-ethoxy]-phenyl} -1,5-dihydro-pyrrol-2-on
3-(3,4-Dichlor-phenyl)-3-hydroxy-1- {4-methoxy-3-[2-(4-methyl-piperidin-1-yl)-ethoxy]-phenyl}-pyrrolidin-2-on
3-(3,4-Dichlor-phenyl)-3-fluor-1- {4-methoxy-3-[2-(4-methyl-piperidin-1-yl)-ethoxy]-phenyl}-pyrrolidin-2-on
3-(3,4-Dichlor-phenyl)-1-{4-methoxy-3-[(1-methyl-pyrrolidin-2-yl)-methoxy]-phenyl}-pyrrolidin-2-on
3-(3,4-Dichlor-phenyl)-1-{4-methoxy-3-[(1-methyl-pyrrolidin-2-yl)-methoxy]-phenyl}-3,4-dihydro-pyrrol-2-on
oder ein pharmazeutisch verträgliches Salz davon.

10. Verfahren zur Herstellung einer Verbindung wie in einem der Ansprüche 1 bis 9 definiert, wobei das Verfahren umfasst:
(a) Umsetzen einer Verbindung der Formel (II): 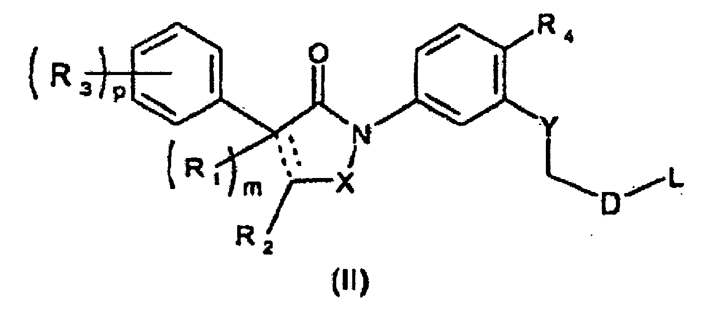 wobei R₁, R₂, R₃, R₄, m, p, X, 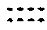 , Y und D die vorstehende für Formel (I) angegebene Bedeutung haben und L eine Abgangsgruppe ist mit einer Verbindung der Formel (III):
Z-H (III)
wobei Z die vorstehende für Formel (I) angegebene Bedeutung hat; oder
(b) Cyclisieren einer Verbindung der Formel (IV): 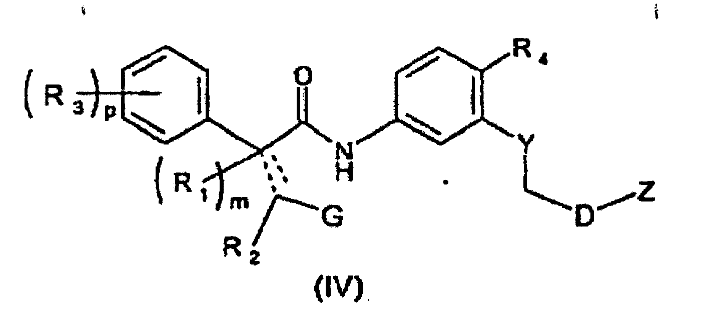
wobei R₁, R₂, m, R₃, p, R₄, Y, D, Z und 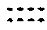 die vorstehende für Formel (I) angegebene Bedeutung haben und G eine Gruppe -X=CH₂ ist, wobei X die vorstehende für Formel (I) angegebene Bedeutung hat und wie erforderlich dehydrogeniert ist;
gegebenenfalls gefolgt von:
Entfernen von Schutzgruppen; und/oder
Umwandeln einer Verbindung der Formel (I) in eine andere Verbindung der Formel (I); und/oder
Bildung eines pharmazeutisch verträglichen Salzes.

11. Arzneimittel, umfassend eine Verbindung wie in einem der Ansprüche 1 bis 9 definiert und einen pharmazeutisch verträglichen Träger oder Exzipienten.

12. Verfahren zur Herstellung eines Arzneimittels wie in Anspruch 11 definiert, wobei das Verfahren das Mischen einer Verbindung wie in einem der Ansprüche 1 bis 9 definiert und eines pharmazeutisch verträglichen Trägers oder Exzipienten umfasst.

13. Verbindung wie in einem der Ansprüche 1 bis 9 definiert zur Verwendung als Therapeutikum.

14. Verbindung wie in einem der Ansprüche 1 bis 9 definiert zur Verwendung bei der Behandlung einer Krankheit des zentralen Nervensystems.

15. Verbindung wie in einem der Ansprüche 1 bis 9 definiert zur Verwendung bei der Behandlung von Depression oder Angst.

16. Verwendung einer Verbindung wie in einem der Ansprüche 1 bis 9 definiert zur Herstellung eines Medikaments zur Verwendung bei der Behandlung einer Krankheit des zentralen Nervensystems.

17. Verwendung einer Verbindung wie in einem der Ansprüche 1 bis 9 definiert zur Herstellung eines Medikaments zur Verwendung bei der Behandlung von Depression oder Angst.

## Revendications

1. Composé de formule (I) ou un de ses sels pharmaceutiquement acceptables, formule dans laquelle : 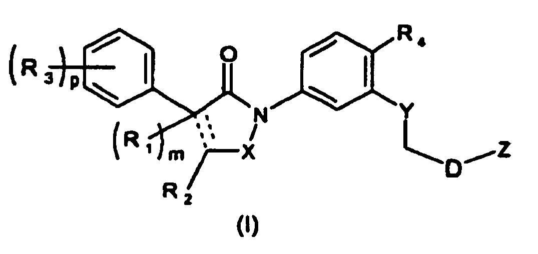
R₁ représente un atome d'hydrogène, un groupe hydroxy, fluoro, chloro, alkyle en C₁ à C₆, cycloalkyle en C₃ à C₇, cycloalkyloxy en C₃ à C₇, alkoxy en C₁ à C₆ ou halogénalkoxy en C₁ à C₆ ;
m est égal à 0 lorsque 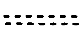 représente une double liaison et m est égal à 1 lorsque 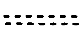 représente une liaison simple ;
R₂ représente un atome d'hydrogène, un atome d'halogène, un groupe cyano, nitro, alkyle en C₁ à C₆, cycloalkyle en C₃ à C₇, cycloalkyloxy en C₃ à C₇, halogénalkyle en C₁ à C₆, alkoxy en C₁ à C₆, halogénalkoxy en C₁ à C₆, alkylthio en C₁ à C₆, amino, mono- ou di (alkyle en C₁ à C₆) amino ou un groupe hétérocyclique tétra- à heptagonal lié par N ;
X représente un groupe -CH₂- ;
R₃ représente un atome d'halogène ;
p est égal à 1 ou 2 ;
R₄ représente un atome d'hydrogène, un atome d'halogène, un groupe hydroxy, cyano, nitro, alkyle en C₁ à C₆, alcanoyle en C₁ à C₆, cycloalkyle en C₃ à C₇, cycloalkyloxy en C₃ à C₇, halogénalkyle en C₁ à C₆, alkoxy en C₁ à C₆, halogénalkoxy en C₁ à C₆, alkylthio en C₁ à C₆, amino, mono- ou di (alkyle en C₁ à C₆) amino ou un groupe hétérocyclique tétra- à heptagonal lié par N ;
Y représente un atome d'oxygène ;
D représente un groupe -CH₂- ; et
Z représente un groupe hétérocyclique tétra- à heptagonal lié par N, facultativement substitué.

2. Composé suivant la revendication 1, dans lequel, lorsque 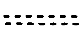 représente une liaison simple, R₁ représente un atome d'hydrogène, un groupe hydroxy ou alkoxy en C₁ à C₆.

3. Composé suivant la revendication 1, répondant à la formule (Ia) suivante : 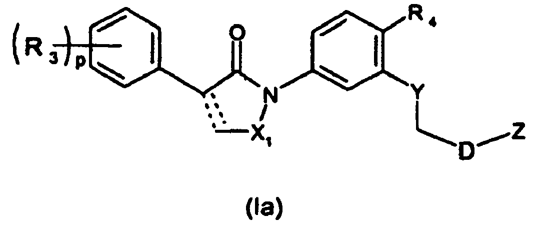 dans laquelle R₃, p, R₄, Y, D, Z, 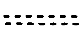 sont tels que définis dans la revendication 1 et X₁ représente un groupe -CH₂-.

4. Composé suivant l'une quelconque des revendications 1 à 3, dans lequel p est égal à 1 ou 2 et le ou les groupes R₃ représentent un atome d'halogène, fixé en position 3 ou en positions 3,4 du noyau phényle.

5. Composé suivant l'une quelconque des revendications 1 à 4, dans lequel R₄ représente un groupe alkoxy en C₁ à C₆, OCF₃, halogéno ou cyano.

6. Composé suivant l'une quelconque des revendications 1 à 5, dans lequel R₃ représente un groupe chloro ou fluoro.

7. Composé suivant l'une quelconque des revendications 1 à 6, dans lequel R₄ représente un groupe méthoxy.

8. Composé suivant l'une quelconque des revendications 1 à 7, dans lequel Z représente un groupe pipéridyle.

9. Composé suivant la revendication 1, qui est :
la 3-(3,4-dichloro-phényl)-3-hydroxy-1-[4-méthoxy-3-(2-pipéridine-1-yl-éthoxy)-phényl]-pyrrolidine-2-one,
la 3-(3,4-dichloro-phényl)-3-hydroxy-1-[4-méthoxy-3-(2-morpholine-4-yl-éthoxy)-phényl]-pyrrolidine-2-one,
la 3-(3,4-dichloro-phényl)-1-[4-méthoxy-3-(2-pipéridine-1-yl-éthoxy)-phényl]-pyrrolidine-2-one,
la 1-[4-chloro-3-(2-pipéridine-1-yl-éthoxy)-phényl]-3-(3,4-dichloro-phényl)-pyrrolidine-2-one,
la 1-[4-chloro-3-(2-pipéridine-1-yl-éthoxy)-phényl]-3-(3,4-dichloro-phényl)-3-hydroxy-pyrrolidine-2-one,
la 3-(3,4-dichloro-phényl)-1-(4-méthoxy-3-[2-(4-méthyl-pipéridine-1-yl)-éthoxy]-phényl)-pyrrolidine-2-one,
la 3-(3,4-dichloro-phényl)-1-[4-méthoxy-3-(2-pipéridine-1-yl-éthoxy)-phényl]-pyrrole-2-one,
la 3-(3,4-dichloro-phényl)-1-[4-méthoxy-3-(2-morpholine-4-yl-éthoxy)-phényl]-1,5-dihydro-pyrrole-2-one,
la 1-[4-chloro-3-(2-pipéridine-1-yl-éthoxy)-phényl]-3-(3,4-dichloro-phényl)-1,5-dihydro-pyrrole-2-one,
la 3-(3,4-dichloro-phényl) -1- [4-méthoxy-3- (2-pyrrolidine-1-yl-éthoxy)-phényl]-1,5-dihydro-pyrrole-2-one,
la 3-(3-fluoro-phényl)-1-[4-méthoxy-3-(2-pipéridine-1-yl-éthoxy)-phényl]-1,5-dihydro-pyrrole-2-one,
la 3-(3,4-dichloro-phényl)-1-(3-[2-(4,4-difluoro-pipéridine-1-yl)-éthoxy]-4-méthoxy-phényl)-1,5-dihydro-pyrrole-2-one,
la 3-(3-fluoro-phényl)-1-[4-méthoxy-3-(2-pipéridine-1-yl-éthoxy)-phényl]-pyrrolidine-2-one,
la 3-(3,4-dichloro-phényl)-1- [4-méthoxy-3-(2-pipéridine-1-yl-éthoxy)-phényl]-pyrrolidine-2-one,
la 3-(3,4-dichloro-phényl)-1- [4-méthoxy-3- (2-pipéridine-1-yl-éthoxy)-phényl]-3-méthyl-pyrrolidine-2-one,
la 3-(3-chloro-phényl)-1-{4-méthoxy-3-[2-(4-méthyl-pipéridine-1-yl)-éthoxy]-phényl}-1,5-dihydro-pyrrole-2-one,
la 3-(3,4-dichloro-phényl)-1-(4-méthoxy-3-[2-(4-méthyl-pipéridine-1-yl)-éthoxy]-phényl)-3,4-dihydro-pyrrole-2-one,
la 3-(3,4-dichloro-phényl)-1-(4-méthoxy-3-[2-(4-méthyl-pipéridine-1-yl)-éthoxy]-phényl)-4-méthyl-1,5-dihydro-pyrrole-2-one,
la 3-(4-chloro-phényl)-1-(4-méthoxy-3-[2-(4-méthyl-pipéridine-1-yl)-éthoxy]-phényl)-3,4-dihydro-pyrrole-2-one,
la 3-(4-chloro-phényl)-1-(4-méthoxy-3-[2-pipéridine-1-yl)-éthoxy]-phényl)-3,4-dihydro-pyrrole-2-one,
la 3-(3,4-dichloro-phényl) -1- [4-méthoxy-3-(pipéridine-3-ylméthoxy)-phényl]-pyrrolidine-2-one,
la 3-(3,4-dichloro-phényl)-1-[4-méthoxy-3-(1-méthyl-pipéridine-3-ylméthoxy)-phényl]-pyrrolidine-2-one,
le *N-*{4-[3-(3,4-dichlorophényl)-2-oxo-2,5-dihydro-1*H-*pyrrole-1-yl]-2-[2-(1-pipéridinyl)-éthoxy]phényl}acétamide,
le *N-*{4-[3-(3,4-dichlorophényl)-2-oxo-pyrrolidine-1-yl]-2-[2-(1-pipéridinyl)éthoxy]phényl}-acétamide,
la 3-(3,4-dichloro-phényl) -1-{4-méthoxy-3- [2- (4-méthyl) - pipéridine-1-yl-éthoxy]-phényl}-3-méthyl-pyrrolidine-2-one,
la 3-(3-chloro-phényl)-1-[4-méthoxy-3-(2-pipéridine-1-yl-éthoxy)-phényl]-pyrrolidine-2-one,
la 3-(3-trifluorométhyl-phényl)-1-[4-méthoxy-3-(2-pipéridine-1-yl-éthoxy)-phényl]-pyrrolidine-2-one,
la 3-(3-trifluorométhyl-phényl)-1-[4-méthoxy-3-(2-pipéridine-1-yl-éthoxy)-phényl]-1,5-dihydro-pyrrole-2-one,
la 3-(3-chloro-phényl)-1-[4-méthoxy-3-(2-pipéridine-1-yl-éthoxy)-phényl]-1,5-dihydro-pyrrole-2-one,
la 3-(4-fluoro-phényl)-1-[4-méthoxy-3-(2-pipéridine-1-yl-éthoxy)-phényl]-1,5-dihydro-pyrrole-2-one,
la 3-(4-fluoro-phényl)-1-[4-méthoxy-3-(2-pipéridine-1-yl-éthoxy)-phényl]-pyrrolidine-2-one,
la 1-{4-méthoxy-3- [2-(4-méthyl-pipéridine-1-yl)-éthoxy] - phényl}-3-(4-méthyl-phényl)-1,5-dihydro-pyrrole-2-one,
la 1-{4-méthoxy-3-[2-(pipéridine-1-yl)-éthoxy]-phényl}-3-(4-méthyl-phényl)-1,5-dihydro-pyrrole-2-one,
la 3-(4-bromo-phényl)-1-{4-méthoxy-3-[2-(4-méthyl-pipéridine-1-yl)-éthoxy]-phényl}-1,5-dihydro-pyrrole-2-one,
la 1-{4-méthoxy-3-[2-(4-méthyl-pipéridine-1-yl)-éthoxy] - phényl}-3-(4-trifluorométhyl-phényl)-1,5-dihydro-pyrrole-2-one,
la 3-(2-chloro-phényl)-1-{4-méthoxy-3-[2-(4-méthyl-pipéridine-1-yl)-éthoxy]-phényl}-1,5-dihydro-pyrrole-2-one,
la 3-(3,4-dichloro-phényl)-3-hydroxy-1-{4-méthoxy-3-[2-(4-méthyl-pipéridine-1-yl)-éthoxy]-phényl}-pyrrolidine-2-one,
la 3-(3,4-dichloro-phényl)-3-fluoro-1-{4-méthoxy-3-[2-(4-méthyl-pipéridine-1-yl) -éthoxy]-phényl}-pyrrolidine-2-one,
la 3-(3,4-dichloro-phényl)-1-{4-méthoxy-3-[(1-méthyl-pyrrolidine-2-yl)-méthoxy]-phényl}-pyrrolidine-2-one,
la 3-(3,4-dichloro-phényl)-1-{4-méthoxy-3-[(1-méthyl-pipéridine-2-yl)-méthoxy]-phényl}-3,4-dihydro-pyrrole-2-one,
ou un de ses sels pharmaceutiquement acceptables.

10. Procédé pour la préparation d'un composé tel que défini dans l'une quelconque des revendications 1 à 9, lequel procédé comprend :
(a) la réaction d'un composé de formule (II) : 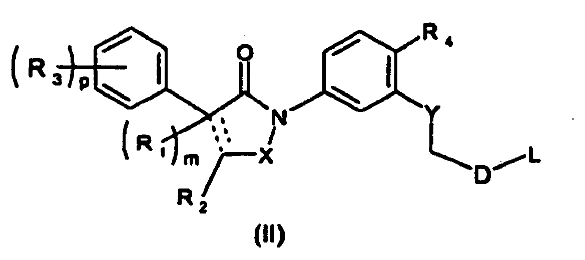 dans laquelle R₁, R₂, R₃, R₄, m, p, X 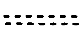 , Y et D sont tels que définis pour la formule (I), et L représente un groupe partant, avec un composé de formule (III) :
Z-H (III)
dans laquelle Z est tel que défini pour la formule (I) ; ou
(b) la cyclisation d'un composé de formule (IV) : 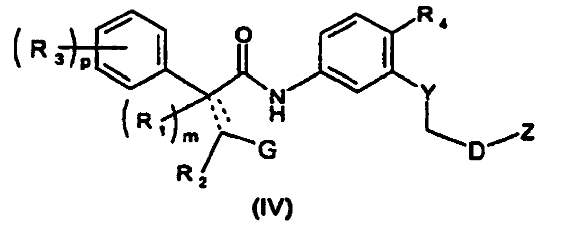
dans laquelle R₁, R₂, m, R₃, p, R₄, Y, D, Z et 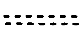 sont tels que définis pour la formule (I), et G représente un groupe -X=CH₂, dans lequel X est tel que défini pour la formule (I), déshydrogéné de la manière requise ; avec ensuite facultativement :
l'élimination de n'importe quels groupes protecteurs ; et/ou
la conversion d'un composé de formule (I) en un autre composé de formule (I) ; et/ou
la formation d'un sel pharmaceutiquement acceptable.

11. Composition pharmaceutique comprenant un composé tel que défini dans l'une quelconque des revendications 1 à 9, et un support ou excipient pharmaceutiquement acceptable.

12. Procédé pour la préparation d'une composition pharmaceutique telle que définie dans la revendication 11, lequel procédé comprenant le mélange d'un composé tel que défini dans l'une quelconque des revendications 1 à 9, et d'un support ou excipient pharmaceutiquement acceptable.

13. Composé tel que défini dans l'une quelconque des revendications 1 à 9, destiné à être utilisé comme substance thérapeutique.

14. Composé tel que défini dans l'une quelconque des revendications 1 à 9, destiné à être utilisé dans le traitement d'un trouble du SNC.

15. Composé tel que défini dans l'une quelconque des revendications 1 à 9, destiné à être utilisé dans le traitement des dépressions ou de l'anxiété.

16. Utilisation d'un composé tel que défini dans l'une quelconque des revendications 1 à 9, dans la production d'un médicament destiné à être utilisé dans le traitement d'un trouble du SNC.

17. utilisation d'un composé tel que défini dans l'une quelconque des revendications 1 à 9, dans la production d'un médicament destiné à être utilisé dans le traitement des dépressions ou de l'anxiété.
